# EUROPEAN PATENT SPECIFICATION

(11) **EP 4 267 229 B1**
(45) Date of publication and mention of the grant of the patent: **11.02.2026**
(21) Application number: 21912204.1
(22) Date of filing: 22.12.2021
(51) Int. Cl.: A61M 25/02

(54) **CATHETER HOUSING**
KATHETERGEHÄUSE
CORPS DE CATHÉTER

(30) Priority: 23.12.2020 US 202063130215 P
(43) Date of publication of application: 01.11.2023
(73) Proprietor: Vasonics, Inc., Corona, CA 92882 (US)
(72) Inventor: ALBANY, Ramy, Corona, California 92882 (US)
(74) Representative: AWA Sweden AB
(86) International application number: PCT/US2021/064998
(87) International publication number: WO 2022/140633

(56) References cited:
- WO-A1-2020/160318
- WO-A2-2006/113620
- US-A- 4 898 587
- US-A1- 2007 043 326
- US-A1- 2012 010 572
- US-A1- 2015 073 347
- US-A1- 2018 177 982
- US-A1- 2020 330 732
- US-B2- 10 537 714

## Description

### TECHNICAL FIELD

In general, the present disclosure relates to catheter stabilization devices, systems, and methods.

### BACKGROUND

Intravenous catheter procedures have been utilized on patients for over a century. Peripheral Intravenous Catheters (PIVC) and Peripherally Inserted Central Catheters (PICC) are commonly employed on patients within a hospital or care facility to provide medication, nutrition, and/or fluids. After cleaning a catheter insertion site, a needle is inserted into a vein of a patient and a catheter is then inserted into the vein. Such catheter is typically coupled with a catheter hub.

US 2018/0177982 A1 describes a catheter housing device designed to secure a catheter and needle assembly to a patient. The device includes components such as a cover, an IV/ catheter lock, a hub, and one or more fastening straps.

US2020330732A1 describes a catheter dressing designed to secure and protect a catheter insertion site.

### SUMMARY

Stabilizing a catheter when inserted into in a catheter insertion is of critical importance. Phlebitis (inflammation of the vein) is commonly caused by movement of the catheter relative to a blood vessel wall. Traditional techniques of stabilizing a catheter involve applying one or more adhesive film dressings directly over a catheter hub connected to a catheter to the patient's skin and over a catheter insertion site. Such techniques have a variety of drawbacks. For example, such techniques do not sufficiently inhibit catheters from becoming dislodged (for example, due to patient movement) and from being pushed further into a patient's arm (or other body part). The adhesive dressings utilized in such techniques cover and incubate germs and bacteria at the insertion site, which can lead to various infections and complications. Frequent replacement of such adhesive dressings or a catheter can increase risk of bacterial infection and cause irritation and trauma to the insertion site (especially with geriatric patients). Traditional techniques also inhibit normal skin respiration and ventilation, raise humidity, and raise temperature at and near the insertion site (which can promote microorganism growth).

Another disadvantage of traditional catheter stabilization techniques is that applying tape directly to a catheter hub (connected to a catheter) over the catheter insertion site results in a tip of the catheter being improperly angled within the vein such that the catheter tip can erode or otherwise cause damage to the vein and/or vein wall. This erosion and other damage can be exacerbated when the patient moves. Such techniques also obscure the catheter insertion site and prevent assessment of catheter status and/or complications. "Kinking" of a catheter is also a common problem in conventional catheter stabilization techniques. Commonly accepted practice requires the removal of catheters after a 72-96 hour dwell time. Thus, even under the best circumstances, with conventional approaches, catheters have a relatively short life span that requires frequent adjustment and/or movement. Catheter failures and frequent catheter movement can result in costly catheter replacements, increased costs due to extended patient care time, and eventually venous depletion. Venous depletion leads to more invasive, risky, and costly venous access devices.

The present disclosure provides devices, systems, and methods (not claimed) for stabilizing a catheter at a catheter insertion site. Disclosed devices, systems, and methods (not claimed) can reduce or eliminate problems associated with conventional techniques for securing a catheter hub and a catheter at a catheter insertion site such as those discussed above. The disclosed devices, systems, and methods (not claimed) can also extend catheter dwell time beyond the current standard dwell period.

Some implementations of the devices, systems, and methods (not claimed) disclosed herein provide various disinfection and/or sterilization capabilities for catheter insertion sites and/or portions of catheters and/or catheter hubs. For example, some implementations of the disclosed catheter housing devices can be supplied with sterilizing inert gas which can suffocate microbes or other contaminants in and around the catheter insertion site, catheter, catheter hub coupled to the catheter, luer connector coupled to the catheter hub, a portion of a connected fluid tube, and/or portions within an interior defined by the catheter housing devices when secured to the subject. The catheter housing devices discussed herein (or portions thereof) can be coated with anti-microbial coating to aid with disinfection and/or sterilization near the catheter insertion site and/or in or around the catheter housing device. Various components of the disclosed catheter housing devices can be structured to form a hermetic seal, which can advantageously inhibit or prevent microbes or other contaminants from entering portions of the catheter housing devices. Implementations of disclosed catheter housing devices can include a gas port for providing sterilizing gases or other gases. Soothing, antiseptic, anesthetic, and/or similar vapor drugs can be provided to a catheter insertion site via the disclosed catheter housing devices in a gaseous form to aid healing or reconstruction in and around the catheter insertion site. Some implementations of the disclosed catheter housing devices include one or more UV SMD ("Surface Mount") LEDs that can provide active sterilization and/or disinfection of areas at or near the catheter insertion site, catheter, catheter hub coupled to the catheter, luer connector connected to the catheter hub, a portion of a connected fluid tube, and/or portions within an interior defined by the catheter housing device when secured to the subject. Such UV SMD LEDs can also illuminate interior regions within the catheter housing device, for example at or near the catheter insertion site.

Disclosed catheter housing devices and methods (not claimed) can: avoid applying pressure directly to a needle, insertion site, and/or catheter; avoid kinking of a catheter; dramatically reduce the potential for contamination in and/or around the catheter insertion site; and/or allow an insertion site to remain readily visible to a caregiver. Some implementations of the disclosed catheter housing devices mechanically isolate the catheter and/or catheter hub from patient movement and hold the catheter and/or catheter hub at a proper (for example, "natural") insertion angle. Some implementations of the disclosed catheter housing devices provide a catheter stabilization system that is highly skin breathable, allowing for patient comfort and reduced skin irritation or skin trauma. Catheter housing devices and methods for securing the same to subjects are described in jointly owned U.S. Pat. Pub. No. 2019/0381 652.

As used herein, the term "catheter hub" (which may also be referred to as a "catheter device" or "catheter hub device") refers to a device coupled to a catheter (which may also be referred to herein as an "intravenous catheter", "IV catheter", "cannula", "intravenous line"). The catheter hubs discussed herein can be connected to a fluid tube via a connector, such as a male luer connector. The catheter hubs discussed herein can include one or more cylindrical portions that are coupled to a portion of the catheter and which can be directly connected to a luer connector (for example, a male luer connector), and such luer connector can indirectly connect the catheter hub to a fluid tube. The catheter housing devices (which may also be referred to herein as "catheter stabilization devices", "catheter housings," and "housing") described herein can stabilize a catheter by securing one or more portions of the catheter hub and/or by securing such luer connector which is connected to such catheter hub. For example, the catheter housing devices discussed herein can secure (or at least partially secure) one or more cylindrical portions of a catheter hub, a luer connector configured to connect to such catheter hub, and/or a portion of the fluid tube connected to such luer connector. Such securement can thereby position and/or stabilize the catheter coupled to the catheter hub when the catheter is inserted at the catheter insertion site (for example, extending through skin and into a vein of a subject). The fluid tubes described herein (which may also be referred to herein as a "tube" and "tubing") can deliver fluids (including medicinal, nutritional, and/or other fluids) to the catheters, and in turn, to the subject.

Disclosed herein is a catheter housing device configured for use at a catheter insertion site on a subject, the catheter housing device comprising a hub and a cover. The hub is configured for placement at the catheter insertion site and comprises: a membrane comprising an opening, the membrane configured to secure to the subject's skin such that the catheter insertion site is positioned within the opening; and at least one arm coupled with the membrane and extending partially across the opening, wherein the at least one arm is configured to be positioned above the subject's skin when the membrane is secured to the subject's skin, and wherein a free end of the at least one arm is spaced inward from a perimeter of the opening and is configured to secure a catheter hub connected to a catheter to minimize movement of the catheter when inserted in the catheter insertion site. The cover is configured to removably secure to the hub and enclose the catheter insertion site on the subject.

In some implementations, said at least one arm comprises a pair of opposing arms, and wherein the free ends of the pair of opposing arms are spaced from one another by a gap and are configured to secure said catheter hub when the catheter housing device is in use. In some implementations, the catheter housing device further comprises a frame connected to the membrane along at least a portion of the perimeter of the opening (for example, all of or less than an entirety of the perimeter of the opening). In some implementations, said at least one arm comprises: a first arm extending outward from a first portion of the frame and partially across the opening, the first arm comprising a connected end that is connected to the first portion of the frame and a free end that is opposite the connected end of the first arm; and a second arm extending outward from a second portion of the frame and partially across the opening towards the first arm, the second arm comprising a connected end that is connected to the second portion of the frame and a free end that is opposite the connected end of the second arm. In some implementations, the free ends of the first and second arms are spaced inward from the perimeter of the opening and spaced from one another by a gap; and are configured to secure said catheter hub when the catheter housing device is in use. In some implementations, the second portion of the frame is opposite the first portion of the frame.

In some implementations, said free ends of the first and second arms are configured to secure said catheter hub in an inclined position relative to the subject's skin when in use. In some implementations, said free ends of the first and second arms are spaced above a plane extending along a bottom surface of the membrane. In some implementations, each of the first and second arms comprises a first section extending outward from the frame and a second section at the free end, said second section connected to the first section and configured to contact the catheter hub when the catheter hub is secured by the free ends of the first and second arms. In some implementations, the second section is transverse relative to the first section. In some implementations, the second section is generally perpendicular relative to the first section. In some implementations, the second section is at least partially curved to conform to and surround a portion of the catheter hub when the catheter hub is secured by the free ends of the first and second arms. In some implementations, surfaces of the second sections of the first and second arms that face in a direction toward one another comprise matching curvatures. In some implementations, the first and second arms are mirror images of one another.

In some implementations, each of the first and second arms comprise a generally T-shaped structure having a stem connected to the first or second portions of the frame and a flange connected to the stem and spaced away from said frame, and wherein at least a portion of said flange is curved to conform to and surround a portion of the catheter hub when the catheter hub is secured by the first and second arms. In some implementations, the frame comprises a first material and the membrane comprises a second material that is different than the first material. In some implementations, the second material is more flexible than the first material. In some implementations, the first material is more rigid than the second material. In some implementations, the frame comprises an annular shape.

In some implementations, no portion of the catheter housing device is positioned between the first and second arms and the subject's skin when the catheter hub is secured by the first and second arms in use. In some implementations, no portion of the cover contacts the catheter hub when the catheter hub is secured by the first and second arms in use. In some implementations, no portion of the membrane contacts the catheter hub when the catheter hub is secured by the first and second arms in use. In some implementations, the catheter hub is spaced inward from the perimeter of the opening when the catheter hub is secured by the first and second arms in use.

In some implementations, the catheter hub comprises a first end connected to the catheter and a second end opposite the first end of the catheter hub, and wherein, when the catheter hub is secured by the first and second arms, the second end of the catheter hub does not contact the subject's skin. In some implementations, when the catheter hub is secured by the first and second arms, only the first end of the catheter hub contacts the subject's skin. In some implementations, when the catheter hub is secured by the first and second arms, no portion of the catheter hub contacts the subject's skin. In some implementations, said free ends of the first and second arms are spaced above a plane extending along a bottom of the frame.

In some implementations, the catheter housing device further comprises a moisture wicking element coupled with the membrane and configured to contact the subject's skin and encircle the catheter insertion site when the membrane is secured to the subject's skin, said moisture wicking element configured to wick moisture away from the catheter insertion site when the catheter housing device is in use. In some implementations, said membrane comprises a plurality of apertures spaced around said opening.

In some implementations, the catheter housing device further comprises one or more adhesive substrates coupled to a bottom surface of the membrane and configured to secure the membrane to the subject's skin. In some implementations, the membrane further comprises a plurality of recessed portions, each of the plurality of recessed portions positioned adjacent to one of the plurality of apertures of the membrane and recessed inward from a plane extending along the bottom surface of the membrane, said plurality of recessed portions configured to allow an adhesive removal liquid to flow under said bottom surface.

In some implementations, the cover comprises a top portion and a wall extending outward from the top portion and defining an interior, and wherein the wall of the cover is configured to removably secure to the frame of the hub. In some implementations, the frame comprises one or more latch legs and the wall of the cover comprises one or more latch protrusions configured to removably secure to the one or more latch legs of the frame.

In some implementations, the one or more latch legs extend from the frame in a direction that is generally perpendicular relative to the first and second arms. In some implementations, the wall comprises at least one latch protrusion at a first end of the wall and at least one latch protrusion at a second end of the wall that is opposite the first end of the wall. In some implementations, the cover further comprises an opening extending through a portion of the wall, wherein the opening of the cover is configured to allow a fluid tube to pass through the wall and connect with the catheter hub. In some implementations, said fluid tube is connected to the catheter hub via a male luer connector. In some implementations, the frame is permanently secured to the membrane.

Disclosed herein is a catheter housing device configured for use at a catheter insertion site on a subject, the catheter housing device comprising a hub and a cover. The hub is configured for placement at the catheter insertion site and comprises: a membrane comprising an opening, the membrane configured to secure to the subject's skin such that the catheter insertion site is positioned within the opening; a frame connected to the membrane along a perimeter of the opening; and a first arm extending outward from a first portion of the frame and partially across the opening, the first arm comprising a first end that is connected to the first portion of the frame and a second end that is opposite the first end of the first arm; and a second arm extending outward from a second portion of the frame and partially across the opening towards the first arm, the second arm comprising a first end that is connected to the second portion of the frame and a second end that is opposite said first end of the second arm. The second ends of the first and second arms are: spaced inward from the perimeter of the opening and spaced from one another by a gap; and configured to secure a catheter hub connected to a catheter to minimize movement of the catheter when inserted in the catheter insertion site. The cover is configured to removably secure to the frame of the hub and enclose the catheter insertion site on the subject.

In some implementations, the second portion of the frame is opposite the first portion of the frame. In some implementations, said second ends of the first and second arms are configured to secure said catheter hub in an inclined position relative to the subject's skin when in use. In some implementations, said second ends of the first and second arms are spaced above a plane extending along a bottom surface of the membrane.

In some implementations, each of the first and second arms comprises a first section extending outward from the frame and a second section at the second end, said second section connected to the first section and configured to contact the catheter hub when the catheter hub is secured by the second ends of the first and second arms. In some implementations, the second section is transverse relative to the first section. In some implementations, the second section is generally perpendicular relative to the first section. In some implementations, the second section is at least partially curved to conform to and surround a portion of the catheter hub when the catheter hub is secured by the second ends of the first and second arms. In some implementations, surfaces of the second sections of the first and second arms that face in a direction toward one another comprise matching curvatures. In some implementations, the first and second arms are mirror images of one another.

In some implementations, each of the first and second arms comprise a generally T-shaped structure having a stem connected to the first or second portions of the frame and a flange connected to the stem and spaced away from said frame, and wherein at least a portion of said flange is curved to conform to and surround a portion of the catheter hub when the catheter hub is secured by the first and second arms. In some implementations, the frame comprises a first material and the membrane comprises a second material that is different than the first material. In some implementations, the second material is more flexible than the first material. In some implementations, the first material is more rigid than the second material. In some implementations, the frame comprises an annular shape.

In some implementations, no portion of the catheter housing device is positioned between the first and second arms of the hub and the subject's skin when the catheter hub is secured by the first and second arms in use. In some implementations, no portion of the cover contacts the catheter hub when the catheter hub is secured by the first and second arms in use. In some implementations, no portion of the membrane contacts the catheter hub when the catheter hub is secured by the first and second arms in use.

In some implementations, the catheter hub is spaced inward from the perimeter of the opening when the catheter hub is secured by the first and second arms in use. In some implementations, the catheter hub comprises a first end connected to the catheter and a second end opposite the first end of the catheter hub, and wherein, when the catheter hub is secured by the first and second arms of the hub, the second end of the catheter hub does not contact the subject's skin. In some implementations, when the catheter hub is secured by the first and second arms of the hub, only the first end of the catheter hub contacts the subject's skin. In some implementations, when the catheter hub is secured by the first and second arms of the hub, no portion of the catheter hub contacts the subject's skin.

In some implementations, said second ends of the first and second arms are spaced above a plane extending along a bottom of the frame. In some implementations, the catheter housing device further comprises a moisture wicking element connected to at least one of the membrane and the frame and configured to contact the subject's skin and encircle the catheter insertion site when the membrane is secured to the subject's skin, said moisture wicking element configured to wick moisture away from the catheter insertion site when the catheter housing device is in use. In some implementations, said membrane comprises a plurality of apertures spaced around said opening. In some implementations, the catheter housing device further comprises one or more adhesive substrates coupled to a bottom surface of the membrane and configured to secure the membrane to the subject's skin. In some implementations, the membrane further comprises a plurality of recessed portions, each of the plurality of recessed portions positioned adjacent to one of the plurality of apertures of the membrane and recessed inward from a plane extending along the bottom surface of the membrane, said plurality of recessed portions configured to allow an adhesive removal liquid to flow under said bottom surface.

In some implementations, the cover comprises a top portion and a wall extending outward from the top portion and defining an interior. In some implementations, the wall of the cover is configured to removably secure to the frame of the hub. In some implementations, the frame comprises one or more latch legs and the wall of the cover comprises one or more latch protrusions configured to removably secure to the one or more latch legs of the frame. In some implementations, the one or more latch legs extend from the frame in a direction that is generally perpendicular relative to the first and second arms. In some implementations, the wall comprises at least one latch protrusion at a first end of the wall and at least one latch protrusion at a second end of the wall that is opposite the first end of the wall. In some implementations, the cover further comprises an opening extending through a portion of the wall, wherein the opening of the cover is configured to allow a fluid tube to pass through the wall and connect with the catheter hub. In some implementations, said fluid tube is connected to the catheter hub via a male luer connector. In some implementations, the frame is permanently secured to the membrane.

Disclosed herein is a catheter stabilization system comprising: a hub; a fluid tube assembly; and a cover. The hub is configured to secure to skin of a subject around a catheter insertion site, the hub comprising an opening configured to be positioned over the catheter insertion site, the hub further configured to secure a catheter hub connected to a catheter to minimize movement of the catheter when inserted in the catheter insertion site. The fluid tube assembly can be configured for delivering fluids to the subject, the fluid tube assembly comprising: a fluid tube having a first end and a second end opposite the first end; a catheter hub connector coupled to the first end of the fluid tube and configured to connect to the catheter hub to provide fluid communication between the fluid tube and the catheter connected to the catheter hub; and a fluid tube lock connector coupled to the fluid tube; and a cover comprising a cover opening configured to receive the fluid tube and a cover connector proximate the cover opening. The cover is configured to removably secure to the hub and enclose the catheter insertion site on the subject. When the fluid tube lock connector and the cover connector are disconnected from one another, movement of the fluid tube lock connector and the cover along the fluid tube is permitted. When the fluid tube lock connector and the cover connector are connected to one another, movement of the fluid tube lock connector and the cover along the fluid tube is inhibited.

In some implementations, the fluid tube assembly further comprises a sleeve coupled to the fluid tube, and wherein, when the fluid tube lock connector and the cover connector are connected to one another, movement of the fluid tube lock connector and the cover along the fluid tube is inhibited by said sleeve. In some implementations, when the fluid tube lock connector and the cover connector are connected to one another, pressure is applied to said sleeve. In some implementations, said sleeve comprises a tubular shape. In some implementations, said sleeve has a smaller length than said fluid tube.

In some implementations, the fluid tube lock connector and the cover connector are configured to threadingly connect to one another. In some implementations, the fluid tube lock connector and the cover connector are configured to connect to one another via a snap-fit engagement. In some implementations, said cover comprises a top portion and a wall extending outward from the top portion and defining an interior, and wherein said cover connector extends outward from the wall of the cover. In some implementations: said fluid tube lock connector comprises body having a threaded interior and an opening configured to receive the fluid tube; and said cover connector comprises a threaded portion configured to threadingly engage said threaded interior of said body. In some implementations: said fluid tube lock connector comprises a notch recessed from an outer surface of said body; and said cover connector further comprises a latch arm configured to snap into engagement with said notch when said threaded portion threadingly engages said threaded interior of said body.

In some implementations, the fluid tube assembly further comprises a stopper permanently secured to a portion of the fluid tube and configured to be positioned between the catheter hub connector and the cover opening, and wherein the stopper is configured to inhibit the fluid tube from being pulled out through said cover opening. In some implementations, the stopper comprises an annular shape extending around an entirety of a cross-section of said portion of the fluid tube. In some implementations, the stopper comprises a circular cross-section. In some implementations, said stopper is spaced from the catheter hub connector by a gap, thereby defining a flexible joint.

In some implementations: when the fluid tube lock connector and the cover connector are disconnected from one another, sliding of the fluid tube lock connector and the cover along the fluid tube is permitted; and when the fluid tube lock connector and the cover connector are connected to one another, sliding of the fluid tube lock connector and the cover along the fluid tube is inhibited. In some implementations, said catheter hub connector is a male luer connector. In some implementations, the wall of the cover is configured to removably connect to the hub.

In some implementations, the hub further comprises: a membrane configured to contact and secure to the subject's skin, said membrane comprising said opening configured to be positioned over the catheter insertion site; and at least one arm coupled with the membrane and extending partially across the opening, wherein the at least one arm is configured to be positioned above the subject's skin when the membrane is secured to the subject's skin, and wherein a free end of the at least one arm is spaced inward from a perimeter of the opening and is configured to secure said catheter hub connected to said catheter to minimize movement of the catheter when inserted in the catheter insertion site. In some implementations, said at least one arm comprises a pair of opposing arms, and wherein the free ends of the pair of opposing arms are spaced from one another by a gap and are configured to secure said catheter hub when the catheter housing device is in use.

In some implementations: the hub further comprises a frame connected to the membrane along at least a portion of the perimeter of the opening (for example, all of or less than an entirety of the perimeter of the opening). In some implementations, said at least one arm comprises: a first arm extending outward from a first portion of the frame and partially across the opening, the first arm comprising a connected end that is connected to the first portion of the frame and a free end that is opposite the connected end of the first arm; and a second arm extending outward from a second portion of the frame and partially across the opening towards the first arm, the second arm comprising a connected end that is connected to the second portion of the frame and a free end that is opposite said connected end of the second arm; and the free ends of the first and second arms are: spaced inward from the perimeter of the opening and spaced from one another by a gap; and configured to secure said catheter hub when the catheter housing device is in use. In some implementations, said free ends of the first and second arms are configured to secure said catheter hub in an inclined position relative to the subject's skin when in use. In some implementations, said free ends of the first and second arms are spaced above a plane extending along a bottom surface of the membrane. In some implementations, each of the first and second arms comprise a generally T-shaped structure having a stem connected to the first or second portions of the frame and a flange connected to the stem and spaced away from said frame, and wherein at least a portion of said flange is curved to conform to and surround a portion of the catheter hub when the catheter hub is secured by the first and second arms. In some implementations, each of the first and second arms comprises a first section extending outward from the frame and a second section connected to the first section at the free end and configured to contact the catheter hub when the catheter hub is secured by the free ends of the first and second arms. In some implementations, the second section is transverse relative to the first section. In some implementations, the second section is at least partially curved to conform to and surround a portion of the catheter hub when the catheter hub is secured by the free ends of the first and second arms. In some implementations, surfaces of the second sections of the first and second arms that face in a direction toward one another comprise matching curvatures.

Disclosed herein is a method (not claimed) of stabilizing a catheter hub connected to a catheter inserted at a catheter insertion site on a subject to minimize movement of the catheter, the method comprising: obtaining a hub comprising; a membrane having an opening; a frame connected to the membrane along a perimeter of the opening; a first arm extending outward from a first portion of the frame and partially across the opening; and a second arm extending outward from a second portion of the frame and partially across the opening towards the first arm; positioning the opening of the membrane over the catheter insertion site and securing the membrane to the subject's skin; securing the catheter hub with the first and second arms of the hub; and enclosing the catheter insertion site by securing a cover to the hub.

In some implementations, the hub further comprises at least one placement indicator, and wherein said positioning the opening of the membrane over the catheter insertion site further comprises aligning said at least one placement indicator with said catheter insertion site. In some implementations, said hub comprises two placement indicators, and wherein said positioning the opening of the membrane over the catheter insertion site further comprises aligning an axis extending between said two placement indicators with the catheter insertion site. In some implementations, said at least one placement indicator extends outward from the frame.

In some implementations, the method further comprises securing a male luer connector to the catheter hub to provide fluid communication between the catheter hub and a fluid tube coupled with the male luer connector. In some implementations, said enclosing the catheter insertion site by securing said cover to the hub occurs after said securing said male luer connector to the catheter hub. In some implementations, said cover comprises a cover opening that receives the fluid tube, and wherein the method further comprises moving the cover along said fluid tube towards the hub prior to securing the cover to the hub.

In some implementations, the cover further comprises a cover connector proximate the cover opening, and wherein the method further comprises securing a fluid tube lock connector coupled with the fluid tube to the cover connector to inhibit the fluid tube lock connector and the cover from moving along said fluid tube. In some implementations, a sleeve is positioned around said fluid tube and within at least a portion of said fluid tube lock connector, and wherein said securing the fluid tube lock connector to the cover connector causes said sleeve to grip said fluid tube to inhibit the fluid tube lock connector and the cover from moving along said fluid tube. In some implementations: said fluid tube lock connector comprises body having a threaded interior and an opening positioned around the fluid tube; said cover connector comprises a threaded portion; and said securing the fluid tube lock connector to the cover connector comprises engaging said threaded portion of the cover connector with the threaded interior of the body of the fluid tube lock connector. In some implementations: said fluid tube lock connector comprises a notch recessed from an outer surface of said body; and said cover connector further comprises a latch arm; and said securing the fluid tube lock connector to the cover connector further comprises engaging said threaded portion of the cover connector with the threaded interior of the body of the fluid tube lock connector until said latch arm snaps into engagement with said notch.

In some implementations, said cover comprises a top portion and a wall extending outward from the top portion and defining an interior, said wall comprising said cover opening, and wherein said securing said cover to the hub comprises securing the wall of the cover to the frame of the hub. In some implementations, the frame comprises one or more latch legs and the wall of the cover comprises one or more latch protrusions, and wherein said securing the wall of the cover to the frame of the hub comprises engaging the one or more latch legs and the one or more latch protrusions.

In some implementations, said securing the membrane to the subject's skin comprises securing the membrane to the subject's skin with an adhesive layer disposed on a bottom surface of the membrane. In some implementations, the method further comprises removing a release liner from an adhesive layer disposed on a bottom surface of said membrane prior to securing the membrane to the subject's skin. In some implementations, the method further comprises: removing a first portion of a release liner from a first portion of an adhesive layer disposed on a bottom surface of said membrane; securing said first portion of the adhesive layer to the subject's skin; removing a second portion of said release liner from a second portion of the adhesive layer; and securing said second portion of the adhesive layer to the subject's skin.

In some implementations: the first arm comprises a first end that is connected to the first portion of the frame and a second end that is opposite the first end of the first arm; the second arm comprises a first end that is connected to the second portion of the frame and a second end that is opposite said first end of the second arm; the second ends of the first and second arms are spaced inward from the perimeter of the opening and spaced from one another by a gap; said securing the catheter hub with the first and second arms of the hub comprises positioning the catheter hub between the second ends of the first and second arms. In some implementations, the second portion of the frame is opposite the first portion of the frame. In some implementations, said securing the catheter hub with the first and second arms of the hub comprises securing the catheter hub in an inclined position relative to the subject's skin. In some implementations, said second ends of the first and second arms are spaced above a plane extending along a bottom surface of the membrane.

In some implementations: each of the first and second arms comprises a first section extending outward from the frame and a second section connected to the first section at the second end and configured to contact the catheter hub when the catheter hub is secured by the second ends of the first and second arms; the second section is at least partially curved; and said securing the catheter hub with the first and second arms of the hub comprises securing the catheter hub between the at least partially curved second sections of the first and second arms.

In some implementations, said securing the catheter hub with the first and second arms of the hub comprises securing the catheter hub with the first and second arms such that no portion of the hub is positioned between the first and second arms and the subject's skin. In some implementations, no portion of the cover contacts the catheter hub when the catheter hub is secured by the first and second arms of the hub. In some implementations, no portion of the membrane contacts the catheter hub when the catheter hub is secured by the first and second arms of the hub.

In some implementations, said securing the catheter hub with the first and second arms of the hub comprises securing the catheter hub with the first and second arms such that the catheter hub is spaced inward from the perimeter of the opening. In some implementations: the catheter hub comprises a first end connected to the catheter and a second end opposite the first end of the catheter hub; and said securing the catheter hub with the first and second arms of the hub comprises securing the catheter hub with the first and second arms such that the second end of the catheter hub does not contact the subject's skin. In some implementations: said securing the catheter hub with the first and second arms of the hub further comprises securing the catheter hub with the first and second arms such that only the first end of the catheter hub contacts the subject's skin. In some implementations: the catheter hub comprises a first end connected to the catheter and a second end opposite the first end of the catheter hub; and said securing the catheter hub with the first and second arms of the hub comprises securing the catheter hub with the first and second arms such that no portion of the catheter hub contacts the subject's skin.

In some implementations, said membrane comprises: a plurality of apertures spaced around said opening; and a bottom surface and an adhesive layer disposed on said bottom surface; a plurality of recessed portions, each of the plurality of recessed portions positioned adjacent to one of the plurality of apertures and recessed inward from a plane extending along the bottom surface of the membrane. The method can further comprise allowing an adhesive removal liquid to flow through the plurality of apertures and along the subject's skin underneath said plurality of recessed portions. In some implementations, said adhesive removal liquid comprises alcohol. In some implementations, the method further comprises removing at least a portion of the bottom surface of the membrane from the subject's skin after said step of allowing said adhesive removal liquid to flow through the plurality of apertures and along the subject's skin underneath said plurality of recessed portions.

Disclosed herein is a fluid tube locking system for use with a fluid tube, the system comprising: a first connector configured to be coupled to the fluid tube; a second connector configured to be coupled to the fluid tube and further configured to removably connect to the first connector; wherein: when the first and second connectors are disconnected from one another, movement of at least one of the first and second connectors relative to the fluid tube is permitted; and when the first and second connectors are connected to one another, movement of the first and second connectors relative to the fluid tube is inhibited.

In some implementations, said first and second connectors are configured to threadingly connect to one another. In some implementations, said first and second connectors are configured to connect to one another via a snap-fit engagement. In some implementations: said first connector comprises a body and an opening configured to receive said fluid tube, said body having a threaded interior; and_said second connector comprises a threaded portion configured to threadingly engage said threaded interior of said body of the first connector. In some implementations: said first connector comprises a notch recessed from an outer surface of said body; and said second connector further comprises a latch arm configured to snap into engagement with said notch when said threaded portion of the second connector threadingly engages said threaded interior of said body. In some implementations, the fluid tube locking system further comprises a sleeve configured to be coupled to the fluid tube, and wherein, when the first and second connectors are connected to one another, movement of the first and second connectors relative to the fluid tube is inhibited by said sleeve.

In some implementations, when the first and second connectors are connected to one another, pressure is applied to said sleeve. In some implementations, said sleeve is operably positioned by the first connector. In some implementations, said sleeve is positioned within an interior of a body of said first connector. In some implementations, said sleeve comprises a tubular shape. In some implementations, said sleeve has a smaller length than said fluid tube. In some implementations, the fluid tube locking system further comprises said fluid tube. In some implementations: when the first and second connectors are disconnected from one another, sliding of the first and second connectors along the fluid tube is permitted; and when the first and second connectors are connected to one another, sliding of the first and second connectors along the fluid tube is inhibited.

In some implementations, the fluid tube locking system further comprises a cover configured to be placed over a catheter insertion site on a subject to enclose the catheter insertion site, wherein said cover comprises said second connector. In some implementations, said cover comprises a top portion and a wall extending outward from the top portion and defining an interior, and wherein said second connector extends outward from the wall of the cover. In some implementations, said cover comprises an opening in the wall that is configured to receive said fluid tube.

Disclosed herein is a catheter housing configured to surround a catheter insertion site on a user, the catheter housing comprising: a hub; a cover; and an extension set. The hub is configured for placement on the user around the catheter insertion site and comprises a membrane configured to secure to the user's skin, the membrane having an opening configured to surround the catheter insertion site when the catheter housing is in use. The cover is configured to at least partially enclose the catheter insertion site and comprises: a top portion and a wall extending transverse with respect to the top portion and defining a perimeter of the cover; and an opening extending through a portion of the wall. The extension set is configured to secure to the cover and a catheter device coupled to a catheter to facilitate fluid communication between the catheter and a tube of the extension set. The extension set comprises: the tube, wherein the tube is configured to be received through the opening of the cover, wherein the tube comprises a first end and a second end opposite the first end; a first connector configured to secure to the first end of the tube and to a portion of the catheter device; and a second connector positioned along the tube and configured to engage a portion of the cover at the opening to secure the extension set to the cover.

In some implementations, the cover and the hub are permanently bonded together. In some implementations, at least one of the cover and hub are configured to slide along the tube when the second connector secures the extension set to the cover. In some implementations, the catheter housing further comprises a third connector, wherein the second and third connectors are configured to secure to one another and sandwich the portion of the cover around the opening. In some implementations, the cover further comprises a rim protruding outward from the wall around the opening, and wherein the second connector is configured to engage the rim of the cover to secure the extension set to the cover. In some implementations, the second connector comprises a flange and a stem, wherein the stem comprises a smaller cross-section than the flange and is configured to extend through at least a portion of the opening of the cover, wherein the flange is configured to contact the cover when the stem is positioned at least partially through the opening of the cover. In some implementations, the extension set further comprises a third connector configured to secure to the stem of the second connector. In some implementations, when the third connector is secured to the stem of the second connector, the second and third connectors sandwich the portion of the cover around the opening. In some implementations, the second connector further comprises a body connected to and positioned between the stem and the flange, the body comprising a smaller cross-section than the flange and further comprising one or more protrusions extending outward from a surface of the body, and wherein the cover further comprises a rim protruding outward from the wall around the opening, the rim comprising one or more notches configured to allow the one or more protrusions to pass therethrough. In some implementations, the second connector comprises an opening extending through a center of the second connector, the opening of the second connector configured to receive the tube. In some implementations, the cover further comprises a bridge extending outward from an interior surface of the top portion of the cover, the bridge configured to surround a portion of the first connector. In some implementations, the bridge is configured to surround less than an entire perimeter of a cross-section of the first connector. In some implementations, the first connector is a male luer connector.

While certain aspects, advantages and novel features of embodiments of the invention are described herein, it is to be understood that not necessarily all such advantages can be achieved in accordance with any particular embodiment of the invention disclosed herein. Thus, the invention disclosed herein can be embodied or carried out in a manner that achieves or selects one advantage or group of advantages as taught herein without necessarily achieving other advantages as can be taught or suggested herein.

### BRIEF DESCRIPTION OF THE DRAWINGS

Certain features of this disclosure are described below with reference to the drawings. The illustrated embodiments are intended to illustrate, but not to limit the embodiments. Various features of the different disclosed embodiments can be combined to form further embodiments, which are part of this disclosure.
FIGS. 1A-1B illustrate top perspective views of a catheter housing device in accordance with aspects of this disclosure.
FIG. 1C illustrates a bottom perspective view of the catheter housing device of FIGS. 1A-1B in accordance with aspects of this disclosure.
FIG. 1D illustrates another top perspective view of the catheter housing device of FIGS. 1A-1B where a cover of the catheter housing device is shown as transparent in accordance with aspects of this disclosure.
FIG. 1E illustrates a partial cross-sectional view of the catheter housing device of FIGS. 1A-1B in use on a subject's skin in accordance with aspects of this disclosure.
FIGS. 1F-1G illustrate top perspective views of a cover of the catheter housing device of FIGS. 1A-1B in accordance with aspects of this disclosure.
FIGS. 1H-1I illustrate top and bottom views of the cover of FIGS. 1F-1G in accordance with aspects of this disclosure.
FIGS. 1J-1K illustrate side views of the cover of FIGS. 1F-1G in accordance with aspects of this disclosure.
FIGS. 1L-1M illustrate back and front views of the cover of FIGS. 1F-1G in accordance with aspects of this disclosure.
FIG. 1N illustrates a cross-section taken through the cover of FIGS. 1F-1G in accordance with aspects of this disclosure.
FIGS. 1O-1P illustrate top and bottom perspective views of a hub of the catheter housing device of FIGS. 1A-1B in accordance with aspects of this disclosure.
FIGS. 1Q-1T illustrate an extension set, and portions thereof, of the catheter housing device of FIGS. 1A-1B in accordance with aspects of this disclosure.
FIGS. 1U-1X, 1Y, and 1Z illustrate alternative implementations of covers for a catheter housing device in accordance with aspects of this disclosure.
FIGS. 2A-2C illustrate perspective views of a catheter housing device in accordance with aspects of this disclosure.
FIGS. 2D-2E illustrate top and bottom views of the catheter housing device of FIGS. 2A-2C in accordance with aspects of this disclosure.
FIG. 2F illustrates a cross-sectional view of the catheter housing device of FIGS. 2A-2C in use on a subject's skin in accordance with aspects of this disclosure.
FIG. 2G illustrates an exploded view of the catheter housing device of FIGS. 2A-2C in accordance with aspects of this disclosure.
FIGS. 2H-2Q illustrate views of a cover of the catheter housing device of FIGS. 2A-2C in accordance with aspects of this disclosure.
FIG. 2R illustrates a perspective view of an extension set of the catheter housing device of FIGS. 2A-2C in accordance with aspects of this disclosure.
FIGS. 2S-2X illustrate views of a hub of the catheter housing device of FIGS. 2A-2C in accordance with aspects of this disclosure.
FIG. 2Y illustrates an exploded view of the hub of FIGS. 2S-2X in accordance with aspects of this disclosure.
FIGS. 2Z-2AA illustrate cross-sectional views taken through the hub of FIGS. 2S-2X in accordance with aspects of this disclosure.

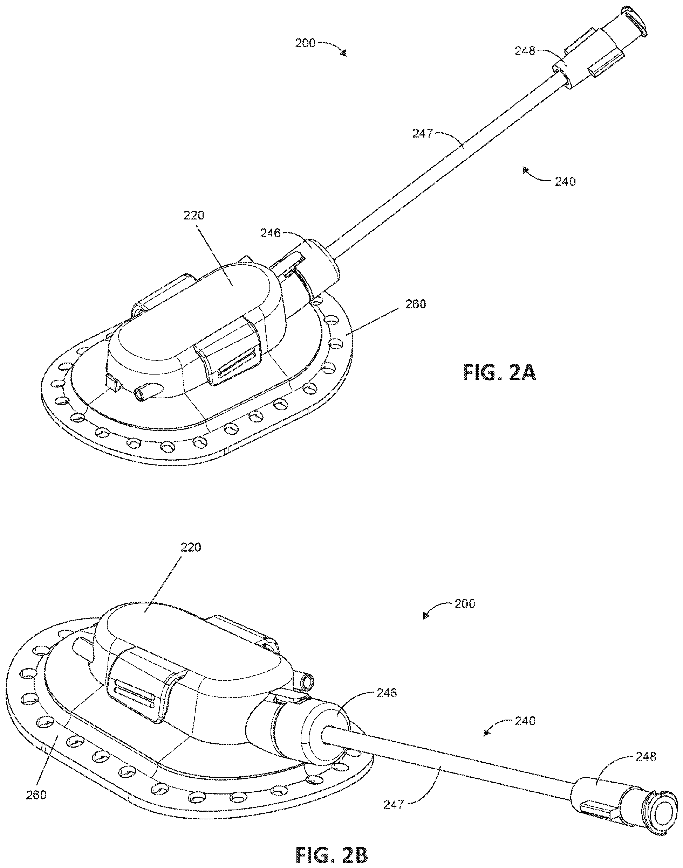

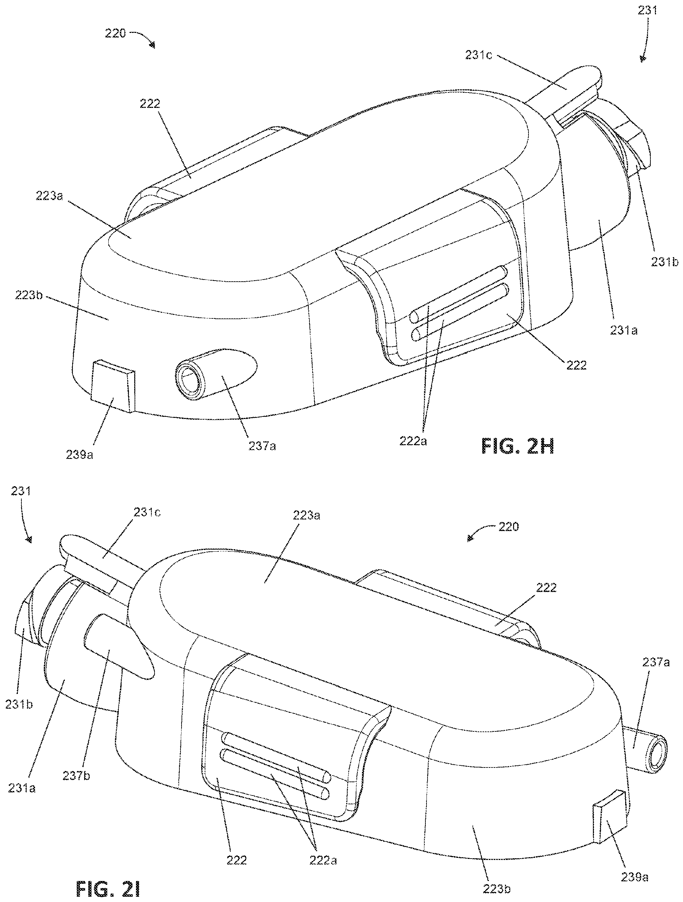

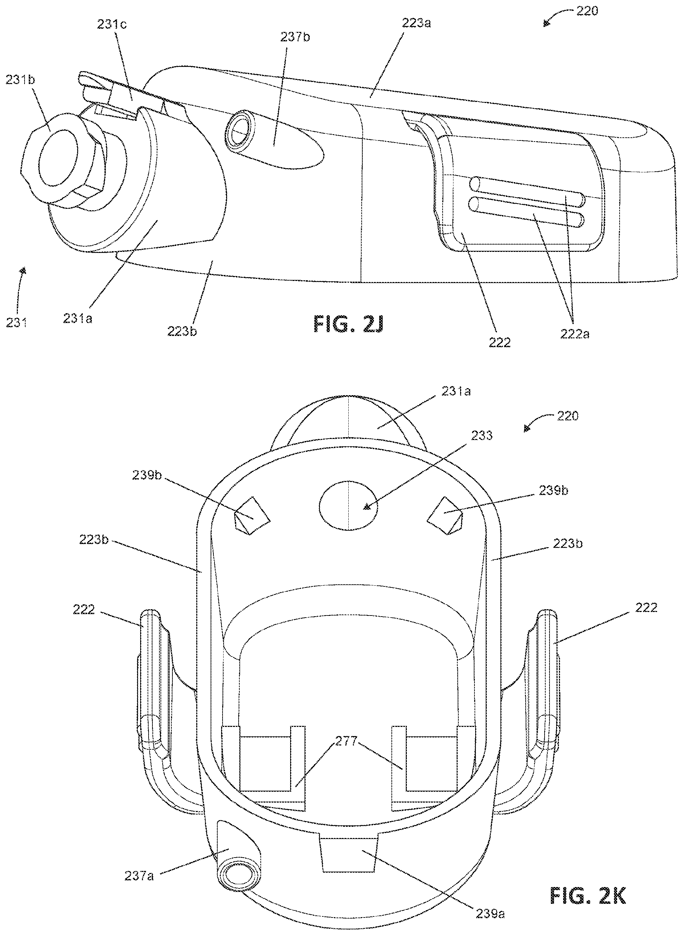

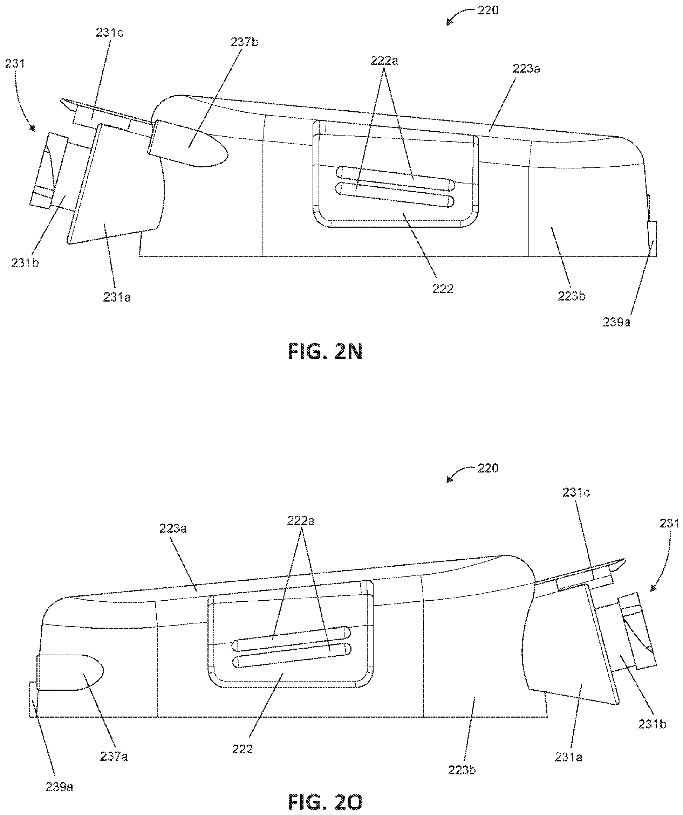

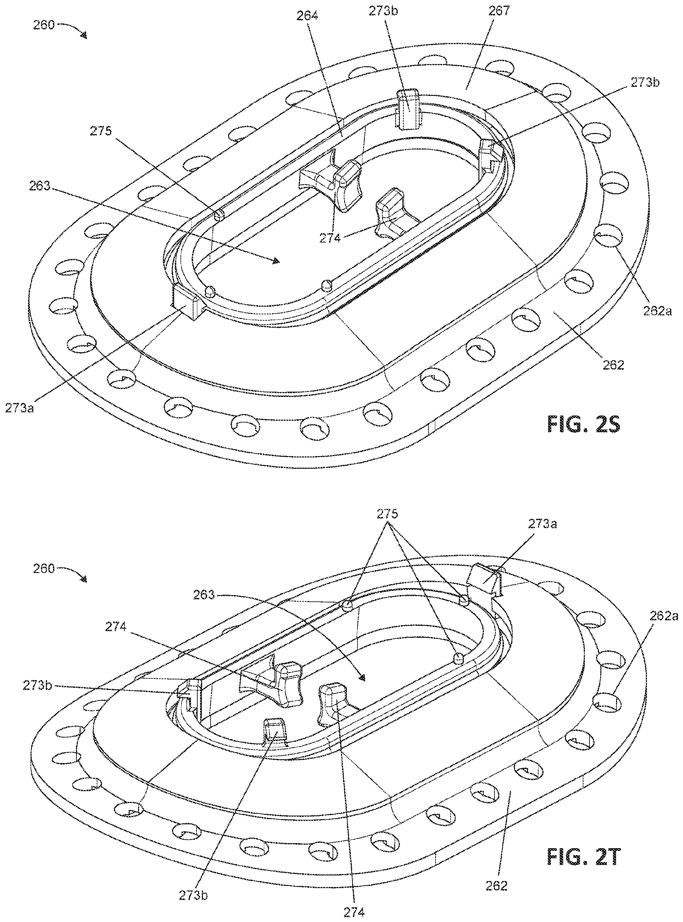

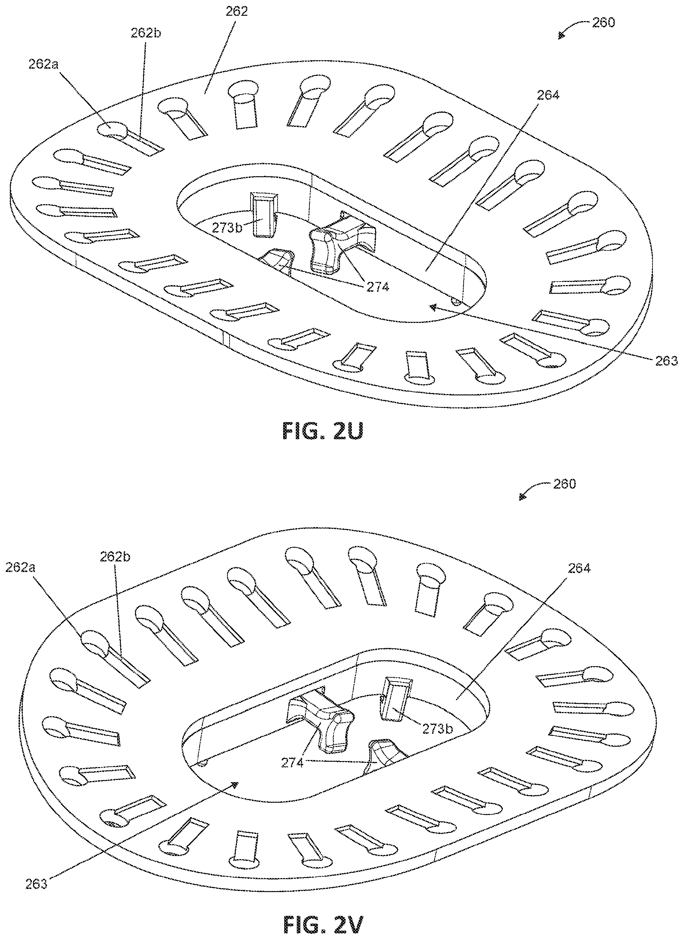

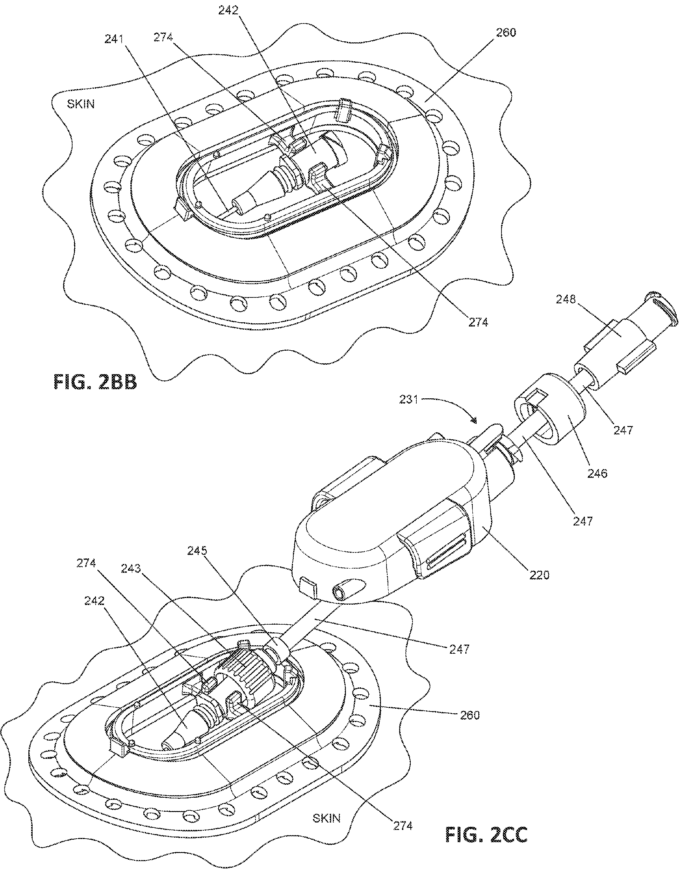

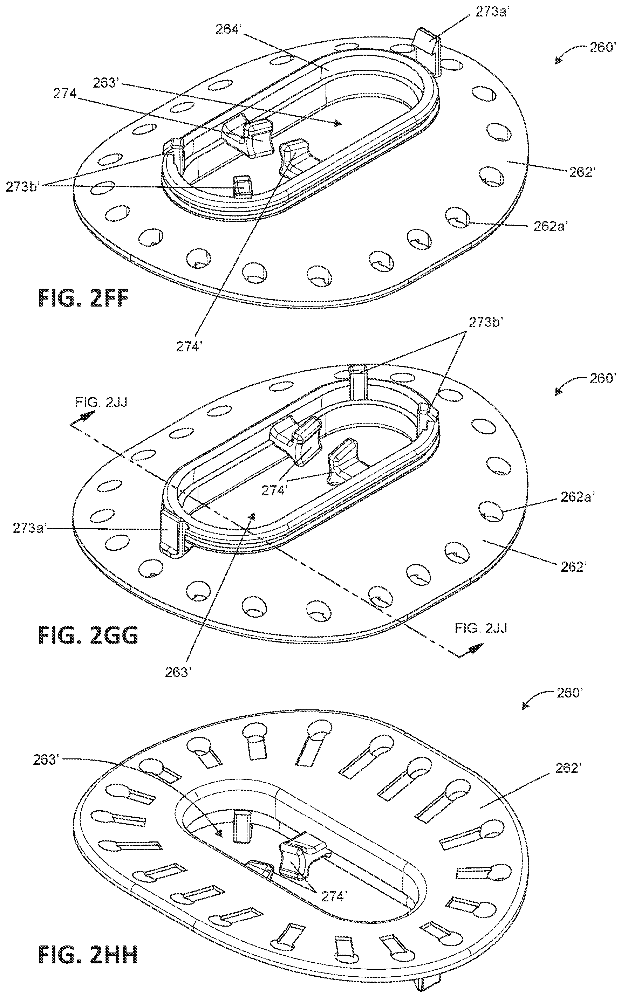

FIGS. 2BB-2EE show an illustrative method of using the catheter housing device of FIGS. 2A-2C in accordance with aspects of this disclosure.
FIGS. 2FF-2II illustrate an alternative implementation for a hub for the catheter housing device of FIGS. 2A-2C in accordance with aspects of this disclosure.

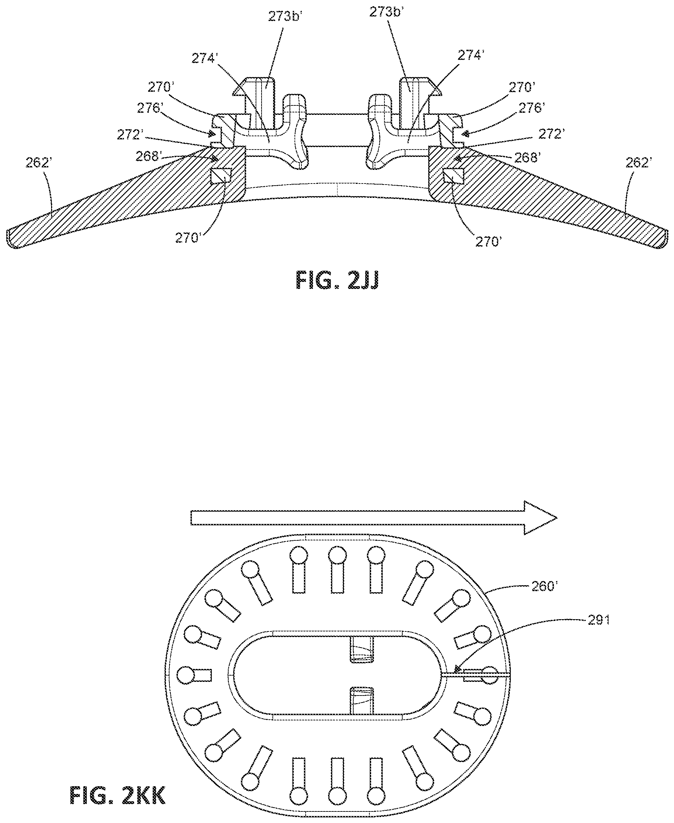

FIG. 2JJ illustrates a cross-section taken through the hub of FIGS. 2FF-2II in accordance with aspects of this disclosure.
FIGS. 2KK-2MM show illustrative implementations of one or more release liners for catheter housing devices in accordance with aspects of this disclosure.

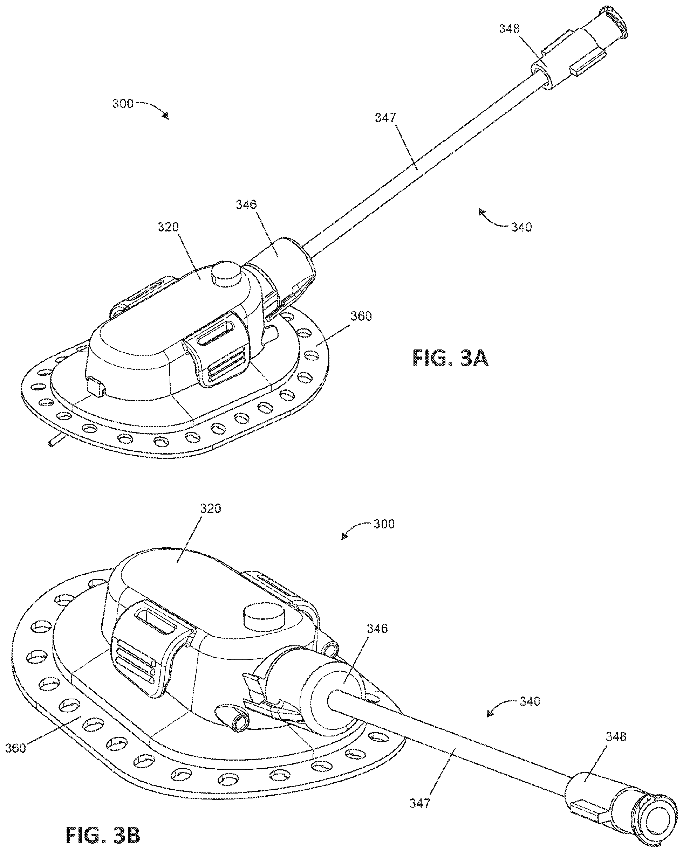

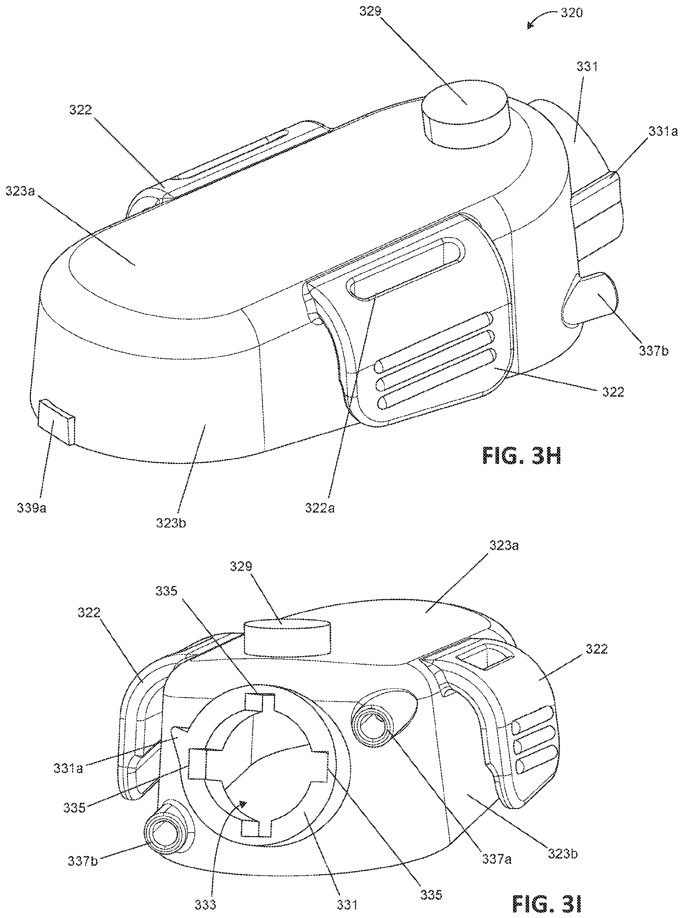

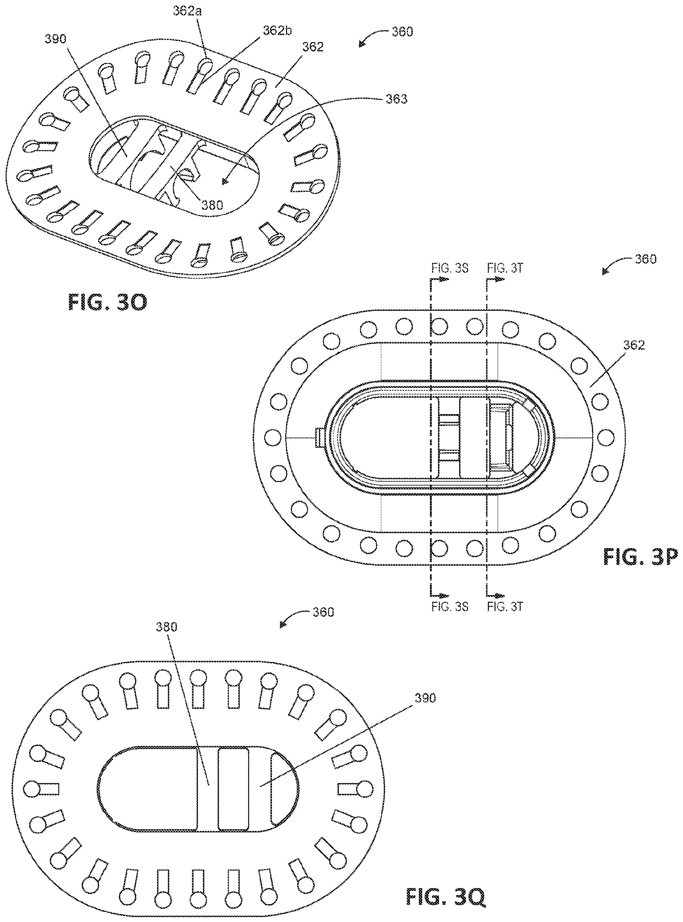

FIGS. 3A-3C illustrate perspective views of a catheter housing device in accordance with aspects of this disclosure.

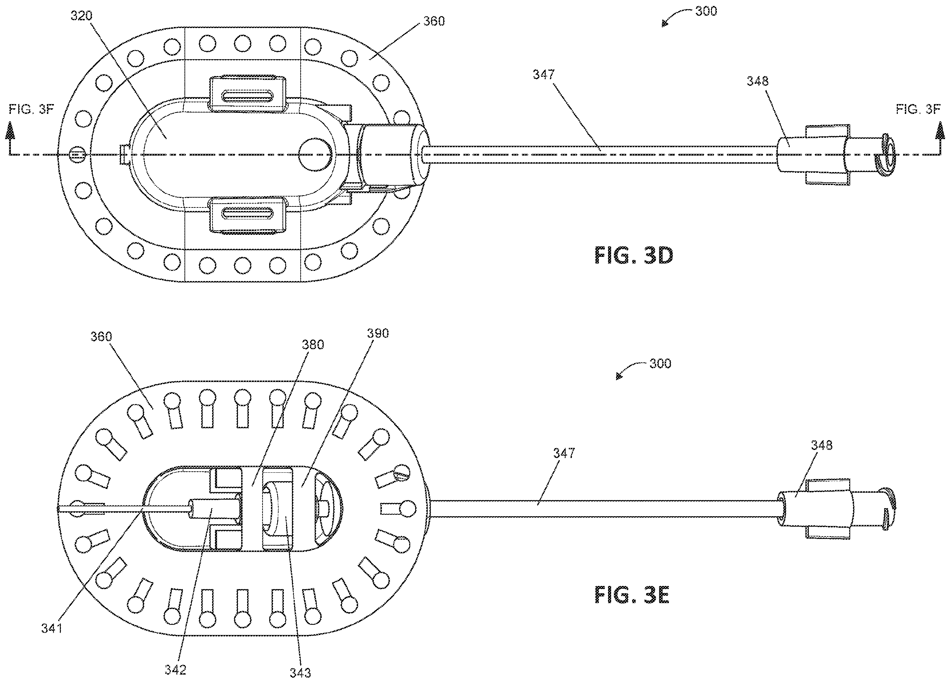

FIGS. 3D-3E illustrate top and bottom views of the catheter housing device of FIGS. 3A-3C in accordance with aspects of this disclosure.

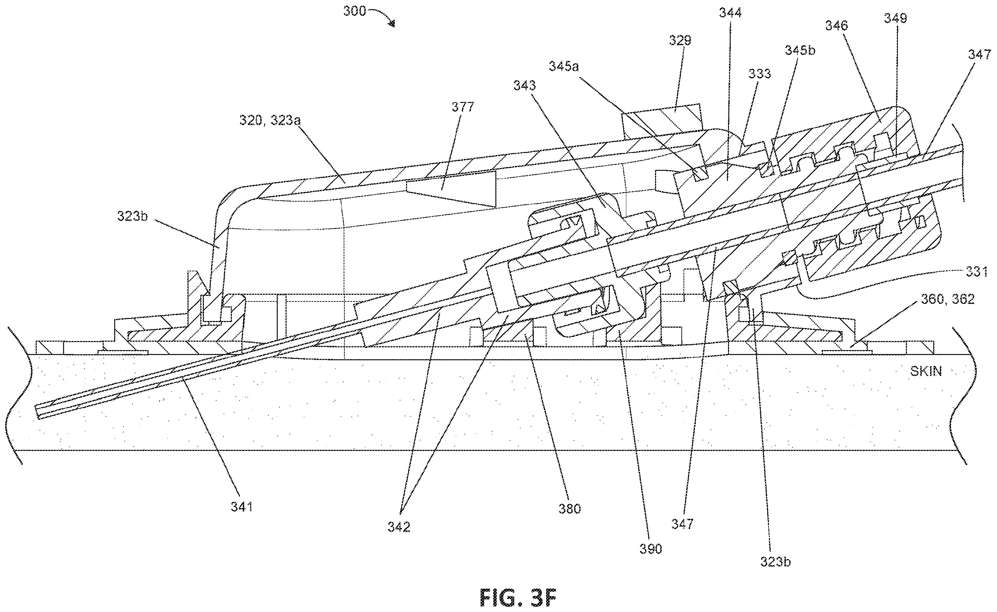

FIG. 3F illustrates a cross-sectional view of the catheter housing device of FIGS. 3A-3C in use on a subject's skin in accordance with aspects of this disclosure.

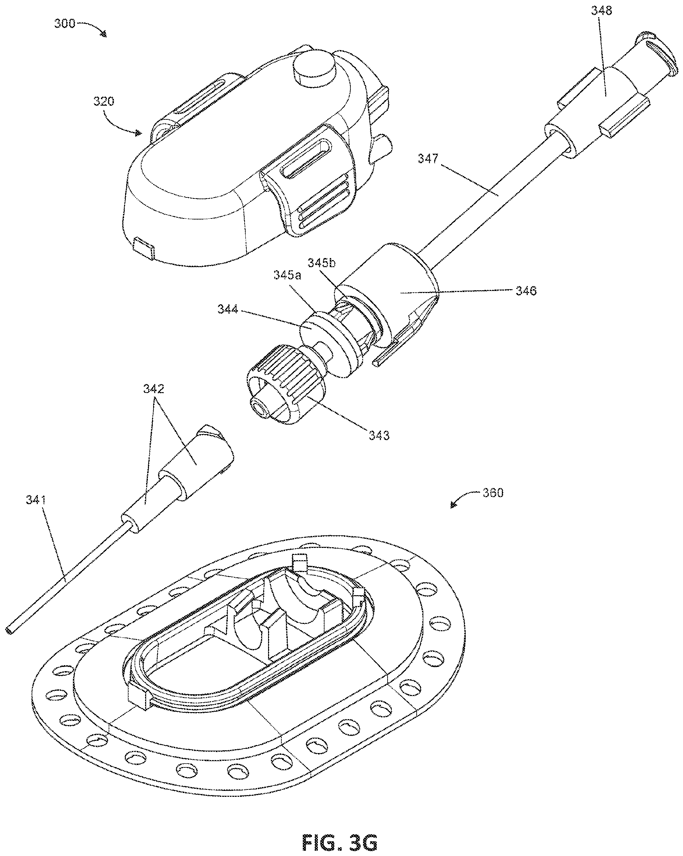

FIG. 3G illustrates an exploded view of the catheter housing device of FIGS. 2A-2C in accordance with aspects of this disclosure.
FIGS. 3H-3K illustrate views of a cover of the catheter housing device of FIGS. 3A-3C in accordance with aspects of this disclosure.

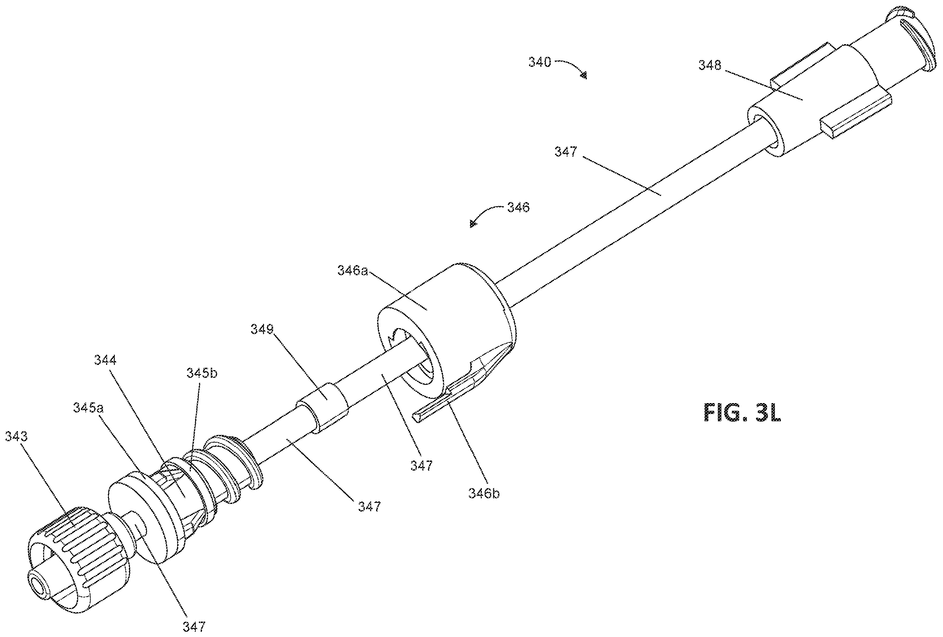

FIG. 3L illustrates a perspective view of an extension set of the catheter housing device of FIGS. 3A-3C in accordance with aspects of this disclosure.

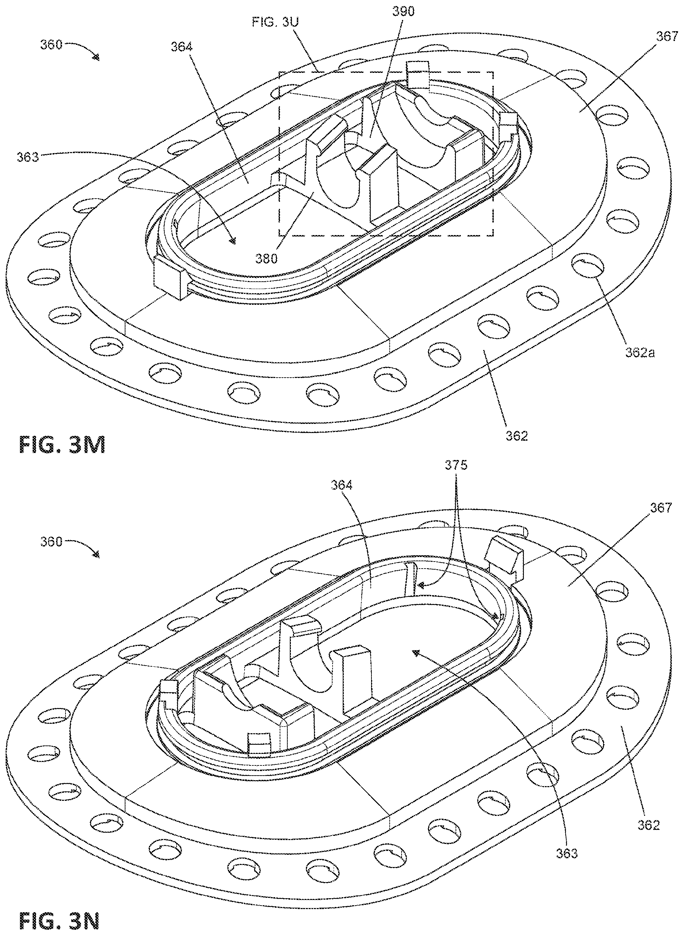

FIGS. 3M-3Q illustrate views of a hub of the catheter housing device of FIGS. 3A-3C in accordance with aspects of this disclosure.

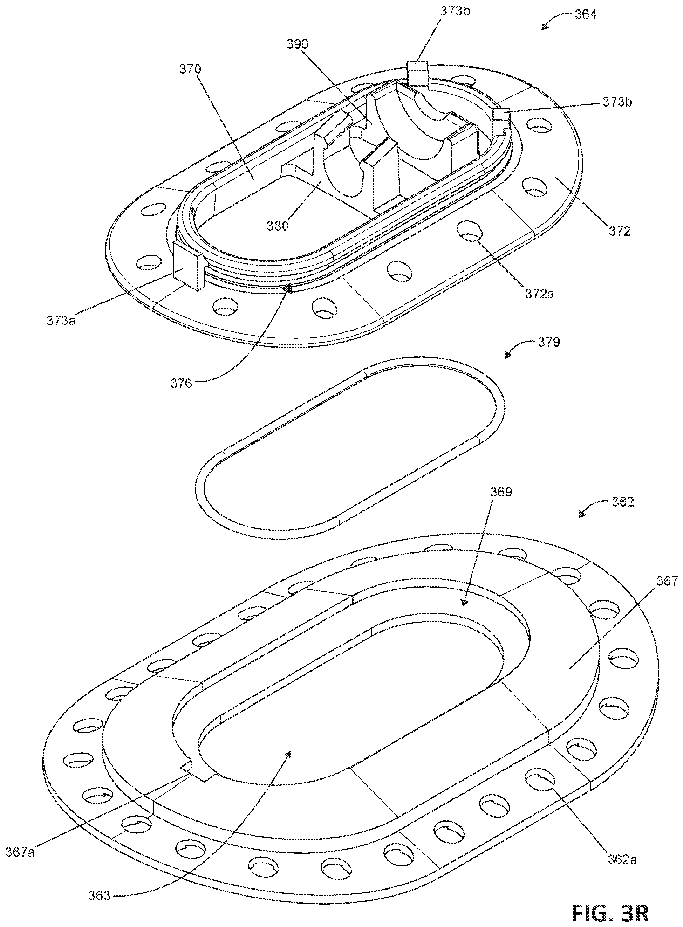

FIG. 3R illustrates an exploded view of the hub of FIGS. 3M-3Q in accordance with aspects of this disclosure.

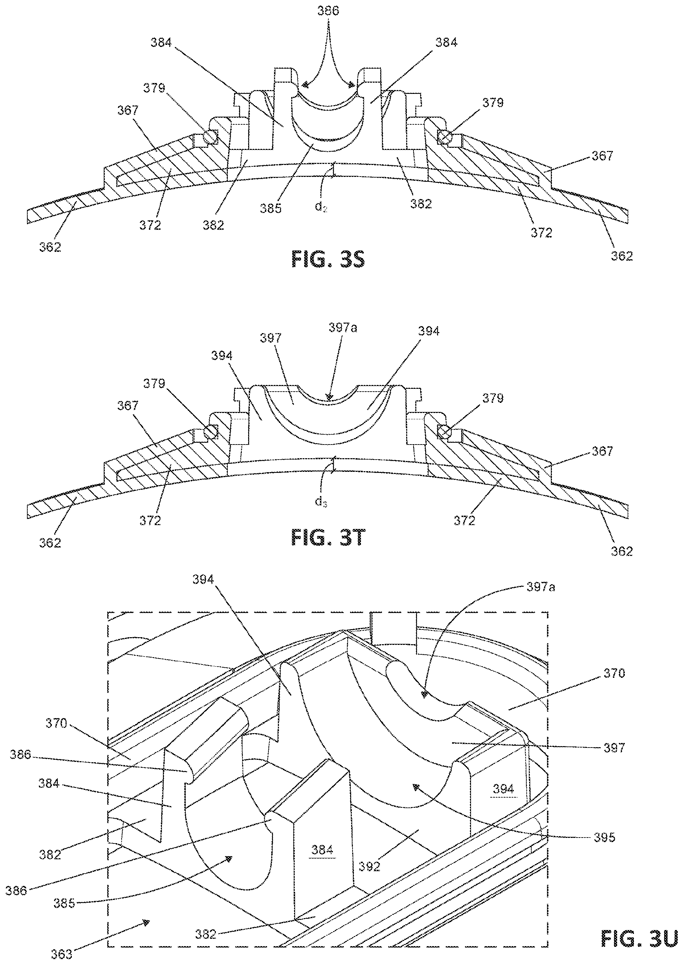

FIGS. 3S-3T illustrate cross-sectional views taken through the hub of FIGS. 3M-3Q in accordance with aspects of this disclosure.
FIG. 3U illustrates an enlarged view of a portion of the hub shown in FIG. 3M in accordance with aspects of this disclosure.

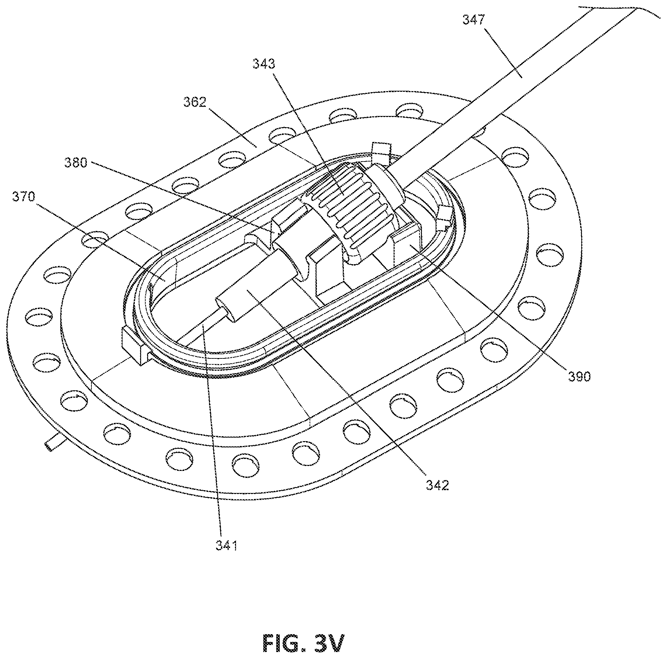

FIG. 3V illustrates a catheter, catheter hub, male luer connector, and a tube secured by the hub of FIGS. 3M-3Q in accordance with aspects of this disclosure.

### DETAILED DESCRIPTION

The present invention is defined in claim 1 while preferred embodiments are set forth in the dependent claims.

Associated surgical methods are also described herein to aid understanding the invention.

These methods do not form part of the invention.

Various features and advantages of the disclosed technology will become more fully apparent from the following description of the several specific embodiments illustrated in the figures. These embodiments are intended to illustrate the principles of this disclosure.

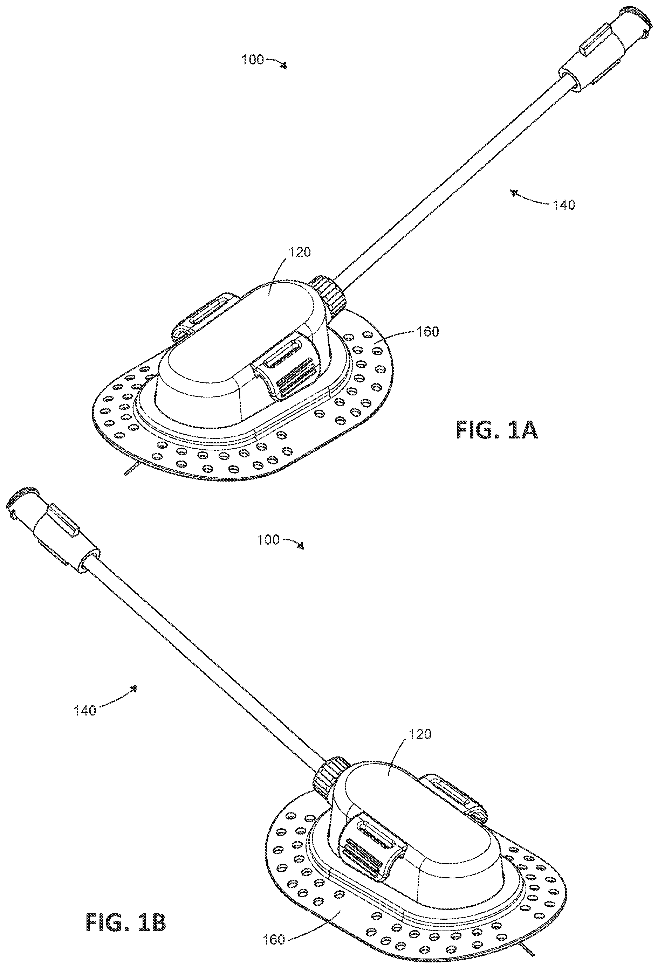

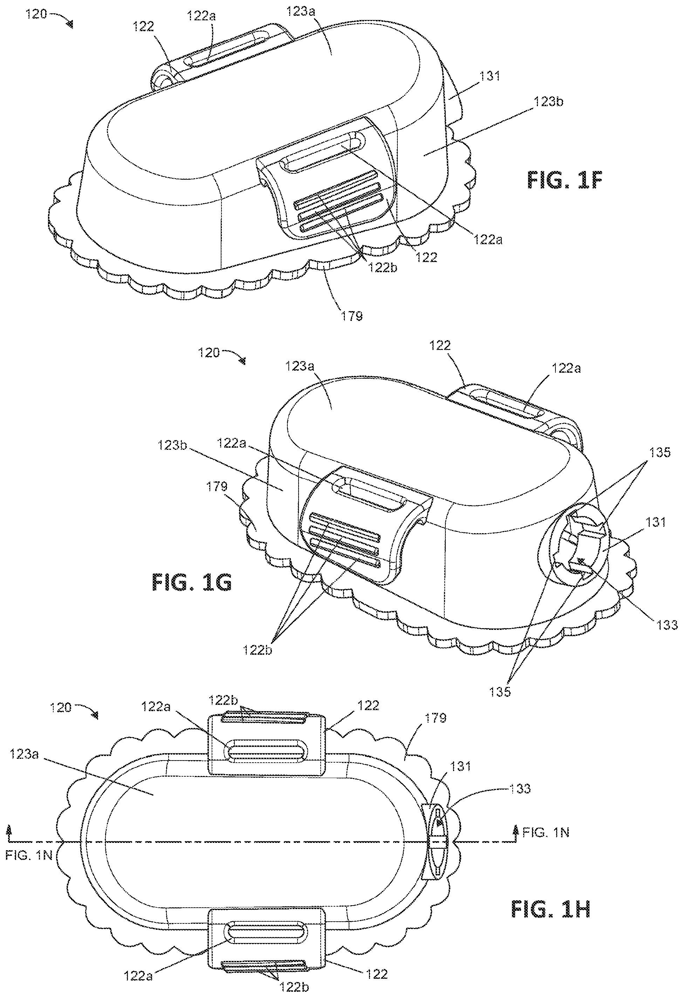

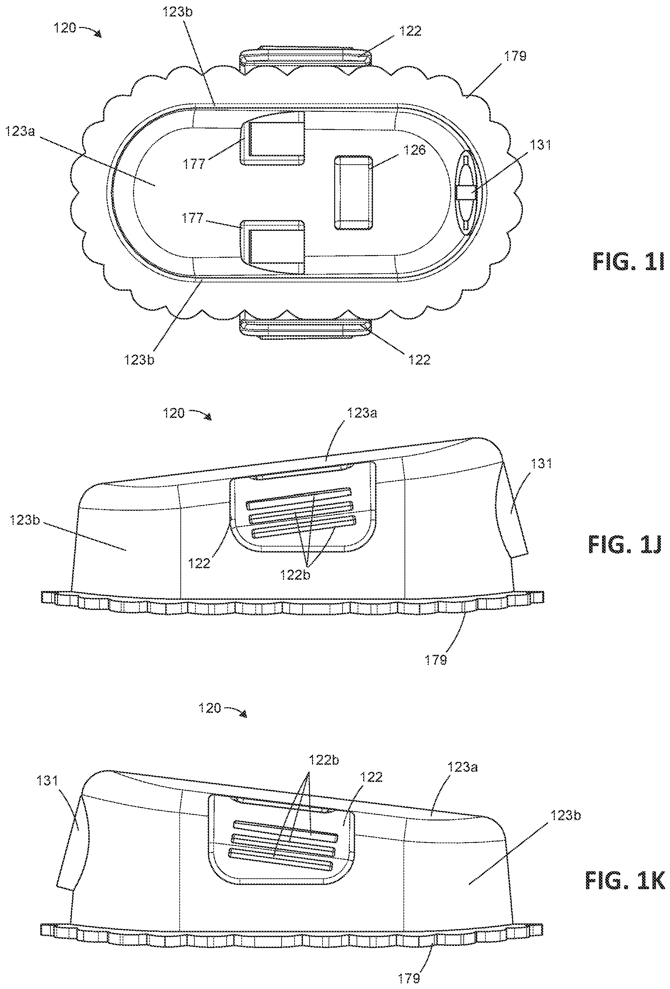

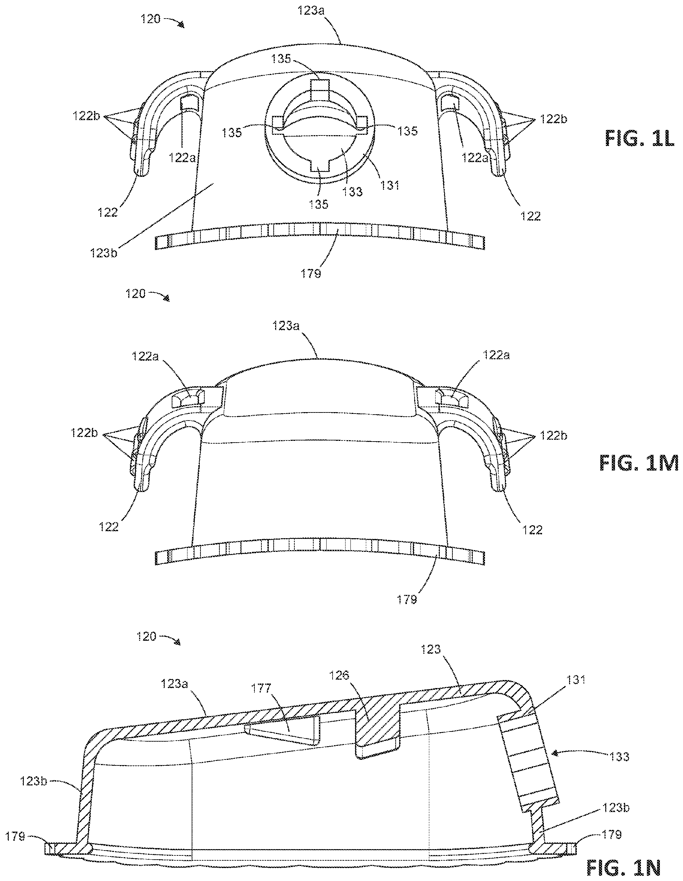

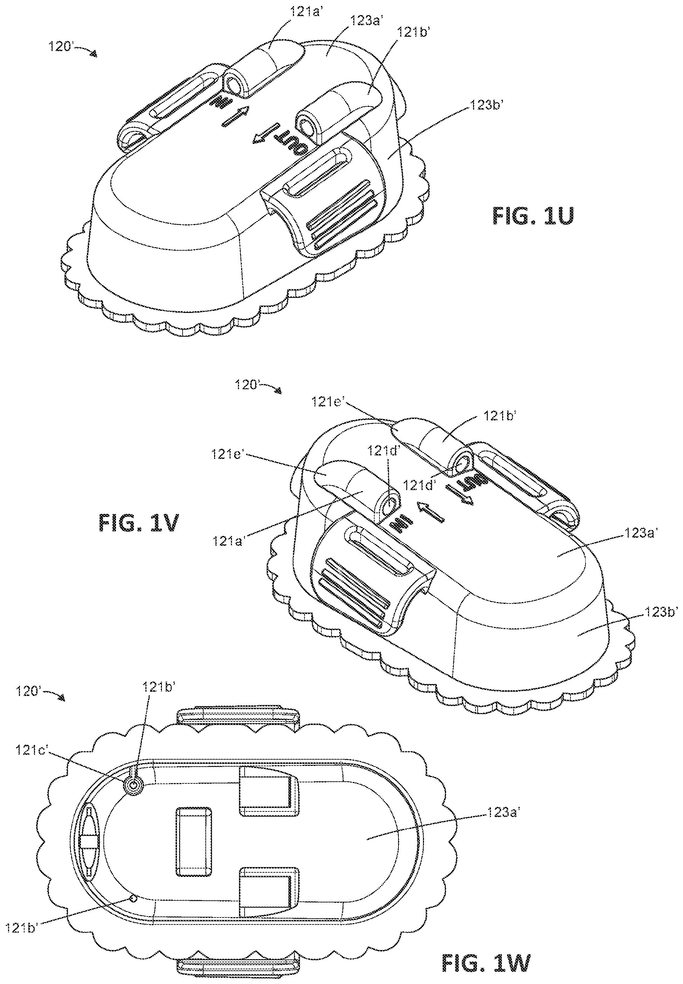

FIGS. 1A-1T illustrate a catheter housing 100 (which may also be referred to as a "catheter housing device", "catheter stabilization device", or "catheter stabilization system") and various aspects and/or portions thereof as further described below. Catheter housing 100 or portions thereof can be similar or identical in some or many respects to any of the catheter housings (or portions thereof) discussed in jointly owned U.S. Pat. Pub. No. 2019/0388 652.

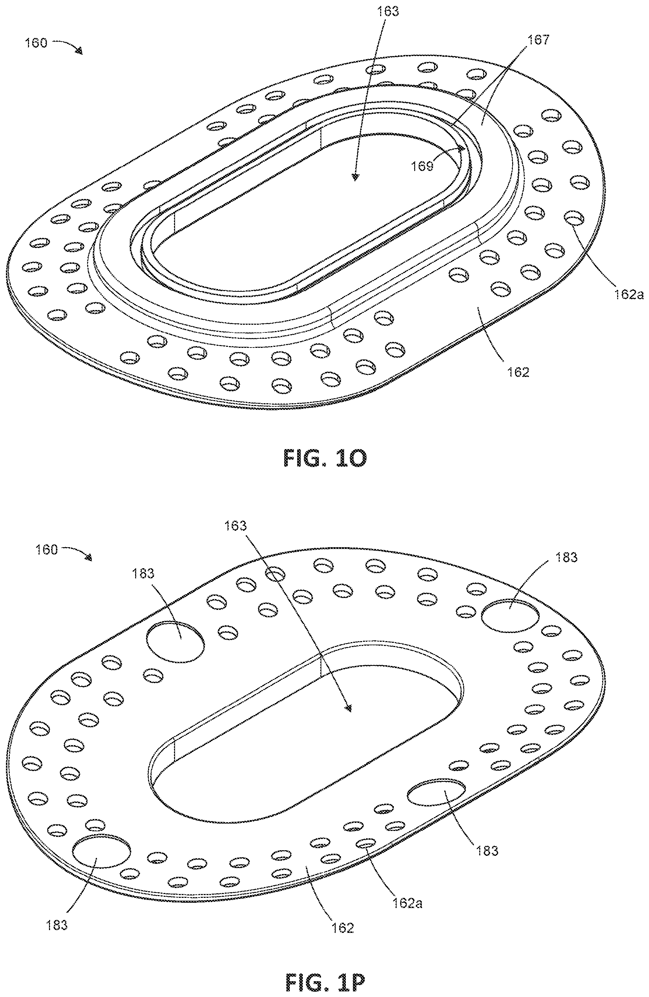

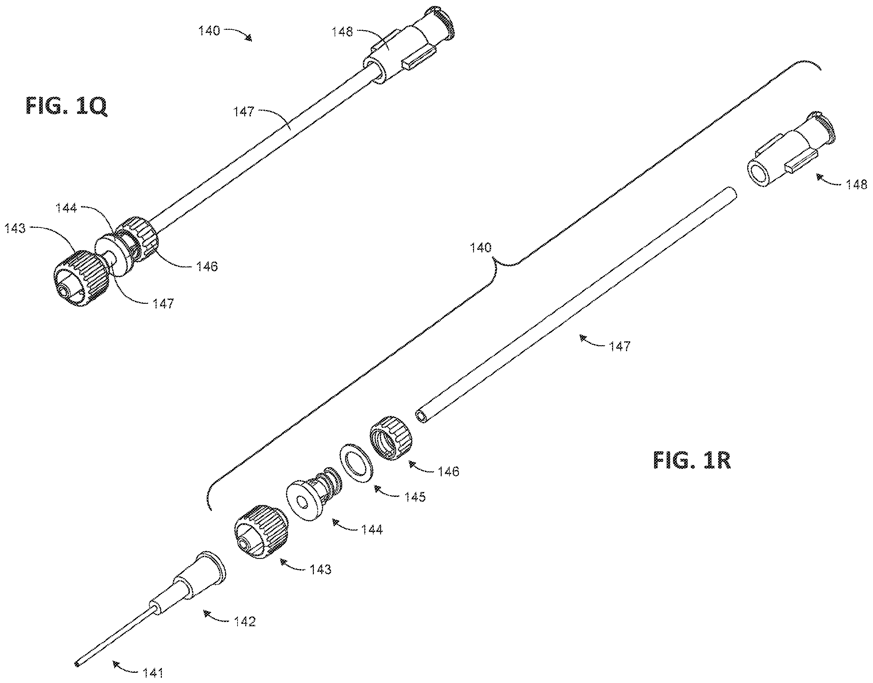

Catheter housing 100 can include a cover 120, which is illustrated in FIGS. 1A-1E with other portions/components of the catheter housing 100 and which is illustrated alone in FIGS. 1F-1N. Additionally, catheter housing 100 can include a hub 160, which is illustrated in FIGS. 1A-1E with other portions/components of the catheter housing 100 and which is illustrated alone in FIGS. 1O-1P. Additionally, catheter housing 100 can include an extension set 140 which is illustrated in FIGS. 1A-1E with other portions/components of the catheter housing 100 and which is illustrated alone in FIGS. 1Q-1R. Some implementations of catheter housing 100 do not include extension set 140. In some implementations, cover 120 and hub 160 form a unitary structure and are coupled to tube 147 of extension set 140 so that cover, hub 160, and extension set 140 comprise a unitary structure. Catheter housing 100 can be used in a manner similar or identical to that discussed in U.S. Pat. Pub. No. 2019/0388652, for example, to stabilize a catheter when the catheter is inserted in a catheter insertion site of a subject. As described in more detail below, stabilization of the catheter can be achieved by engagement with a catheter hub connected to such catheter. Additionally or alternatively, in some implementations, stabilization of the catheter is achieved by engagement with a male luer connector connected to the catheter hub.

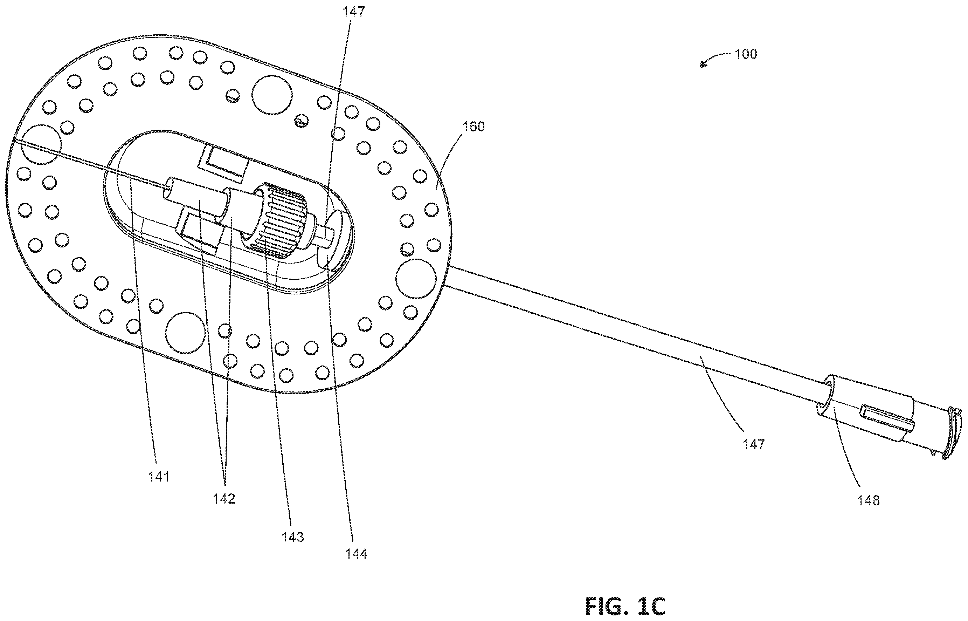

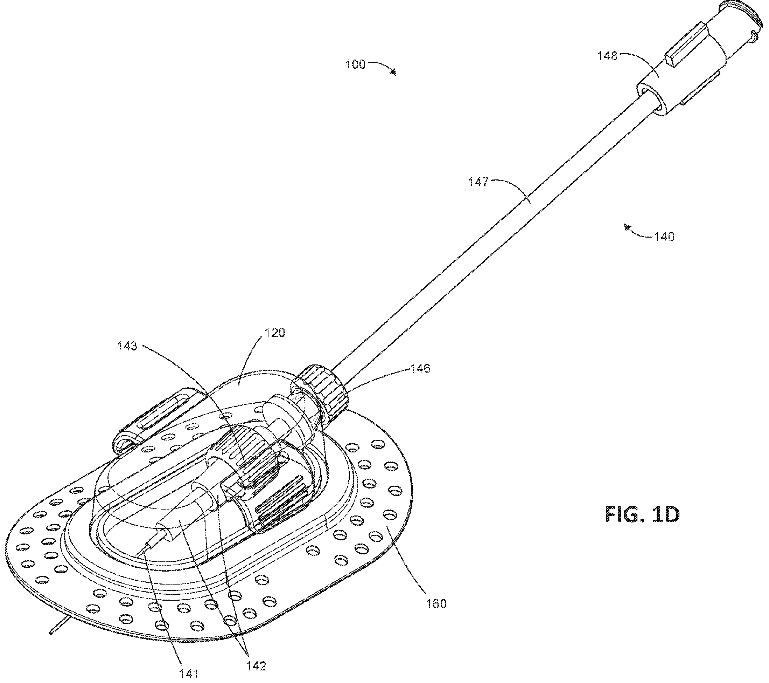

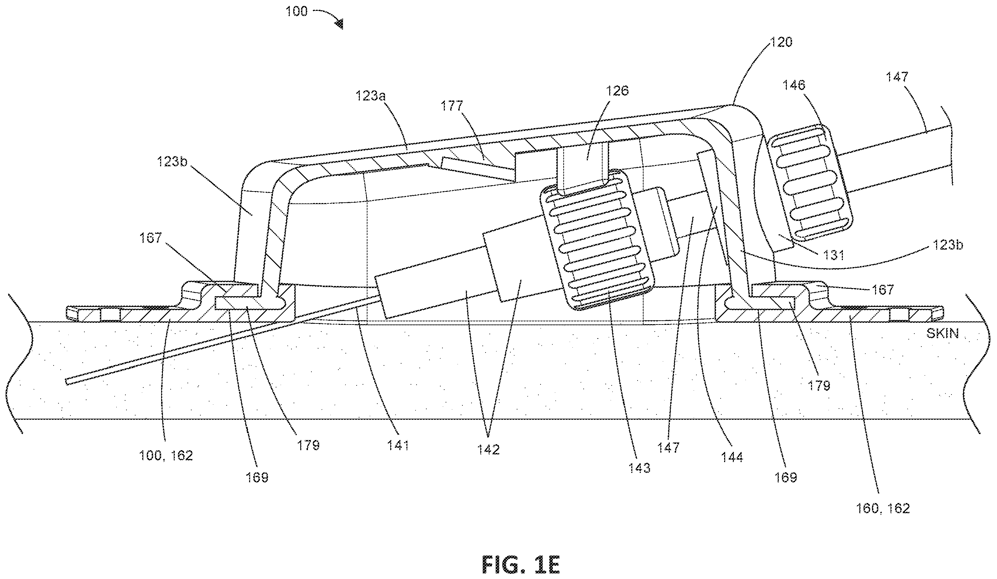

FIGS. 1A-1B illustrate top perspective views of catheter housing 100, while FIG. 1C illustrates a bottom perspective view of the catheter housing 100. FIG. 1D illustrates a top perspective view of the catheter housing 100 with the cover 120 illustrated as transparent to better show an interior of the cover 100, a catheter 141, catheter hub 142, and connector 143 (which also may be referred to herein as "catheter hub connector"). FIG. 1E illustrates a cross-section taken through a portion of the catheter housing 100 and is further discussed below. FIG. 1E shows an illustrative manner in which the catheter housing 100 can be placed and/or secured to the subject's skin when in use. FIG. 1E illustrates a partial cross-section taken through cover 120 and hub 260 to better illustrate components inside an interior region defined by the cover 120, hub 260 and skin of the subject. FIG. 1E further illustrates a connecter 146, and a tube 147 (which can also be referred to as "tubing" or "fluid tube") each of which are described further below.

FIGS. 1F-1N illustrate various views of the cover 120. Cover 120 can be similar or identical to any of the covers discussed in U.S. Pat. Pub. No. 2019/0388652 in some or many respects. Cover 120 can include a top portion 123a and a wall 123b that extends outward from the top portion 123a, and such wall 123b can define an interior of the cover 120. Such top portion 123a and wall 123b can form a main body of the cover 120. In some implementations, such as that illustrated in FIGS. 2H-2Q, cover 120 includes only a single wall extending from top portion 123a (wall 123b), and such single wall 123b defines an interior of cover 120. Wall 123b can extend transverse (for example, generally perpendicular to) such top portion 123a. Wall 123b can define a perimeter of the cover 120. Cover 120 can be made of a variety of materials, including plastic, such as polyphenyl ether (PPE), polycarbonate (PC), among others. In some implementations, cover is transparent or semi-transparent. In some implementations, cover 120 is made of a more rigid material than hub 160 or portions of hub 160. For example, in some implementations, cover 120 is made of a more rigid material than membrane 162 of hub 160.

In some implementations, cover 120 includes one or more wings 122 which can extend outward from wall 123b and/or top portion 123a. Wing(s) 122 can extend outward from the wall 123b and/or top portion 123a and can be utilized to secure tube 147 when in use. For example, tube 147 can be secured to wing(s) 122 and wrapped around a portion of the cover 120 when in use to reduce the likelihood that tube 147 interferes with IV procedures and/or gets inadvertently pulled. Wings 122 can include openings 122a (which may also be referred to as "slots") that can allow a portion of the tube 147 to be viewed, for example, to determine whether fluid is in and/or flowing through tube 147 to the subject. Such openings 122a can additionally facilitate removal of the tube 147 from the wing(s) 122, for example, by allowing a finger or tool to be inserted through opening 122a to push the tube 147 out of securement with wing(s) 122. Wings 122 can include one or more ribs 122b (for example, one, two, three, four, five, or six or more ribs 122b), which can be spaced apart from one another and can aid a user in gripping and/or handling the cover 120 and/or catheter housing 100.

Wall 123b can define a perimeter of the cover 120. In some implementations, cover 120 includes a flange 179 (which can also referred to herein as a "bottom flange") extending outward from wall 123b (for example, a bottom edge of wall 123b that is opposite top portion 123a). With reference to at least FIGS. 1F-1G, 1E, and 1O, flange 179 can be positioned within a slot 169 of the hub 160 during manufacturing of the catheter housing 100 in order to permanently seal and/or secure the cover 120 to the hub 160, as discussed further below. In some variants, the flange 179 has a non-uniform shape and/or perimeter (such as a flower shape as shown), which can provide space for a sealing agent/material to be positioned between the flange 179 and portions of the slot 169 of the hub component 160 to facilitate bonding.

As shown in FIGS. 1G and FIG. 1L, cover 120 includes an opening 133 (which may also be referred to herein as "cover opening"). Opening 133 can be positioned at or near an end of the cover 120. Opening 133 can extend through wall 123b. Cover 120 can include rim 131 (which may also be referred to herein as a "connector" or "cover connector") extending outward from an interior and/or exterior surface of the cover 120 (for example, of wall 123b) around the opening 133. The rim 131 can extend outward from the exterior surface of the cover 120 around opening 133 a distance that is greater than, equal to, or less than a distance that it extends inward from the interior surface of the cover 120 around opening 133 (see, for example, FIG. 1N). Rim 131 can be cylindrical, among other shapes. Rim 131 can include one or more notches 135, such as one, two, three, four, five, or six or more notches 135. Rim 131 can include a plurality of notches 135 that are spaced from one another, for example, radially spaced from one another, along and/or around a perimeter and/or circumference of the rim 131, and/or radially spaced from one another with respect to an axis extending through a center of opening 133. In some implementations, four notches 135 are disposed 90 degrees from one another along rim 131 (see FIG. 1L). Rim 131 can be sized and/or shaped to receive and/or secure to connecter 144 and/or connector 146 of extension set 140 (see FIGS. 1E and 1R-1T), as further described below. Securement between the rim 131 and the connector 144 and/or 146 can advantageously inhibit movement of cover 120 and/or hub 160 along tube 147, for example, so as to keep cover 120 and/or hub 160 in place when secured to the subject's skin.

With reference to FIGS. 1I and 1N, cover 120 can include an arm 126 (which can also be referred to as a "stabilizing arm" or "stabilizing portion" or "mechanical stabilizer") that can help stabilize catheter 141 when inserted at a catheter insertion site by engaging connector 143 which can connect to catheter hub 142 that is connected to catheter 141. Although arm 126 is illustrated and described as engaging connector 143, in some variants, arm 126 can be configured to engage catheter hub 142. Arm 126 can advantageously facilitate stabilization of catheter 141 by retaining, securing, and/or aligning connector 143. This can advantageously minimize movement of catheter 141 when inserted at the catheter insertion site. As described elsewhere herein, arm 126 can cooperate with connector 144, opening 133, connector 146, and/or tube 147 to stabilize catheter 141 (for example, by positioning and stabilizing catheter 141, catheter hub 142 at an appropriate inclined position relative to the catheter insertion site.

Arm 126 can extend from an interior surface of cover 120. For example, as shown, arm 126 can extend from an interior surface of the cover 120 opposite an exterior surface of the cover 120 along the top portion 123a of the cover 120 and/or can extend in a direction at least partially toward the subject's skin. The size, shape, and/or configuration of arm 126 of cover 120 can be similar or identical in some or many respects to that discussed with reference to the lock and/or bridge of any of the covers described in U.S. Pat. Pub. No. 2019/0388652.

Arm 126 can extend transverse (for example, perpendicular) from the cover 120 (for example, from an interior surface of the top portion 123a of cover 120). Arm 126 can have a first end connected to top portion 123a (such as an interior surface of the top portion 123a) and a second end opposite such first end that includes a recess. Such recess can be sized and/or shaped to surround a portion of connector 143 (and/or catheter hub 142 where arm 126 is configured to engage catheter hub 142). Such recess can be rounded and/or circular or arch shaped, for example, among other shapes. Such recess can be configured to surround less than an entire perimeter of a cross-section of connector 143 when the catheter housing 100 is in use, for example, less than approximately 80%, less than approximately 70%, less than approximately 60%, less than approximately 50%, less than approximately 40%, or less than approximately 30% of an entire perimeter of a cross-section of connector 143. Such configurations can allow the cover 120 to engage and disengage the connector 143 more easily, thereby facilitating simpler assembly of the catheter housing 100 on a subject.

In some implementations, catheter housing 100 includes one or more UV light sources that illuminate and/or disinfect the catheter insertion site where the catheter 141 is inserted in the subject and/or areas within an interior of the cover 120 and/or surrounding various components housed within the cover 120 when the catheter housing 100 is in use. With reference to FIGS. 1I and 1N, the cover can include one or more light source housings 177, such as one, two, three, or four or more light source housings 177. The light source housings 177 can extend from a portion of cover 120, such as an interior surface of a top portion 123a of cover 120. The light source housings 177 can include a recess and/or cavity to receive and/or secure UV light sources. The light source housings 177 and/or the recesses/cavities defined thereby can be sized, shaped, and/or otherwise configured to direct the emitted light from the UV light sources toward the catheter insertion site, skin of the patient, catheter 141, catheter hub 142, connector 143, and/or a portion of tube 147 positioned in an interior region at least partially defined by cover 120 (see FIG. 1E). In some implementations, light source housings 177 and/or the recesses/cavities defined thereby can be sized, shaped, and/or otherwise configured to direct the emitted light from the UV light sources toward the catheter insertion site, skin of the patient, catheter 141, and/or catheter hub 142 at an angle. For example, the light source housing 177 and/or the recesses/cavities defined thereby can be angled as shown in FIGS. 1N and 1E such that emitted light is directed primarily to the catheter insertion site. The UV light sources can be UV Surface Mount LED (SMD LED) and/or UV-C light sources. The UV light sources can comprise UV LEDs in some implementations. In some implementations, cover 120 includes two light source housings 177 spaced apart from one another and extending from an interior surface of top portion 123a of cover 120 and positioned proximate opposite sides/portions of walls 123b of cover 120 (see FIG. 1I).

The UV light sources can be controlled by a controller (such as a 4-bit processor) which can implement different user selectable schemes by controlling the intensity as well as the duration of the exposure of emitted light. The controller can allow for various sophisticated disinfection schemes based upon the patient type and/or their condition. For example, the UV light sources may be turned on intermittently, on a regular basis with a predefined on-time or as needed based upon a measure of the site infection. One measure is the required dose (of UV light) to inactivate certain types of bacteria or a virus which may be present on the skin. This can be controlled by the intensity of the UV source as well as its duration. In some implementations, the UV source is configured to automatically turn off when cover 120 and/or hub 160 is moved (for example, dislodged from the patient's skin). The controller can comprise a processor and/or memory, and can be embodied in one or more printed circuit boards. Such controller can be mounted to a portion of catheter housing 100 in some implementations. However, in some variants, such controller is contained in a separate device and can communicated (for example, wirelessly) with a communication module mounted to catheter housing 100 coupled with the UV light sources.

The UV light sources can be operated continuously or periodically/intermittently. For example, the UV light sources can be configured to turn on and/or off according to timing protocols, such as once every half hour, hour, 2 hours, 3 hours, 4 hours, among other values. The UV light sources can be configured to irradiate skin at and/or around the catheter insertion site at various wavelengths. The UV light sources can be configured to emit light at a wavelength of between approximately 100 and approximately 500 nm, between approximately 200 and approximately 400 nm, between approximately 200 and approximately 300 nm, between approximately 210 and approximately 290 nm, between approximately 220 and approximately 280 nm, between approximately 230 and approximately 270 nm, between approximately 240 and approximately 260 nm, between approximately 200 and approximately 250 nm, between approximately 220 and approximately 230 nm, or between approximately 220 and approximately 350 nm or any value or range therebetween or bounded by any combination of these values or ranges, although values outside these values or ranges can be used in some cases. In some configurations, the UV light sources can be configured to emit light at a wavelength at approximately 220 nm. The UV light sources can be configured to irradiate skin at and/or around the catheter insertion site at short wavelength ultraviolet radiation. In some configurations, the radiation exposure time of the UV light sources can be selected so as to provide beneficial disinfection of the skin at and/or around the catheter insertion site while also minimizing the amount of time by which the skin is being subjected to the radiation. For example, the UV light sources can be configured to emit light for short time periods, such as 0.1 seconds, 0.2 seconds, 0.3 seconds, 0.4 seconds, 0.5 seconds, 1 second, 1.5 seconds, 2 second, 3 second, 4 seconds, or 5 seconds, or any value or range therebetween or bounded by any combination of these values or ranges, although values outside these values or ranges can be used in some cases.

With reference to at least FIGS. 1E, 1I, 1N, and 1O, the UV light sources (which can be mounted within light source housings 177) can be configured to emit light through the opening 163 of the hub 160/membrane 162 and around the catheter insertion site. With reference to FIG. 1E, the UV light sources can emit light towards the catheter insertion site, and, in some implementations, the housings 177 are angled such that the UV light sources emitted light having a triangular shape on the subject's skin. The angle defined by a vertex/corner of such triangular shape of the emitted light can define an angle that is less than 90 degrees in order to appropriately focus the emitted light at and around the catheter insertion site. Such angle can be, for example, less than 85 degrees, less than 80 degrees, less than 75 degrees, less than 70 degrees, less than 65 degrees, less than 60 degrees, less than 55 degrees, or less then 50 degrees, or any value or range therebetween or bounded by any combination of these values or ranges. With further reference to FIG. 1E, in some cases the catheter 141 and/or the catheter hub 142 may block some of such emitted light and cast a shadow therebelow on the patient's skin. In some configurations, the UV light sources are wide source UV light sources that can better illuminate such "hidden" portions of the skin, and/or one or more UV light sources are positioned and/or spaced within cover 120 to illuminate such hidden portions of the skin. As another example, one or more mirrors can be incorporated into catheter housing 100 (for example, on interior surfaces of cover 120) to help redirect the emitted light to illuminate such hidden portions of the skin.

FIGS. 1O-1P illustrate hub 160 of catheter housing 100. Hub 160 can be similar or identical to any of the hubs discussed in U.S. Pat. Pub. No. 2019/0388652 in some or many respects. Hub 160 can include and/or be defined by membrane 162. Membrane 162 can include one or more perforations 162a (also referred to herein as "apertures") to facilitate breathability of the subject's skin when membrane 162 is secured thereto. Membrane 162 can include a plurality of perforations 162a spaced along and around opening 163 of membrane 162, such as one or more, two or more, three or more, four or more, five or more, six or more, seven or more, eight or more, nine or more, ten or more, or eleven or more, between ten and fifty, or between fifty and one hundred perforations 162a, although a different amount of perforations 162a can be present in some implementations.

Membrane 162 can be made of a flexible material. Membrane 162 can be made of a different material than cover 120. Membrane 162 can comprise, for example, thermoplastic elastomers (TPE), thermoplastic polyurethane (TPU), and/or polyvinyl chloride (PVC), among other materials. In some implementations, membrane 162 comprises a material that is more flexible than a material comprised by cover 120. In some implementations, membrane 162 and cover 120 comprise the same material.

Membrane 162 can include an opening 163 which can be positioned over a site where a needle and/or catheter 141 is to be (or has been) inserted into a patient. In some implementations, such as that shown, such opening 163 is not partitioned and/or divided by any portion of membrane 162. Opening 163 can have a rounded shape. For example, opening 163 can comprise an oblong shape which is not partitioned and/or divided by any portion of membrane 162. In some implementations, when catheter housing 100 is assembled and secured to the subject's skin, no portion of membrane 162 is positioned between the catheter hub 142 and/or connector 143 and the subject's skin. In some implementations, hub 160 and/or membrane 162 (and/or cover 120) is made of a transparent or semi-transparent material, which can allow for inspection of the catheter insertion site, catheter 141, catheter hub 142, connector 143 when catheter housing 100 is in use.

As mentioned above, cover 120 and hub 160 can be permanently secured together to form a unitary structure. With reference to FIG. 1O, hub 160 can include a raised portion 167 extending above and/or outward from a portion of membrane 162 and around a perimeter of opening 163. Such raised portion 167 can include a slot 169 (which can also be referred to as a "channel") which can be sized and/or shaped to receive the flange 179 of cover 120. Such slot 169 can extend around opening 163 as shown in F1G. 1O. During manufacturing, flange 179 can be inserted into the slot 169 and permanently secured (for example, bonded) to the hub 160. Such permanent securement can be via adhesive and/or ultrasonic welding, among other things. Such permanent securement can advantageously reduce the number of components of catheter housing 100 and therefore reduce difficulty of assembly and/or use of catheter housing 100 by a caregiver. In some variations, the cover 120 and hub 160 can be integrally formed during manufacturing, for example, via an overmolding process. In some of such cases, cover 120 does not include flange 179 and hub 100 does not include slot 169 (and/or raised portion 167).

In some implementations, catheter housing 100 includes one or more sensors that can be used to measure various physiological parameters and/or condition of a patient. Such one or more sensors can include a temperature sensor (for example, a topical temperature sensor), a blood pressure sensor, a blood oxygen saturation sensor, a sensor for liquid and blood leakage, and/or a skin humidity sensor. Such sensors can be located in various locations on the membrane 162. For example, with reference to FIG. 1P, the membrane 162 can include cavities 183 recessed from a surface of the membrane 162 (for example, a bottom surface) that are sized and/or shaped to receive the one or more sensors. The membrane 162 can include, one, two, three, four, five, six, seven, or eight or more cavities 183. The membrane 162 can include a plurality of cavities 183 spaced apart from one another and positioned around the opening 163. Such cavities 183 can comprise a rounded shape, for example, a circular shape, among others. In some cases, the catheter housing 100 includes electrodes positioned within the cavities 183. In some variations, such electrodes can be used for electrotherapy purposes to apply electrical energy to the wearer of the catheter housing 100 at and/or around the catheter insertion site, which can provide benefits such as pain reduction, among others.

FIGS. 1Q-1R illustrate various components which may together form an extension set 140 of the catheter housing 100. FIG. 1Q illustrates the extension set 140 detached/decoupled from the cover 120 and hub 160 to better illustrate aspects of the extension set 140. FIG. 1R illustrates an exploded view of extension set 140 and also the catheter hub 142 and connected catheter 141. The extension set 140 and/or portions thereof can be coupled to the catheter 141 and catheter hub 142 and can transfer fluid to the catheter 141 from a location external to the catheter housing 100, for example, from a fluid source (such as an IV bag). In some variants, the extension set 140 can be utilized to transfer fluid (for example, blood) from the subject to a fluid collection container via catheter 141, however. FIG. 1R illustrates a connector 143 (which can be a male luer connector), a connector 144, an annular ring 145, a connector 146, a tube 147, and a connector 148 (which can be a female luer connector). Connectors 143 and 148 can connect to opposite ends of tube 147. Connector 148 can be configured to connect to a fluid source. Tube 147, connector 143, and/or connector 148 can be similar or identical to any of the tubes and/or connectors described in U.S. Pat. Pub. No. 2019/0388652.

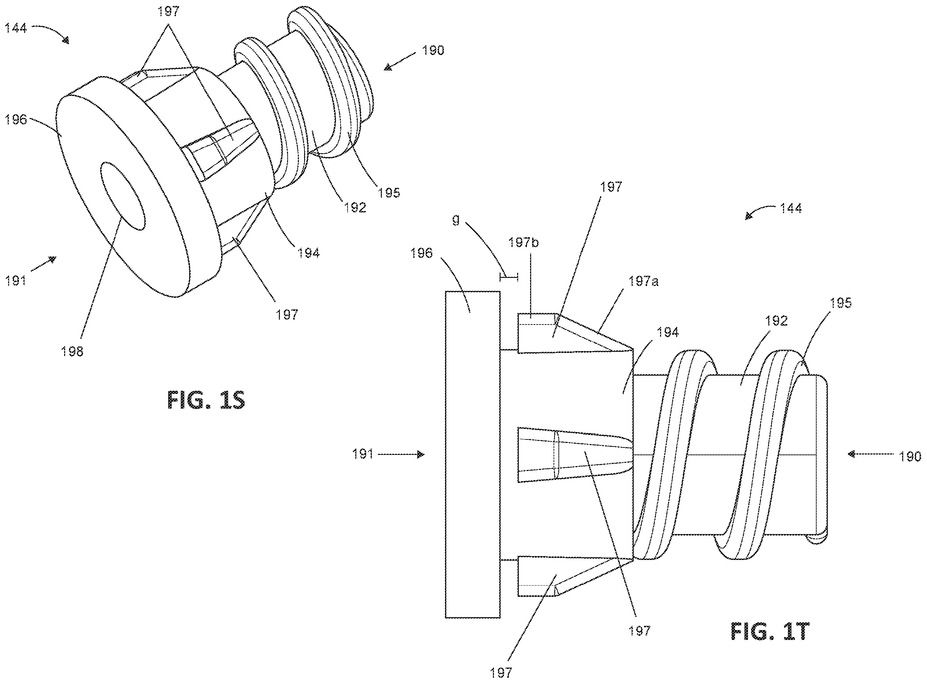

Connector 144 of extension set 140 can include an opening 198 (see FIG. 1S) sized to accommodate tube 147. Connector 144 can advantageously secure the extension set 140 to the cover 120 alone and/or in combination with the connector 146 as further described below. Connector 146 can secure to connector 144 and to cover 120, for example, at rim 131 and opening 133 of cover 120 as discussed further below. In some implementations, extension set 140 includes an annular ring 145 that can be positioned (for example, "sandwiched") in between the a portion of the connector 144 (for example, flange 196) and rim 131 within an interior of cover 120. The extension set 140 can be coupled with the catheter hub 142 and catheter 141, for example, via securement of connector 143 to an end of catheter hub 142, as further discussed below.

FIGS. 1S-1T illustrate perspective and side views (respectively) of connector 144. Connector 144 can advantageously allow the extension set 140 to couple with cover 120 to form a unitary structure with the cover 120 and/or hub 160. Further, in some implementations, securement of connector 144 to connector 146 on opposite sides of rim 131 of cover 120 can inhibit cover 120 from being able to move along tube 147, which can be advantageous when cover 120 and hub 160 are secured to the subject's skin. Connector 144 can include a first end 190 and a second end 191 opposite the first end 190. Connector 144 can include a flange 196 (for example, at end 191), a body 194, and a stem 192. The body 194 can be sized and/or shaped to be received within the rim 131 and/or through opening 133 of cover 120. The body 194 can comprise a cylindrical or partially cylindrical shape and/or a circular cross-section. The body 194 can include one or more protrusions 197 extending away from a surface of the body 194 and/or along a length of the body 194. For example, the body 194 can include one, two, three, or four or more protrusions 197. In some implementations, the number of protrusions 197 corresponds with the number of notches 135 on rim 131 of cover 120. The body 194 can include a plurality of protrusions 197 that are spaced from one another (for example, radially spaced from one another along a circumference of the body 194). In some implementations, connector 144 includes four protrusions 197 spaced 90 degrees from one another relative to an axis extending through a center of connector 144. The protrusions 197 can be sized and/or shaped to fit within and/or through the notches 135 of cover 120, which are described above. With reference to FIG. 1T, protrusions 197 can include an inclined portion 197a having a linearly increasing/decreasing height (measured from the surface of the body 194) and a non-inclined portion 197b having a uniform height (measured from the surface of the body 194). In some implementations, the protrusions 197 extend along a surface of the body 194 from a first end of the body 194 towards a second end of the body 194 and stop prior to reaching said second end of the body 194 such that a gap "g" exists between an end of the protrusions 197 and the flange 196 (see FIG. 1T). Such gap can be sized and/or shaped to fit annular ring 145. Connector 144 can include a stem 192 extending outwards from body 194. Stem 192 can be sized and/or shaped to fit within an opening extending through connector 146. Stem 192 can comprise a cylindrical or partially cylindrical shape and/or a circular cross-section. Stem 192 can include threads/threading 195 extending along a surface of stem 192 that can be configured to secure to threads/threading within connector 146. Flange 196 of connector 144 can have a cross-section that is greater than cross-sections of one or both of body 194 and stem 192. Such configuration can ensure that flange 196 "seats" against a portion (end) of rim 131 of cover 120 within an interior of cover 120. As shown in FIG. 1S, connector 144 can include an opening 198 extending through a length of the connector 144. Such opening 198 can be sized and/or shaped to receive tube 147 such that tube 147 can extend through connector 144. Opening 198 can have a circular cross-section and can be sized to receive tube 147.

As discussed above, FIG. 1E illustrates a cross-section through catheter housing 100 and further illustrates how the extension set 140, cover 120, and hub 160 can form a unitary structure that can couple with a catheter hub 142 and catheter 141 when the catheter 141 is inserted into a catheter insertion site (for example, inserted through skin and into a subject's vein) Cover 120 and hub 160 can be secured (for example, permanently bonded) together as discussed above via securement of flange 179 within slot 179.

Extension set 140 can be secured to the cover 120 and hub 160 (for example, via direct securement to the cover 120) in a variety of ways to form a unitary structure, for example, during manufacturing of catheter housing 100. Various ones of the connector 143, connector 144, annular ring 145, connector 146, and/or connector 148 can be positioned along (for example, over) and/or secured to and/or over portions of tube 147 before or after tube 147 is inserted through the opening 133 of cover 120. Connector 144 (for example, via opening 198), annular ring 145, and/or connector 146 can be positioned around and/or along the tube 147. Connector 144 (for example, via opening 198), annular ring 145, and/or connector 146 can be configured to slide along the tube 147, for example, slide longitudinally along an axis extending through tube 147. Such configuration can advantageously allow the connector 144, annular ring 145, and/or connector 146 to be positioned at or near the rim 131 and/or opening 133 of the cover 120. The stem 192 and body 194 of the connector 144 can be positioned within and/or through (or partially within and/or through) the opening 133 and/or rim 131 of the cover 120, for example, via inserting the one or more protrusions 197 through the one or more notches 135 of the rim 131 such that the flange 196 contacts a portion (for example, end) of the rim 131 in an interior of the cover 120 (see FIG. 1E). Contact between flange 196 and such portion of the rim 131 can prevent further movement of the connector 144 through the rim 131. In some cases where the extension set 140 includes an annular ring 145, such annular ring 145 can be positioned (for example, sandwiched) between the flange 196 and such portion of the rim 131. In some implementations, the protrusions 197 can be inserted through the notches 135 and the connector 144 can be rotated with respect to the rim 131 (for example, an axis extending through a center of rim 131 and/or opening 133) such that the protrusions 197 do not align with the notches 135 after such rotation. In such cases, rim 131 can contact and/or prevent connector 144 from being moved out of opening 133. After connector 144 is positioned within and/or through (or partially through) the opening 133 and/or rim 131 of the cover 120, the connector 146 can be secured (for example, threadingly secured) to the connector 144, for example, via engagement between threads of the connector 146 and threads 195 of stem 192. When such securement takes place, flange 196 of connector 144 and connector 146 can sandwich rim 131 of cover 120. Such securement can advantageously couple the extension set 140 to the cover 120 and hub 160.

In some cases, cover 120 and/or hub 160, when coupled along the tube 147, can slide along the tube 147, for example, slide longitudinally along an axis extending through tube 147. Connector 143 can be secured to an end of tube 147 and connector 148 can be secured to an opposite end of tube 147. In some implementations, when connector 144 and connector 146 are disconnected from one another, the cover 120 and hub 160 can be moved along the tube 147 toward and/or away from the connector 143. Such configurations can advantageously allow a caregiver to secure the catheter housing 100 to a catheter hub 142 and catheter 141 in a quick and efficient manner. For example, after the caregiver has inserted the catheter 141 into the patient (for example, in a vein of the patient), the caregiver can move the cover 120 and hub 160 (which are positioned along the tube 147) away from the connector 143 along a portion of the length of the tube 147 and then secure (for example, threadingly secure) the connector 143 of the extension set 140 to an end of the catheter hub 142. Thereafter, the caregiver can move (for example, slide) the cover 120 and hub 160 toward the male connector 143, catheter hub 142, and catheter 141 to position and secure the cover 120 and hub 160 to skin around the catheter insertion site. Such positioning can involve engaging connector 143 with arm 126 such that connector 143 is positioned within a recess of arm 126 as described above. After the cover 120 and hub 160 are positioned and/or secured to the skin, the caregiver can secure connector 146 to connector 144 (for example, as discussed above) to minimize or prevent movement of the cover 120 and hub 160 relative to tube 147. With reference to FIG. 1E, when connector 146 is secured to connector 144 in such manner, connectors 144, 146 can sandwich a portion of cover 120 around the opening 133, such as the rim 131. Advantageously, securement of extension set 140 with cover 120 at opening 133 and/or rim 131, alone and/or in combination with the arm 126, provides robust stabilization of catheter hub 142 and connector 143 among other components, that prevents or reduces potential movement of the catheter 141 when inserted into the patient. Additionally, such securement of extension set 140 with cover 120 at opening 133 and/or rim 131 prevent or reduces issues that may arise when tube 147 is pulled in various directions relative to cover 120.

With reference to FIGS. 1C-1E, in some configurations, the cover 120 and/or hub 160 can be secured to extension set 140 in such a manner that the connector 143 is spaced from the connector 144 and/or rim 131 (within an interior of the cover 120) by a gap, for example, along the tube 147. Such gap can be seen by the portion of tube 147 that is visible within the interior region enclosed by cover 120 in FIG. 1E. Such gap can advantageously allow a portion of tube 147 (inside the interior of the cover 120) to act as a flexible joint (for example, when tube 147 comprises a flexible material), which can minimize or prevent forces applied to cover 120, hub 160, connector 146, connector 144, and/or portions of tube 147 external to cover 120, from transferring to connector 143 and thus the catheter hub 142 and/or catheter 141. For example, if a force is applied to cover 120, hub 160, or portion of the tube 147 external cover 120 in a lateral direction (for example, "into" or "out of" the page given the view shown in FIG. 1E), such gap between connector 143 and connector 144 (and/or the opening 133, rim 131, or other portion of the cover 120) can provide a flexible joint which minimizes or prevents such force from translating to the connector 143, catheter hub 142, and/or catheter 141. Such result can significantly reduce likelihood of catheter "kinking" and/or other issues that can arise when catheters 141 are moved relative to a patient's vein and/or skin at or near a catheter insertion site.

FIGS. 1U-1X illustrate an alternative design for a cover 120'. Cover 120' can be used with catheter housing 100 in a similar manner as cover 120. Cover 120' can be identical to cover 120 in every respect except that cover 120' additionally includes ports 121a', 121b'. Ports 121a', 121b' can be similar or identical to ports 521a, 521b discussed and illustrated in U.S. Pat. Pub. No. 2019/0388652 in some or many respects. Ports 121a', 121b' can be used to allow gas to flow into and/or out of an interior of the cover 120'. Port 121a' can be a gas inlet port configured to secure to a tube to allow gases from the tube to be inserted through a portion of the cover 520' and towards the catheter insertion site. For example, sterilizing and/or anesthetic gas can be inserted through port 121a'. Port 121b' can be a gas outlet port configured to allow gases to flow out from the catheter housing 100 and through a portion of the cover 120'. Port 121b' can include a relief valve that allows gas to escape from an interior of the cover 120' and/or catheter housing 100, for example, where a certain pressure is attained. The relief valve of can be configured to open when gas is inserted through gas inlet port 121a'. In some implementations, ports 121a', 121b' do not include any valves and/or are entirely open.

Ports 121a', 121b' can extend through various portions of cover 120'. For example, one or both ports 121a', 121b' can extend through a top portion 123a' of cover 120', from which wall 123b' extends. Ports 121a', 121b' can define an opening on the interior surface of cover 120'. Cover 120' can comprise a chamber 121c' that surrounds a perimeter of the opening defined by the port 121a'. Chamber 121c' can be cylindrical, for example. One or both of ports 121a', 121b' can be positioned proximate wall 123b' of cover 120'. Ports 121a', 121b' can be positioned proximate opposite sides/surfaces of wall 123b' of Port 121a' and/or port 121b' can be configured to secure a tube via a snap fit, friction fit, press fit, or another type of securement.

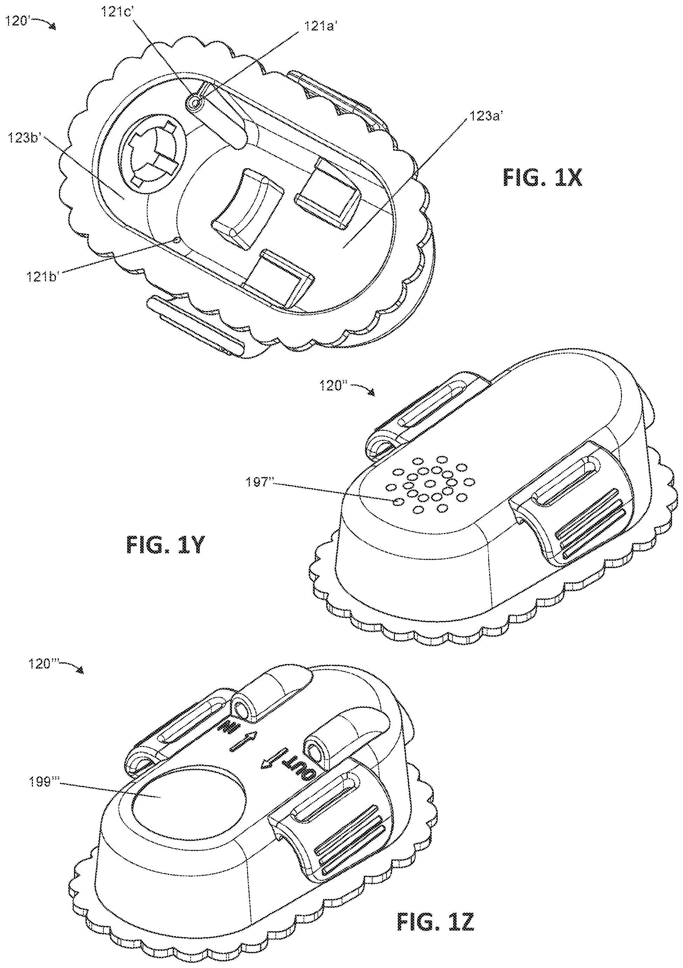

Gas ports 121a', 121b' can protrude outward and/or along top portion 123a' of cover 120'. Ports 121a', 121b' can define a chamber along such length of extension, and the chamber can be sized and/or shaped to receive and/or secure to a tube. Ports 121a', 121b' can comprise a rounded shape. Ports 121a', 121b' can comprise cylindrical cross section or partially cylindrical cross section along their length. Ports 121a', 121b' can have a first end 121d' defining an opening and a second end 121e' opposite the first end 121 d'. The second end 121e' can slope or taper in cross section so as to transition the fluid passage defined by the ports 121a', 121b' to the opening in the portion of the cover 120'. For example, a chamber defined by ports 121a', 121b' can transition from a direction parallel to an axis running through openings at ends 121d' (see FIG. 1V) to a direction perpendicular to such axis near the openings through the portion of the cover 120' (see FIG. 1X).

FIG. 1Y illustrates an alternative design for a cover 120". Cover 120" can be used with catheter housing 100 in a similar manner as cover 120. Cover 120" is identical to cover 120 in every respect except that it includes a plurality of holes 197" extending through a top portion of the cover 120. Such holes 197" can allow interior portions of the catheter housing 100 and/or the catheter insertion site to be ventilated (for example, in fluid communication with ambient air). In some variations, the holes 197‴ can be covered, on an exterior and/or interior surface of the cover 120 with a semi-permeable film, which can be transparent. Such semi-permeable film can be secured to an interior surface of cover 120" adjacent (for example, underneath) holes 197". Such semi-permeable film can be a plastic sheet of polyurethane bonded to such interior surface of cover 120" via acrylic adhesive or any other type of adhesive. Inclusion of a semi-permeable film proximate holes 197" can advantageously provide ventilation while also reducing the potential of objects and/or fluid passing through the cover 120" via the holes 197". Although the cover 120" is illustrated as non-transparent in FIG. 1Y, the cover 120" can comprise a transparent or semi-transparent material.

FIG. 1Z illustrates an alternative design for a cover 120‴. Cover 120‴ can be used with catheter housing 100 in a similar manner as cover 120. Cover 120‴ can be identical to cover 120' in every respect except that it includes a recessed portion 199‴ that is recessed from a top external surface of the cover 120"'. In some variations, a camera can be positioned and/or secured to the recessed portion 199". Although the cover 120‴ is illustrated as non-transparent in FIG. 1Z, the cover 120" can comprise a transparent or semi-transparent material. Such configuration can advantageously allow a camera mounted in such recessed portion 199" to view the catheter insertion site and/or other components housed within the catheter housing 100 during use, for example, the catheter 141, catheter hub 142 coupled with the catheter 141, the connector 143, contact between the connector 143 and the arm 126 of the cover 120, etc. Such configuration can also advantageously allow a caregiver to determine if the catheter 141 is inserted (and/or inserted correctly) at a catheter insertion site and/or whether the catheter hub 142 is aligned, angled, and/or positioned appropriately. The recessed portion 199" can have a circular cross-section, among others.

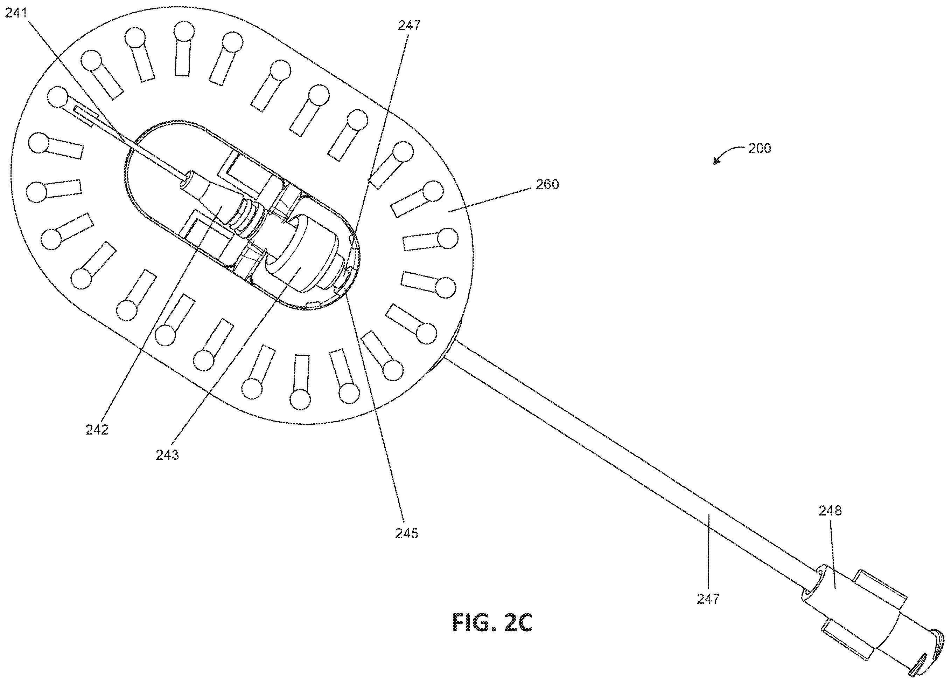

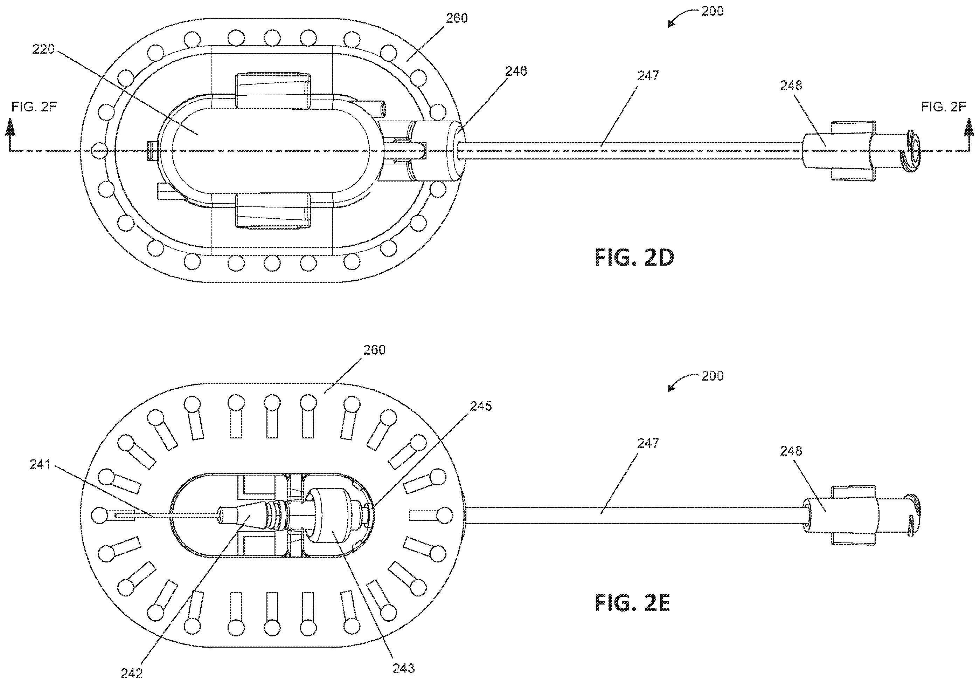

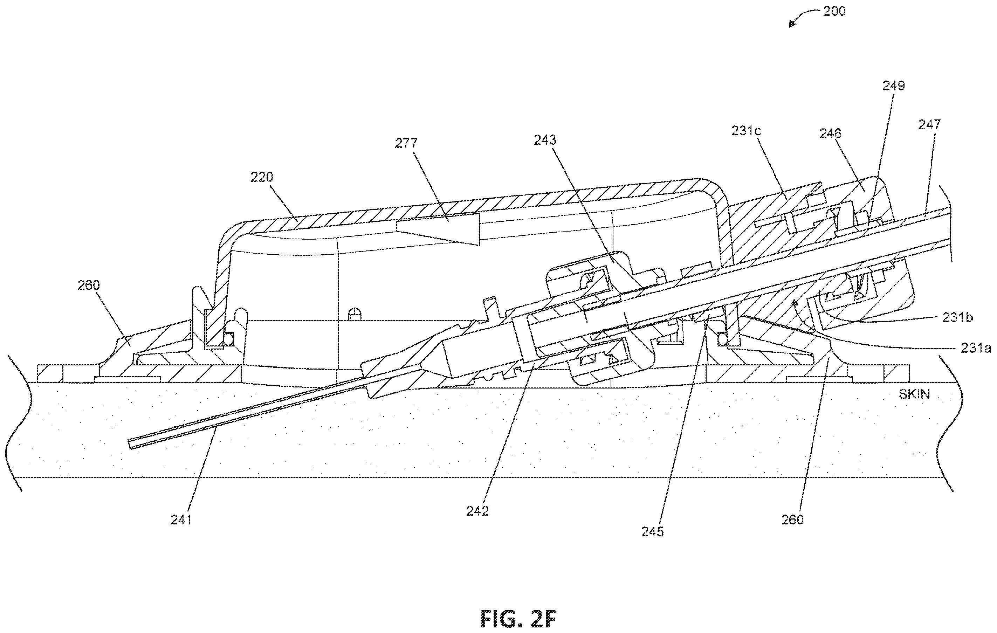

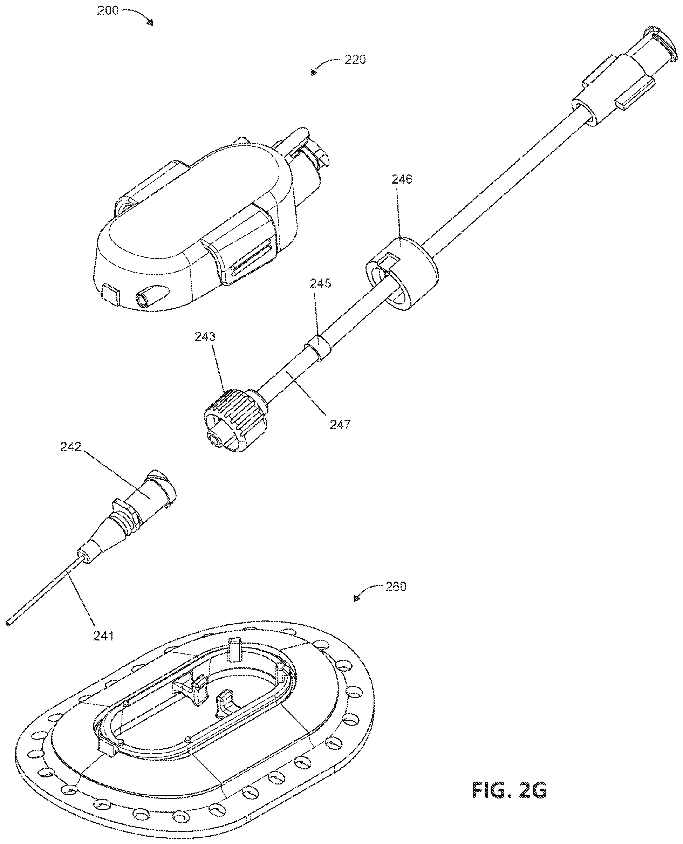

FIGS. 2A-2EE illustrate a catheter housing 200 (which may also be referred to as a "catheter housing device", "catheter stabilization device", or "catheter stabilization system") and various aspects and/or portions thereof as further described below. Catheter housing 200 can be advantageously be used to stabilize a catheter when the catheter is inserted in a catheter insertion site of a subject, in a similar manner as that described elsewhere herein, for example, with reference to catheter housing 100. As described in more detail below, stabilization of a catheter 241 can be achieved by engagement with a catheter hub 242 connected to such catheter 241. Additionally or alternatively, in some variants, stabilization of the catheter 241 is achieved by engagement with a male luer connector 243 connected to the catheter hub 242. As shown, catheter housing 200 can include a cover 220, a hub 260, and an extension set 240 (also referred to herein as a "fluid tube assembly"). FIGS. 2A-2B illustrate top perspective views of the catheter housing 200, FIG. 2C illustrates a bottom perspective view of the catheter housing 200, and FIGS. 2D-2E illustrate top and bottom views of the catheter housing 200. Each of FIGS. 2C and 2E illustrate catheter hub 242 (connected to a catheter 241) secured by the catheter housing 200, as further described below. Catheter 241 and/or catheter hub 242 can be similar or identical to any of the catheters and/or catheter hubs described herein. FIG. 2F illustrates a cross-sectional view taken through the catheter housing 200 (see FIG. 2D) in a condition of use in which catheter housing 200 is secured to skin of a subject. Although not shown, it is to be understood that catheter 241 can be positioned at least partially within a vein of the subject, in accordance with conventional catheter insertion practices, so that catheter 241 can deliver fluid to the subject. Stabilization of catheter 241 (for example, via securement of catheter hub 242) is described in more detail below. FIG. 2G illustrates an exploded view of catheter housing 200, illustrating the cover 220, hub 260, and extension set 240 separated from one another and from catheter 241 and catheter hub 242. Some implementations of catheter housing 200 do not include extension set 240.

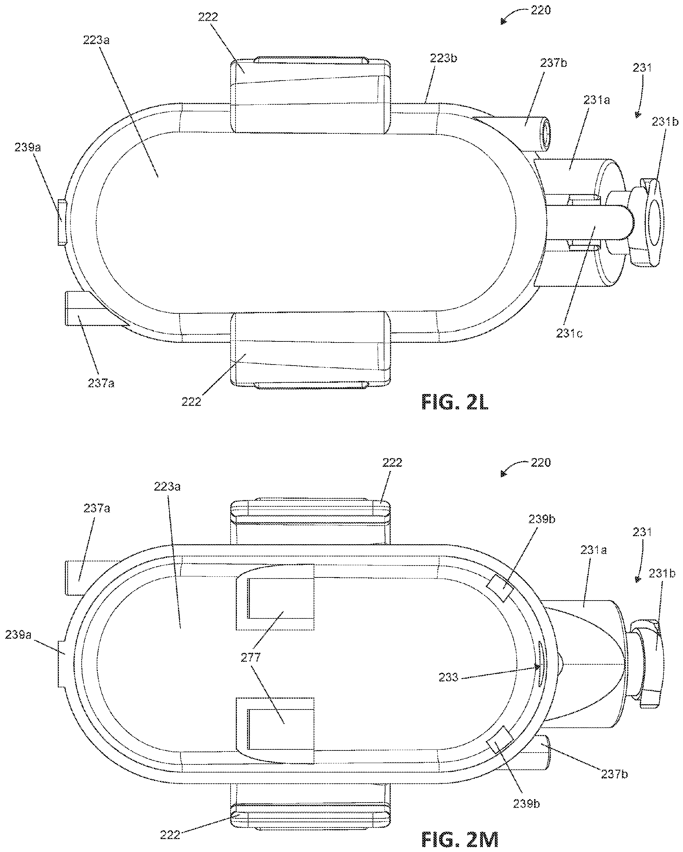

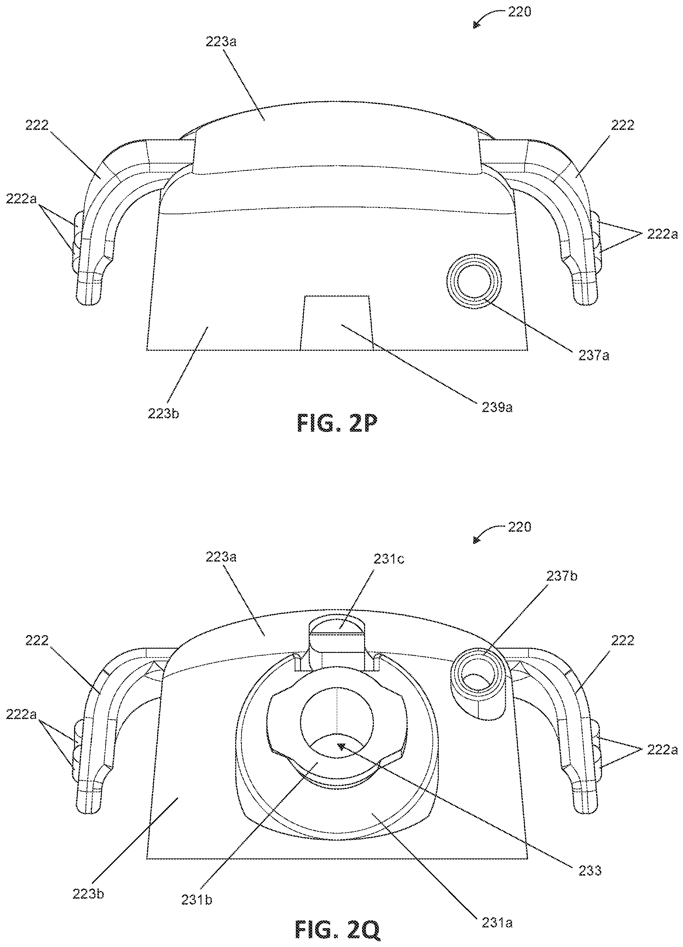

FIGS. 2H-2J illustrate top perspective views of cover 220, FIG. 2K illustrates a bottom perspective view of cover 220, FIGS. 2L-2M illustrate top and bottom views of cover 220, FIGS. 2N-2O illustrate side views of cover 220, and FIGS. 2P-2Q illustrate front and back views of cover 220. Cover 220 can include a top portion 223a and a wall 223b that extends outward from the top portion 223a, and such wall 223b can define an interior of the cover 220. Such top portion 223a and wall 223b can form a main body of the cover 220. In some implementations, such as that illustrated in FIGS. 2H-2Q, cover 220 includes only a single wall 223b extending from top portion 223a, and such single wall 223b defines an interior of cover 220. Wall 223b can extend transverse (for example, generally perpendicular to) such top portion 223a. Wall 223b can define a perimeter of the cover 220. Cover 220 can be made of a variety of materials, including plastic, such as polyphenyl ether (PPE), polycarbonate (PC), among others. In some implementations, cover 220 is transparent or semi-transparent. In some implementations, cover 220 is made of a more rigid material than hub 260 or portions of hub 260. For example, in some implementations, cover 220 is made of a more rigid material than membrane 262 of hub 260.

In some implementations, cover 220 includes one or more wings 222 extending outward from the top portion 223a and/or wall 223b which can be utilized to secure tube 247 when in use. Wings 222 can be similar or identical to wings 122 in some or many respects. In some implementations, wings 222 include one or more ribs 222a (for example, one, two, three, four, five, or six or more ribs 222a), which can be similar or identical to ribs 122b in some or many respects.

In some implementations, cover 220 includes one or more gas ports configured to allow gas to be delivered into an interior region of the cover 220 and, when cover 220 is coupled with hub 260 and secured to a subject (see FIG. 2F), to and/or towards a catheter insertion site enclosed by the cover 220 and/or hub 260. For example, cover 220 can include gas ports 237a and 237b. Gas port 237b can be configured to allow gas to flow into such interior region (for example, through wall 223b) and gas port 237a can be configured to allow gases to flow out of such interior region (for example, through wall 223b). In some variants, gas port 237a is configured to allow gas to flow into such interior region (for example, through wall 223b) and gas port 237b is configured to allow gases to flow out of such interior region (for example, through wall 223b). Gas port 237b can be configured to secure to a gas supply tube to allow gases (for example, sterilizing and/or anesthetic gases) to be inserted through wall 223b and towards the catheter insertion site. Such configurations can advantageously allow such gases to flow to and/or around the catheter insertion site, catheter 241, catheter hub 242, luer connector 243 (which may also be referred to herein as "catheter hub connector") within an interior region defined by the catheter housing 200 when secured to the subject's skin. In some implementations, such as that shown, gas ports 237a, 237b extend outward from cover 220 (for example, from wall 223b) in opposite directions and/or from opposite ends of cover 220. Such implementations can advantageously provide desirable gas flow (for example, "flushing") into and out of the interior region defined by the catheter housing 200 when in use.

With reference to FIG. 2K, in some implementations, cover 220 includes one or more UV light sources that illuminate and/or disinfect the catheter insertion site where the catheter 241 is inserted in the subject and/or areas within an interior of the cover 220 and/or surrounding various components housed within the cover 220 when the catheter housing 200 is in use. In such implementations, cover 220 can include one or more light source housings 277 which can be similar or identical to light source housings 177 described above with respect to cover 120. The arrangement and/or utilization of such optional light source housings 277 and UV light sources mounted therein can be similar or identical to the arrangement and/or utilization discussed above with respect to light source housings 177.

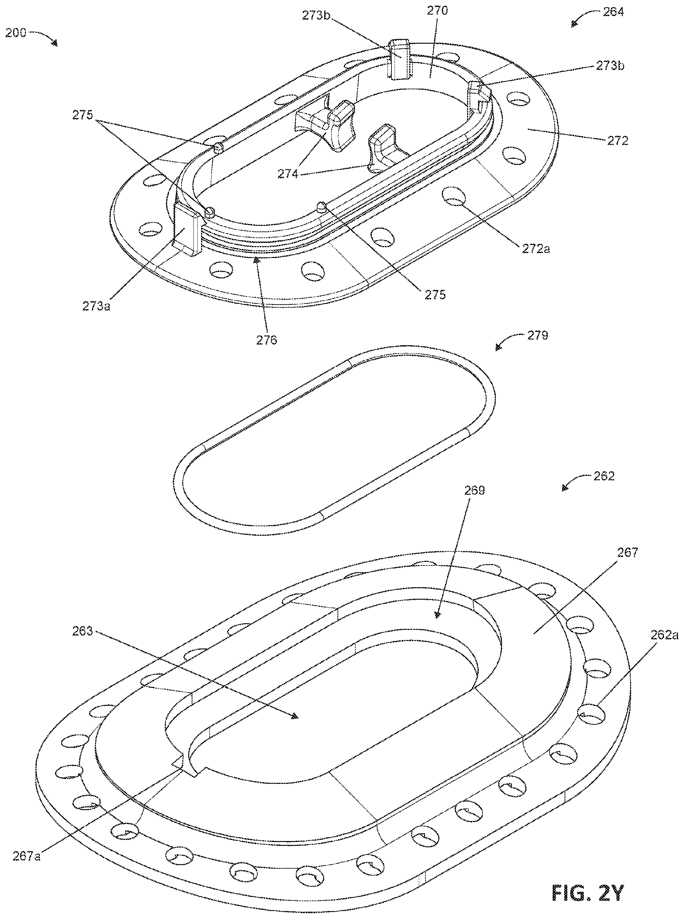

Cover 220 can be configured to removably secure to hub 260. In some implementations, cover 220 includes a protrusion 239a (see FIG. 2H-2I and 2K-2P). Protrusion 239a can be configured to engage latch arm 273a of hub 260 (see FIGS. 2S-2T and 2Y), and such engagement can allow cover 220 and hub 260 to secure to one another. Additionally or alternatively, cover 220 can include one or both of protrusions 239b shown in FIGS. 2K and 2M. Protrusions 239b can be configured to engage latch arms 273b of hub 260 (see FIGS. 2S-2T and 2Y), and such engagement can allow cover 220 and hub 260 to secure to one another. As shown, protrusion 239a can extend from an outer surface of wall 232a and protrusions 239b can extend from an interior surface of wall 232a. Protrusions 239a, 239b may also be referred to herein as "latch protrusions". Latch arms 273a, 273b of hub 260 may also be referred to herein as "latch legs". In some implementations, protrusions 239a, 239b comprise a tapered profile.

The engagement between protrusion 239a and latch arm 273a and/or between protrusions 239b and latch arms 273b can be, for example, a snap fit engagement. For example, in some cases, cover 220 can be secured to hub 260 by moving cover 220 onto (for example, from above) hub 260 such that protrusion 239a engages latch arm 273a and/or such that protrusions 239b engage latch arm 273b. Protrusion 273a can have a tapered surface that contacts a tapered end of latch arm 273a and/or that causes latch arm 273a to be pushed outward (for example, away from an interior or center of hub 260) until protrusion 239a passes the tapered end of latch arm 273a, at which point, such tapered end of latch arm 273a can snap into securement with protrusion 239a. Protrusions 239b can have tapered surfaces that contact tapered ends of latch arms 273b, pushing latch arms 273b inward (for example, towards an interior or center of hub 260) until protrusions 239b pass such tapered ends of latch arms 273b, at which point, such tapered ends of latch arms 273b can snap into securement with protrusions 239b. To remove cover 220 from hub 260, latch arm 273a can be pushed outward, thereby allowing an end (for example, a "front") of cover 220 to be removed. Thereafter, protrusions 239b can be disengaged from latch arms 273b and cover 220 can be separated from hub 260.

With reference to FIG. 2K and 2Q, cover 220 includes an opening 233 to allow tube 247 (which can also be referred to as "tubing" or "fluid tube") to extend through cover 220 so as to allow tube 247 to deliver fluids to catheter 241 via catheter hub 242 when cover 220 is secured to hub 260 on a subject. Opening 233 (which may also be referred to herein as "cover opening") can be positioned at or near an end of the cover 220. Opening 233 can extend through wall 223b, for example, as shown.

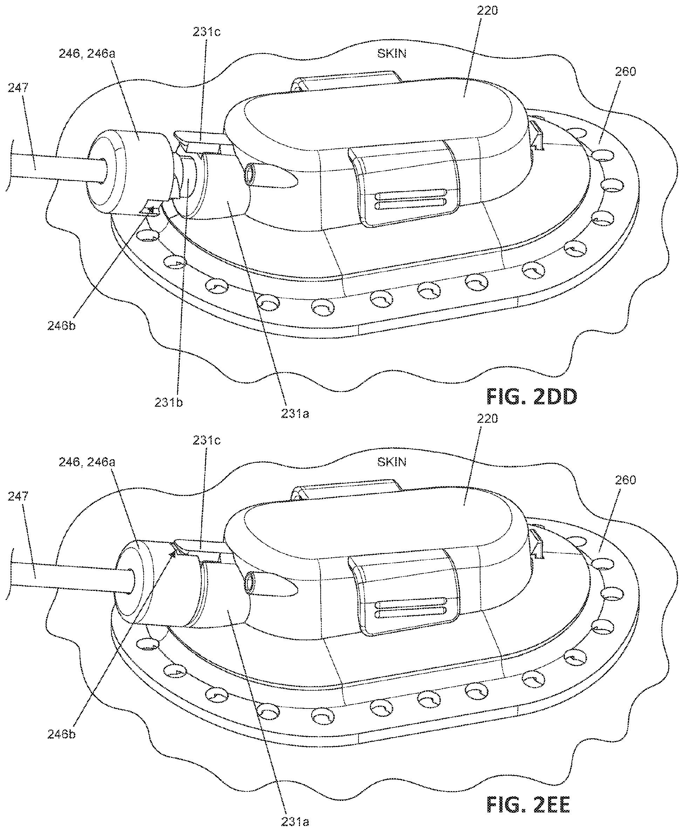

In some implementations, cover 220 includes a connector (which may also be referred to as a "connector portion") configured to facilitate connection to extension set 240. For example, cover 220 can include a connector 231 (which may also be referred to herein as "cover connector"). Connector 231 can extend from wall 223b of cover 220 around opening 233. Connector 231 can be secured to connector 246 (also referred to herein as "fluid tube lock connector") as described further below. As also described further below, when connectors 231, 246 are connected to one another, movement (for example, sliding) of cover 220 and/or connector 246 relative to tube 247 can be inhibited (for example, prevented), and when connectors 231, 246 are disconnected to one another, movement (for example, sliding) of cover 220 and/or connector 246 relative to tube 247 can be permitted. Such connection can advantageously prevent the pulling of tube 247 from a location outside the cover 220 from causing movement of the catheter 241, catheter hub 242, luer connector 243 and/or a portion of tube 247 housed within an interior of cover 220. Connector 231 and connector 246 can be configured to threadingly connect to one another, for example, via threads thereof, and/or via a snap-fit engagement. Connector 231 can extend from wall 223b at an end (for example, a "front" end) of cover 220. Connector 231 can include a connector body 231a extending outward from wall 223b and a threaded portion 231b extending outward from connector body 231a. Connector body 231a and/or threaded portion 231b can have a generally cylindrical shape and have an opening aligning with opening 233 so as to allow tube 247 to extend therethrough. In some implementations, threaded portion 231b have a smaller cross-section than connector body 231 a. In some implementations, connector 231 additionally or alternatively includes a latch arm 231c (which may also be referred to herein as "latch" or "cover connector latch"). Latch arm 231c can extend outward from wall 223b and/or connector body 231a and can be positioned adjacent connector body 231a. Latch arm 231c can engage notch 246b of connector 246 as described further below with respect to FIGS. 2DD-2EE. Latch arm 231c can be resilient, to allow latch arm 231c to flex during engagement with connector 246 and notch 246b as described below.

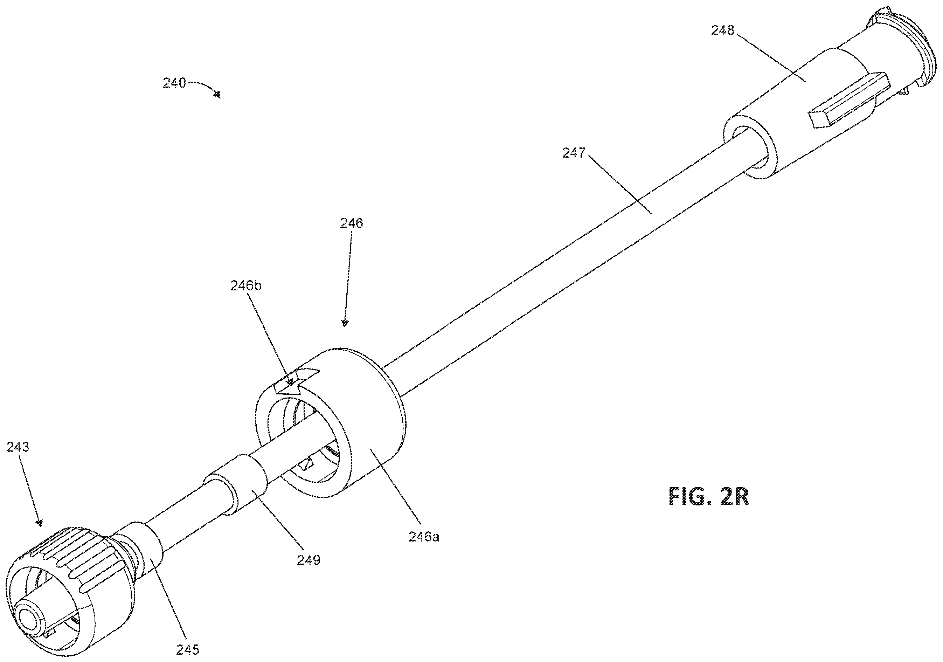

FIG. 2R illustrates an enlarged perspective view of an extension set 240 (also referred to herein as a "fluid tube assembly"). Extension set 240 can be utilized to deliver fluids to the subject via catheter 241. Extension set 240 can include a connector 243 (which may be a male luer connector), a stopper 245, a sleeve 249, a connector 246 (also referred to herein as a "fluid tube lock connector"), tube 247, and/or a connector 248 (which may be a female luer connector). Connectors 243, 248, and/or tube 247 can be similar or identical to connectors 143, 148, and/or tube 147 (respectively) discussed above with respect to extension set 140. Connectors 243, 248 can connect to opposite ends of tube 247 as shown. Connector 248 can be configured to connect to a fluid source that holds fluids for providing infusion therapy to a subject. Connector 243 can be configured to connect to catheter hub 242, for example, as shown in FIGS. 2C, 2E, and 2F.

As mentioned previously, connector 246 can advantageously be utilized with connector 231 of cover 220 to inhibit (for example, prevent) movement of cover 220 and/or connector 246 relative to tube 247. Connector 246 can include a body 246a which can include an interior having threads that can be configured to secure to threaded portion 231b of connector 231 of cover 220. Body 246a can include an opening that allows connector 246 to be coupled to tube 247, for example, such that connector 246 can be moved (for example, slid) along tube 247. In some implementations, connector 246 includes a notch 246b recessed from an outer surface of body 246a that can be configured to engage latch arm 231c of connector 231 of cover 220 as mentioned above and as described in more detail below. Notch 246b can be sized and/or shaped to accommodate a portion of latch arm 231c. Connector 246 can have a generally cylindrical shape, among others. With reference to FIG. 2CC, extension set 240 and cover 220 can be coupled to one another such that tube 247 extends through connector 231 (for example, through connector body 231a and threaded portion 231b), through opening 233 in wall 223b of cover 220, and such that cover 220 (for example, wall 223b of cover 220) is positioned along tube 247 between connector 243, stopper 245 and connectors 246, 248. In such configuration, cover 220 and connector 246 can be configured to slide along tube 247 (for example, along a longitudinal axis of tube 247), for example, prior to connection of connector 246 to connecter 231 as described below.

FIG. 2F illustrates catheter housing 200 assembled and secured to a subject's skin as mentioned previously. FIG. 2F illustrates connector 246 secured to connector 231 of cover 220. Connector 246 can be connected to connector 231 via engagement between threads in body 246a of connector 246 and threaded portion 231b of connector 231. As mentioned previously, in some implementations, connector 231 includes a latch arm 231c, and in such implementations, latch arm 231c can engage with notch 246b of connector 246. For example, latch arm 231c can engage with notch 246b after body 246a is threadingly secured to threaded portion 231b. With reference to FIG. 2DD, connector 246 can be threaded onto threaded portion 231b (for example, via rotation), and during such threading engagement, body 246a (for example, an outer surface of body 246a) of connector 246 can push latch arm 231c, causing latch arm 231c to flex in a direction away from connector body 231a. After continual threading (via rotation of connector 246), latch arm 231c can snap into notch 246b of connector 246, thereby "locking" connector 231 to connector 246 (see FIG. 2EE). Disconnection of connectors 231, 246 from one another can be achieved by flexing latch arm 231c (for example, in a direction away from connector body 231a) such that latch arm 231c is removed from notch 246b, thereby allowing connector 246 to be rotated in a opposite rotational direction as that done when threadingly securing connector 246 to threaded portion 231b. As discussed above, extension set 240 can include a sleeve 249. Sleeve 249 can be operably positioned by connector 246 such that pressure is applied to sleeve 249 when connectors 246. 231 are secured to one another (see FIG. 2F). When connectors 246, 231 are connected to one another, movement of connector 246 and the cover 220 along the tube 247 can be inhibited (for example, prevented), for example, via a gripping force applied by sleeve 249 onto tube 247 induced by pressure applied to sleeve 249 by connectors 246, 231. Such configurations can allow the connectors 246, 231 to "lock" a portion of tube 247 such that forces applied to tube 247 outside cover 220 are not translated to a portion of tube 247 inside cover 220 and/or to connector 243, catheter hub 242, and catheter 241. This can advantageously inhibit dislodgement of catheter 241 and/or catheter hub 242, and can inhibit trauma to the catheter insertion site. Sleeve 249 can comprise a tubular shape and/or can comprise the same material as tube 247. Sleeve 249 can be moveable (for example, slidable) along tube 247, for example, when connectors 246, 231 are not secured to one another. Sleeve 249 can have a smaller length than tube 247. Sleeve 249 can be made of a variety of materials, such as polyvinyl chloride (PVC), a latex free material, and/or other materials.

In some implementations, extension set 240 include a stopper 245 (see FIGS. 2F, 2G, and 2R). With reference to FIG. 2E, stopper 245 can be coupled to tube 247 and can be positioned between connector 243 and opening 233 and/or wall 223a of cover 220 when cover 220 and extension set 240 are secured to one another (and/or when cover 220 and extension set 240 are secured to hub 260). Stopper 245 can have an annular (for example circular) shape. Stopper 245 can have a length that is smaller than a length of tube 247. Stopper 245 can be permanently bonded to a portion of tube 247. Advantageously, with reference to FIG. 2F, stopper 245 can prevent tube 247 from being pulled out of cover 220 and/or can isolate a portion of tube 247 (inside cover 220), connector 243, catheter hub 242, and/or catheter 241 from forces applied to tube 247 outside cover 220. For example, if a force is applied to a portion of tube 247 outside cover 220 in a direction along a longitudinal axis of tube 247 and/or transverse (for example, perpendicular) to such axis, stopper 245 can engage wall 223a and prevent such force from causing movement of connector 243, catheter hub 242, and/or catheter 241. Stopper 245 can be utilized alone or in combination with sleeve 249. In some cases, stopper 245 can be engaged if the force applied to tube 247 exceeds a capacity of sleeve 249 to resist such force.

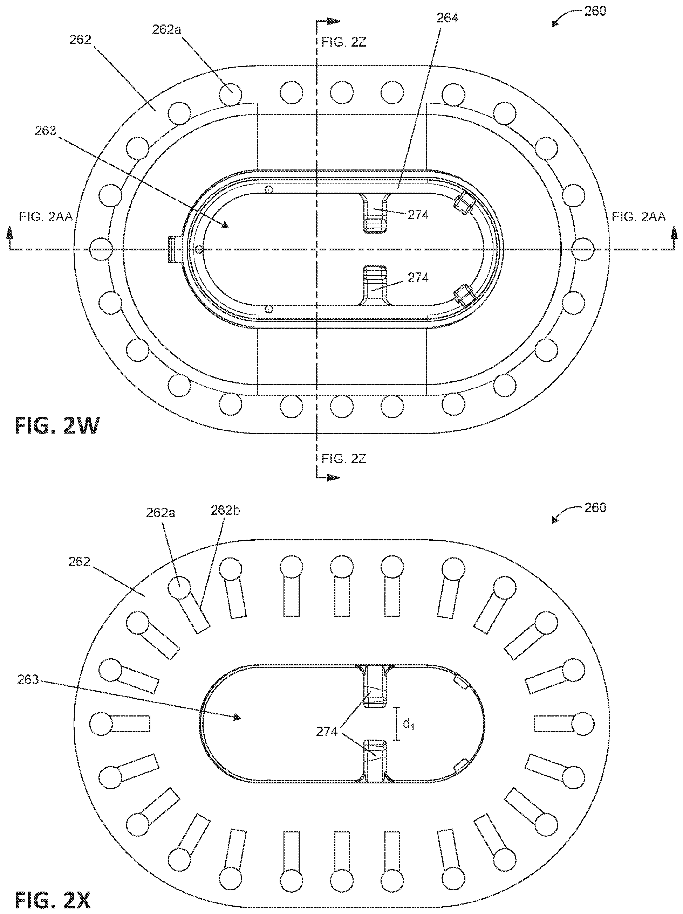

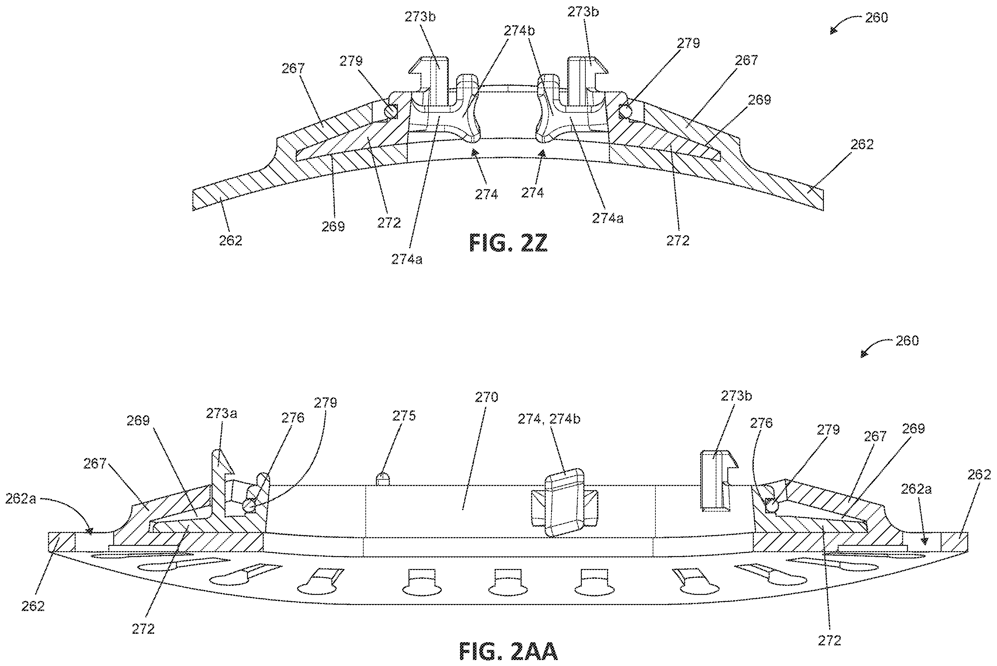

FIGS. 2S-2T illustrate top perspective views, FIGS. 2U-2V illustrate bottom perspective views, and FIGS. 2W-2X illustrate top and bottom (respectively) views, of hub 260. FIG. 2Y illustrates an exploded perspective view of hub 260 and FIGS. 2Z-2AA illustrate cross-sectional views taken through a portion of hub 260 (see FIG. 2W). Hub 260 can be configured to secure to skin of a subject around a catheter insertion site. Hub 260 (for example, membrane 262) can include an opening 263 configured to be positioned over the catheter insertion site when hub 260 is secured to the subject's skin. Hub 260 can be configured to secure catheter hub 242 connected to catheter 241 to minimize movement of catheter 241 when inserted in a catheter insertion site. Securement of catheter hub 242 with hub 260 (for example, as opposed to cover 220) can advantageously allow the catheter insertion site, catheter 241, catheter hub 242, and/or other components to be viewed by a caregiver prior to enclosure of the catheter insertion site with cover 220. This can in turn allow the caregiver to conveniently assess whether the catheter hub 242 has been appropriately secured and/or positioned, for example, so as to operably position catheter 241 at an appropriate angle relative to the subject's skin and/or a vein in which the catheter 241 is inserted.

Hub 260 can include a membrane 262 and a frame 264. Membrane 262 can include one or more perforations 262a (also referred to herein as "apertures") to facilitate breathability of the subject's skin when membrane 162 is secured thereto. The number, arrangement, and characteristics of perforations 262a can be similar or identical to perforations 162a discussed above with respect to membrane 162. Membrane 262 can include an opening 263 which can be positioned over a site where a needle and/or catheter 241 is to be (or has been) inserted into a subject (for example, patient). In some implementations, such as that shown, such opening 163 is not partitioned and/or divided by any portion of membrane 262. Opening 263 can have a rounded shape. For example, opening 263 can comprise an oblong shape which is not partitioned and/or divided by any portion of membrane 262. In some implementations, when catheter housing 200 is assembled and secured to the subject's skin as shown in FIG. 2F, no portion of membrane 262 is positioned between the catheter hub 242 (and/or connector 243) and the subject's skin. In some implementations, hub 260 and/or membrane 262 (and/or cover 220) is made of a transparent or semi-transparent material, which can allow for inspection of the catheter insertion site, catheter 241, catheter hub 242, connector 243 when catheter housing 200 is in use. Membrane 262 can be made of a flexible material. Membrane 262 can be made of a different material than cover 220. Membrane 262 can comprise, for example, thermoplastic elastomers (TPE), thermoplastic polyurethane (TPU), and/or polyvinyl chloride (PVC), among other materials. In some implementations, membrane 262 comprises a material that is more flexible than a material comprised by cover 220. Membrane 262 can be more flexible than frame 264. In some implementations, membrane 262 is transparent or semi-transparent. In some variants, hub 260 does not include frame 264. In such variants, features of frame 264 (such as arms 274, latch arms 273a, 273b, and/or indicators 275) are part of membrane 262.

In some implementations, one or more substrates (which may also be referred to as "layers") are disposed on a bottom surface of membrane 262 so as to allow membrane 262 to secure to the subject's skin. In some implementations, one or more adhesive layers are secured to a bottom surface of membrane 262. In some implementations, a top layer (for example, comprising an adhesive material such as a rubber-based adhesive) is bonded to the bottom surface of membrane 262, an intermediate layer (for example, comprising foam, a rubber-based or acrylic based adhesive, a polyurethane film, a double sided adhesive layer, among other materials) is secured to the top layer, and a bottom layer (for example, comprising an adhesive material such as an acrylic-based adhesive) is secured to the intermediate layer. Such intermediate layer can bond together the top layer (intended to secure to membrane 262) and the bottom layer (intended to secure to the subject's skin). In some implementations, one or more release liners (which may also be referred to as "release layers") are secured to membrane 262 (for example, to one or more adhesive layers disposed on a bottom surface of membrane 262). Such release liner(s) can be removed prior to securement of membrane 262 to the subject's skin. Illustrative release liner configurations are shown and described with reference to FIGS. 2JJ-2LL. With reference to FIGS. 2U-2V and 2X, in some implementations, membrane 262 comprises one or more or a plurality of recessed portions 272b positioned adjacent to apertures 262a. Recessed portions 272b can be recessed from a bottom surface of membrane 262 and can extend from apertures 262a inward, for example, towards a center of membrane 262. Recessed portions 272b can advantageously allow an adhesive removal liquid (comprising, for example, alcohol) that passes through apertures 262a to flow underneath the bottom surface of membrane 262 so as to loosen the adhesive engagement with the skin. Such configurations can advantageously allow membrane 262 (and hub 260) to be removed from the subject's skin more easily.

FIG. 2Y illustrates membrane 262 separated from frame 264. Membrane 262 can include a raised portion 267 extending above and/or outward from a portion of membrane 262 (for example, spaced inward from a perimeter of membrane 262) and around a perimeter of opening 263. Such raised portion 267 can include a slot 269 (which can also be referred to as a "channel") that can extend around a perimeter of opening 263. Slot 269 can be sized and/or shaped to receive a flange 272 of frame 264 as described further below. During manufacturing, flange 272 can be inserted into slot 269 and permanently secured (for example, bonded) to the membrane 262. Such permanent securement can be via adhesive and/or ultrasonic welding, among other things. In some cases, membrane 262 can be overmolded onto frame 264. In such cases, flange 272 can include one or more openings 272a (such as one, two, three, four, between one and twenty openings 272a) that allow the overmolded material of membrane 262 to pass therethrough, thereby forming a locking bond through openings 272a. Accordingly, although slot 269 is illustrated as being open in FIG. 2Y, it is to be understood that slot 269 could be filled (and thus nonexistent) if membrane 262 is overmolded onto frame 264. In some implementations, membrane 262 includes a notch 267a configured to accommodate latch arm 273a, as shown in FIGS. 2S and 2Y.

In some implementations, hub 260 comprises a moisture wicking element configured to wick moisture away from the catheter insertion site and/or all or a portion of the subject's skin that is encircled by opening 263. For example, in some implementations, a moisture wicking element is disposed on membrane 262 along all of or less than an entirety of the perimeter of opening 263. Such moisture wicking element can have an annular shape, for example, that corresponds to a shape of opening 263, and can extend along all of or less than an entirety of the perimeter of opening 263. Such moisture wicking element can comprise a tricot fabric. Such moisture wicking element can comprise nylon and spandex, or polyester and spandex for example. Additionally or alternatively, such moisture wicking element can comprise a hydrocolloid.

Frame 264 can be made of a variety of materials. For example, frame 264 can comprise plastic. Frame 264 can comprise polyphenyl ether (PPE) and/or polycarbonate (PC), among other materials. In some implementations, frame 264 is transparent or semi-transparent. In some implementations, frame 264 and cover 220 are made of the same material. In some implementations, frame 264 is made of a more rigid material than membrane 262. With continued reference to FIG. 2Y, frame 260 can include a frame body 270 and a flange 272 extending outward from frame body 270, for example, along all of a portion of a perimeter of frame body 270. Frame body 270 can have an annular and/or oblong shape, among others. Frame 264 can be connected with membrane 262 along all of or less than an entirety of a perimeter of opening 263 of membrane 262. In some implementations, latch arm 273a and/or latch arms 273b (discussed previously) extend outward from flange 272. In some variants, latch arm 273a and/or latch arms 273b extend outward from frame body 270. In some implementations such as that illustrated in FIG. 2Y, latch arms 273b are positioned at an opposite end of frame 264 (for example, frame body 270) as latch arm 273a.

Hub 260 can include one or more indicators that can advantageously aid in positioning hub 260 relative to a catheter insertion site and/or relative to catheter 241 and/or catheter hub 242. For example, frame 264 can include indicators 275 extending outward from a surface of frame body 270. In some cases, frame 264 includes two indicators 275 positioned on opposite sides of frame body 270 and/or an indicator 275 positioned along an end of frame body 270. Such indicator(s) 275 can be aligned with a catheter insertion site so that hub 260 can be appropriately secured in place, for example, such that frame 264 (for example, arms 274) can be operably positioned to secure catheter hub 242. In some implementations, frame body 270 includes a groove 276 extending around all or a portion of a perimeter of frame body 270. Such groove 276 can be sized and/or shaped to receive an annular member 279. FIGS. 2Z-2AA illustrate annular member 279 secured within groove 276 and FIG. 2F illustrates annular member 279 secured within groove 276 and cover 220 and hub 260 secured together. Annular member 279 can be utilized to seal an interior region defined between catheter housing 200 and the subject's skin when cover 220 and hub 260 are secured to one another. Such seal can inhibit (for example, prevent) gas from entering and/or exiting such interior region along a connection point or region of cover 220 and hub 260. Annular member 279 can be an O-ring, for example.

As mentioned previously, hub 260 can be configured to secure catheter hub 242 to minimize movement of catheter 241 when inserted in a catheter insertion site. Hub 260 can include one or more arms configured to secure catheter hub 242 to minimize movement of catheter 241 when inserted in a catheter insertion site. For example, as shown in FIGS. 2S-2Y, hub 260 (for example, frame 264) can include arms 274. Arms 274 can be coupled with membrane 262 via frame 264. Arms 274 can extend outward from portions of frame 264 and across (for example, partially across) an opening of frame 264 and/or opening 263 when frame 264 and membrane 262 are coupled with one another. FIG. 2Z illustrates a cross-sectional view taken through hub 260. In some implementations such as that illustrated, arms 274 do not extend completely across opening 263. Arms 274 can include a first end (which may also be referred to herein as a "connected end") connected to a portion of frame 264 (for example, a portion of frame body 270) and a second end (also referred to herein as a "free" or "cantilevered" end) opposite such first end. Arms 274 can extend outward from a portion of frame 264, and such "free" ends can be spaced from one another by a gap, represented by "d₁" in FIG. 2X. Such second or "free" ends of the arms 274 can be spaced inward from frame body 270, a perimeter of an opening of frame 264, and/or a perimeter of opening 263. As mentioned previously, frame 264 (for example, frame body 270) can be connected to membrane 262 along all or a portion of a perimeter of opening 263. In some implementations, arms 274 can each comprise a generally T-shaped structure.

With reference to FIG. 2Z, arms 274 can include a first section 274a (which may also be referred to as a "stem" or "first segment") extending outward from a portion of frame 264 (for example, a portion of frame body 270) and a second section 274b (which may also be referred to as a "flange" or "second segment") connected to an end of the first section 274a (which can be a "free" end of arm 274). Second section 274b can be spaced from the portion of the frame 264 from which the first section 274a extends (for example, spaced from the frame body 270). In some implementations, the second section 274b is transverse (for example, generally perpendicular) relative to the first section 274a. In some implementations, the second sections 274b are at least partially curved (see FIG. 2Z) (for example, curved toward one another). For example, surfaces of second sections 274b of arms 274 that face toward one another can be curved. The second section 274b can be at least partially curved to conform to and/or surround a portion of catheter hub 242 when catheter hub 242 is secured by arms 274. In some implementations, surfaces of second sections 274b of arms 274 that face toward one another comprise matching curvatures. In some implementations, arms 274 are mirror images of one another. In some implementations, as shown in FIG. 2AA, the second sections 274b are inclined relative to a plane extending along a top of frame body 270, a plane extending along a bottom surface of membrane 262, and/or the subject's skin (when hub 260 is secured to the subject's skin). Such implementation can allow arms 274 to secure catheter hub 242 at an inclined angle relative to the subject's skin when hub 260 is in use (for example, at an angle shown in FIG. 2F).

Arms 274 (or portions thereof) can be sized and/or shaped to secure any of a variety of types of catheter hubs, such as any of the catheter hubs discussed herein, among others. In some implementations, frame 264 and arms 274 comprise a rigid material, and in such implementations, arms 274 can provide rigid securement for catheter hub 242. In some variants, however, frame 264 and/or arms 274 comprise a flexible material (for example, similar or identical to the materials discussed above with respect to membrane 262). In some variants, arms 274 comprise a more flexible material than frame body 270, from which arms 274 can extend.

In some implementations, when catheter hub 242 is secured by arms 274 in use and hub 260 is secured to the subject's skin: no portion of catheter housing device 200 is positioned between arms 274 and the subject's skin; no portion of the cover 220 contacts catheter hub 242; and/or no portion of membrane 262 contacts catheter hub 242. With reference to FIG. 2F, catheter hub 242 can have a first end connected to catheter 241 and a second end connected to connector 243. In some implementations, when catheter hub 242 is secured by arms 274 in use and hub 260 is secured to the subject's skin: such second end of the catheter hub 242 does not contact the subject's skin; only the first end of catheter hub 242 contacts the subject's skin; and/or no portion of catheter hub 242 contacts the subject's skin. With reference to FIG. 2Z and 2AA, in some implementations, arms 274 are spaced from (for example, positioned above) a plane extending along a bottom surface of membrane 262. Such configuration can cause arms 274 to be spaced from (for example, above) the subject's skin when hub 260 is in use, which in turn advantageously inhibits (for example, prevents) arms 274 from causing potential irritation to the subject's skin.

Although FIGS. 2S-2M illustrate two arms 274, in some variants, hub 260 (for example, frame 264) includes an alternative amount of arms 274. For example, in some variants, hub 260 (for example, frame 264) includes one arm 274 extending outward from a portion of frame 264 that is configured to secure catheter hub 242 to minimize movement of catheter 241 when inserted in a catheter insertion site.

FIGS. 2BB-2EE show an illustrate manner by which catheter housing 200 can be assembled and secured to a subject. Catheter 241 can be inserted at a catheter insertion site in accordance with conventional approaches. After catheter 241 is inserted, hub 260 can be positioned atop the catheter insertion site such that opening 263 is positioned over the portion of the catheter 241 that is outside the skin and/or over the catheter hub 242 connected to catheter 241. Hub 260 can be secured to the subject's skin, for example, via adhesive securement of membrane 262 to the skin. In some implementations, indicators 275 (which can be optionally included in hub 260) are utilized to position and/or align hub 260 with the insertion site, catheter 241, and/or catheter hub 242. Catheter hub 242 can then be secured by arms 274, as shown in FIG. 2BB, for example, in an inclined position relative to the subject's skin where arms 274 are so configured. With reference to FIG. 2CC, connector 243 can then be connected to catheter hub 242. Where cover 220 is coupled to tube 247 as discussed above and as shown in FIG. 2CC, cover 220 can be moved along tube 247 towards hub 260. Cover 220 can be secured to hub 260, for example, via engagement of protrusions 239a, 239b of cover 220 and latch arms 273a, 273b as described elsewhere herein. Securement of cover 220 to hub 260 can therefore enclose the catheter insertion site, catheter hub 242, and/or connector 243. With reference to FIGS. 2CC-2DD, connector 246 can be moved along tube 247 towards cover 220, and secured to connector 231 of cover 220 as described elsewhere herein. As discussed previously, connector 246 can be threadingly secured to connector 231 (for example, via engagement between threads of connector 246 and threaded portion 231b). As also discussed previously, connector 246 can be secured to connector 231 in a snap-fit engagement via securement of latch 231c and notch 246b. Such connection can "lock" tube 247 relative to cover 220 and connector 246, for example, to inhibit movement of cover 220 and/or connector 246 relative to tube 247 as described previously. Such assembly of catheter housing 200 can be a simple yet effective manner for stabilizing catheter 241 and enclosing the catheter insertion site. Catheter housing 200 can be assembled and secured to a subject in an alternative manner than that discussed above, however, and the above-described manner is not intended to be limiting.

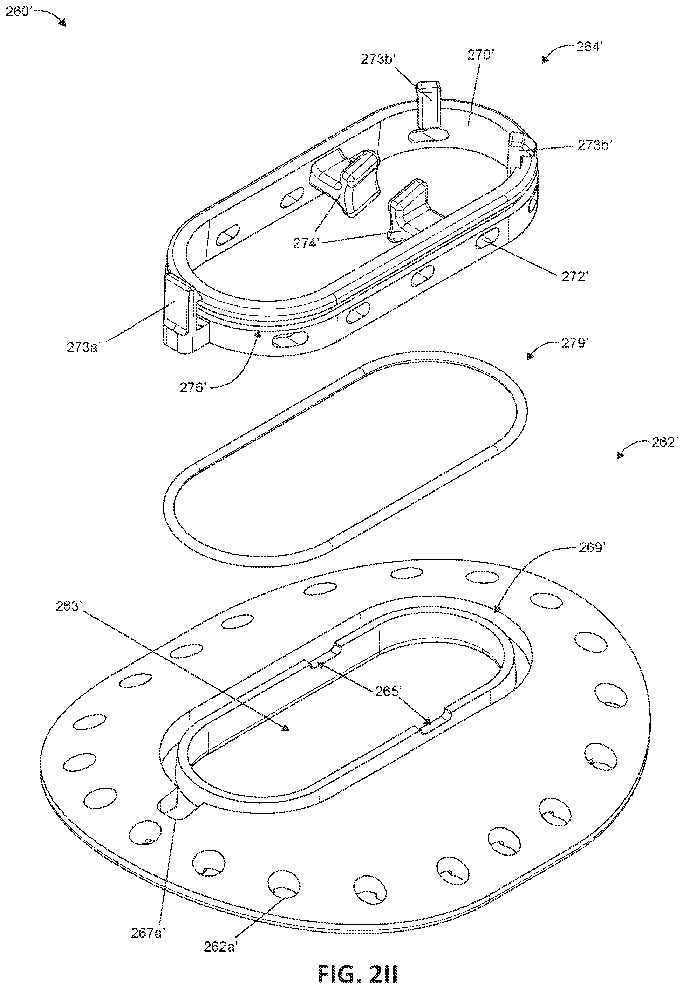

FIGS. 2FF-2II illustrate an alternative implementation of a hub 260'. Hub 260' can be similar or identical to hub 260 in some or many respects. Hub 260' includes a frame 264' and a membrane 262'. FIG. 2II illustrates an exploded view of hub 260'. Frame 264' and membrane 262' can be similar or identical to frame 264 and membrane 262 (respectively) in some or many respects.

Membrane 262' can include one or more perforations 262a' (also referred to herein as "apertures") to facilitate breathability of the subject's skin when membrane 262' is secured thereto. The number, arrangement, and characteristics of perforations 262a' can be similar or identical to perforations 162a and/or 262a discussed above. Membrane 262' can include an opening 263' which can be positioned over a site where a needle and/or catheter 241 is to be (or has been) inserted into a subject (for example, patient). In some implementations, such as that shown, such opening 263' is not partitioned and/or divided by any portion of membrane 262'. Opening 263' can have a rounded shape. For example, opening 263' can comprise an oblong shape which is not partitioned and/or divided by any portion of membrane 262'. In some implementations, hub 260' (for example, frame 264' and/or membrane 262') is made of a transparent or semi-transparent material. Membrane 262' and/or frame 264' can be made of any of the materials discussed herein with reference to membrane 262 and frame 264 (respectively), among other materials. One of more substrates and/or release liners can be disposed on a bottom surface of membrane 262' such as any of those discussed above with respect to membrane 262.

With continued reference to FIG. 2II, membrane 262' can include a slot 269' (which can also be referred to as a "channel") that can extend around a perimeter of opening 263'. Slot 269' can be sized and/or shaped to receive a portion of frame body 270'. During manufacturing, a portion of frame body 270' can be inserted into slot 269' and permanently secured (for example, bonded) to the membrane 262'. Such permanent securement can be via adhesive and/or ultrasonic welding, among other things. In some cases, membrane 262' is overmolded onto frame 264'. In such cases, frame body 270' can include one or more openings 272' (such as one, two, three, four, between one and twenty openings 272') can allow the overmolded material of membrane 262' to pass through and thereby form a locking bond through openings 272'. Reference numerals 268' in the cross-section shown in FIG. 2JJ illustrate portions of membrane 262' that extend through opening 272' in frame body 270' to form a locking bond between frame 264' and membrane 262' after membrane 262' is overmolded onto frame 264'. Although slot 269' is illustrated as being open in FIG. 2Y, it is to be understood that slot 269' can be filled (and thus nonexistent) if membrane 262' is overmolded onto frame 264'. In some implementations, membrane 262' includes a notch 267a' configured to accommodate latch arm 273a'. In some implementations, membrane 262' includes notches 265' along a portion of membrane 262' proximate opening 263' that are sized to accommodate a portion of arms 274'.

Arms 274' can extend outward from a portion of frame 264' (for example, from opposite sides of frame body 270') toward one another and can be similar or identical to arms 274. Therefore, the discussion above with respect to arms 274 is equally applicable to arms 274'.

Frame 264' includes a frame body 270' and latch arms 273a', 273b' extending outward from frame body 270'. Latch arms 273a', 273b' can be configured to engage protrusions 239a, 239b of cover 220 in a similar or identical manner as that described above with respect to latch arms 273a, 273b. Frame body 270' can be similar or identical to frame body 270 in some or many respects. Frame body 270' can comprise an annular (for example, oblong) shape, among others. Frame 264' can be connected with membrane 262' along all or a portion of a perimeter of opening 263 of membrane 262. One difference between frame 264' and frame 264 is that frame 264' does not include flange 272. In some implementations, frame body 270' includes a groove 276' that can be similar or identical to groove 276 of frame body 270, and such groove 276' is configured to receive an annular member 279' (that can be similar or identical to annular member 279).

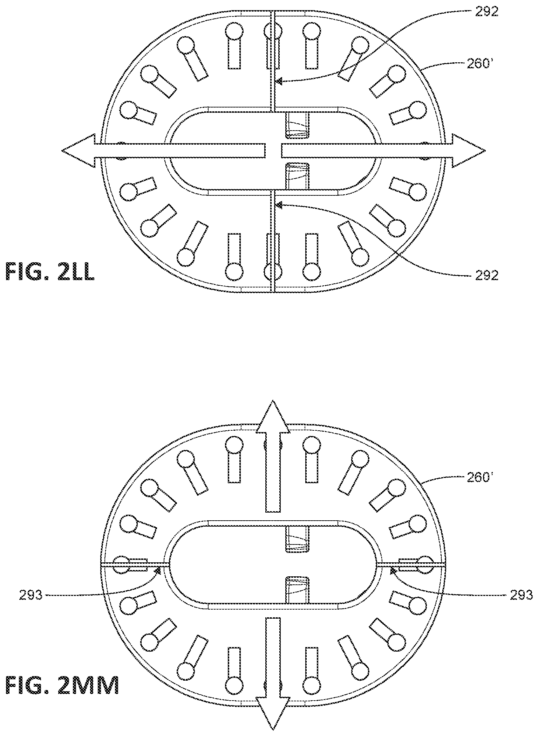

FIGS. 2JJ-2LL show illustrative implementations of release liners that can be employed in any of the catheter housing devices disclosed herein. FIGS. 2KK-2MM illustrate bottom views of hub 260' with a first release liner configuration (FIG. 2KK), a second release liner configuration (FIG. 2LL), and a third release liner configuration (FIG. 2MM). Any of the of the catheter housing devices disclosed herein can include one or more release liners disposed on one or more adhesive layers coupled to a bottom surface of a hub (for example, a bottom surface of a membrane of such hub). Although FIGS. 2KK-2MM show hub 260', the release liner configurations discussed with reference to FIGS. 2KK-2MM and hub 260' is equally applicable to any of the other hubs discussed herein (such as hubs 160, 260, and/or 360). FIG. 2KK illustrates a release liner having a single cut or slit 291, which can allow removal of the release liner (prior to securement of hub 260' to the subject's skin) in a direction indicated by the arrow. Such slit 291 allows the release liner to be removed from hub 260' and a connected cover (for example cover 220) and extension set (such as extension set 240). FIG. 2LL illustrates two release liners separated by two slits 292 (oriented perpendicular to a long axis of the hub 260'), which allows such release liners to be removed sequentially (in a direction indicated by the arrows), for example, to allow a first portion of the bottom surface of hub 260' to be pressed down and secured to the subject's skin before a second portion of the bottom surface of the hub 260'. FIG. 2MM illustrates two release liners separated by two slits 293 (oriented parallel to a long axis of the hub 260'), which allows such release liners to be removed sequentially (in a direction indicated by the arrows), for example, to allow a first portion of the bottom surface of hub 260' to be pressed down and secured to the subject's skin before a second portion of the bottom surface of the hub 260'.

FIGS. 3A-3V illustrate a catheter housing 200 (which may also be referred to as a "catheter housing device", "catheter stabilization device", or "catheter stabilization system") and various aspects and/or portions thereof as further described below. Catheter housing 300 can be advantageously be used to stabilize a catheter when the catheter is inserted in a catheter insertion site of a subject, in a similar manner as that described elsewhere herein, for example, with reference to catheter housing 100 and/or 200. As described in more detail below, stabilization of a catheter 341 can be achieved by engagement with a catheter hub 342 connected to such catheter and/or by engagement with a connector 343 connected to catheter hub 342

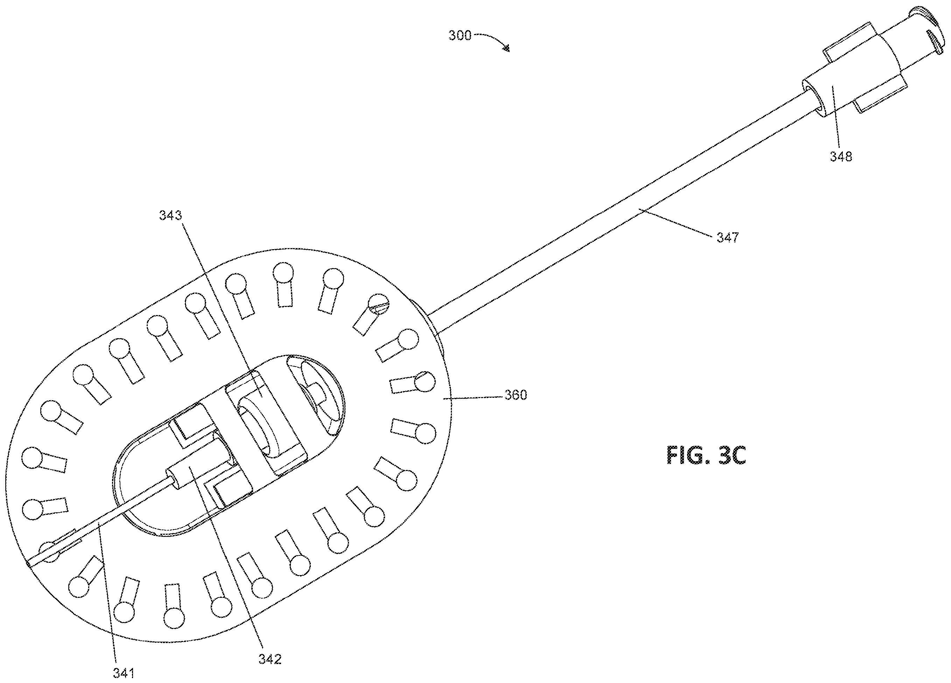

As shown, catheter housing 300 can include a cover 320, a hub 360, and an extension set 340 (also referred to herein as a "fluid tube assembly"). FIGS. 3A-3B illustrate top perspective views, FIG. 3C illustrates a bottom perspective view, and FIGS. 3D-3E illustrate top and bottom views, of catheter housing 300. Each of FIGS. 3C and 3E illustrate catheter hub 342 (connected to a catheter 341) and connector 343 secured by the catheter housing 300, as further described below. Catheter 341 and/or catheter hub 342 can be similar or identical to any of the other catheters and/or catheter hubs described herein. Some implementations of catheter housing 300 do not include extension set 340.

FIG. 3F illustrates a cross-sectional view taken through the catheter housing 300 (see FIG. 3D) in a condition of use in which catheter housing 300 is secured to skin of a subject. Although not shown, it is to be understood that catheter 341 can be positioned at least partially within a vein of the subject, in accordance with conventional catheter insertion practices, so that the catheter 341 can deliver fluid to the subject. Stabilization of catheter 341 is described in more detail below. FIG. 3G illustrates cover 320, hub 360, and extension set 340 separated from one another and from catheter 341 and catheter hub 342.

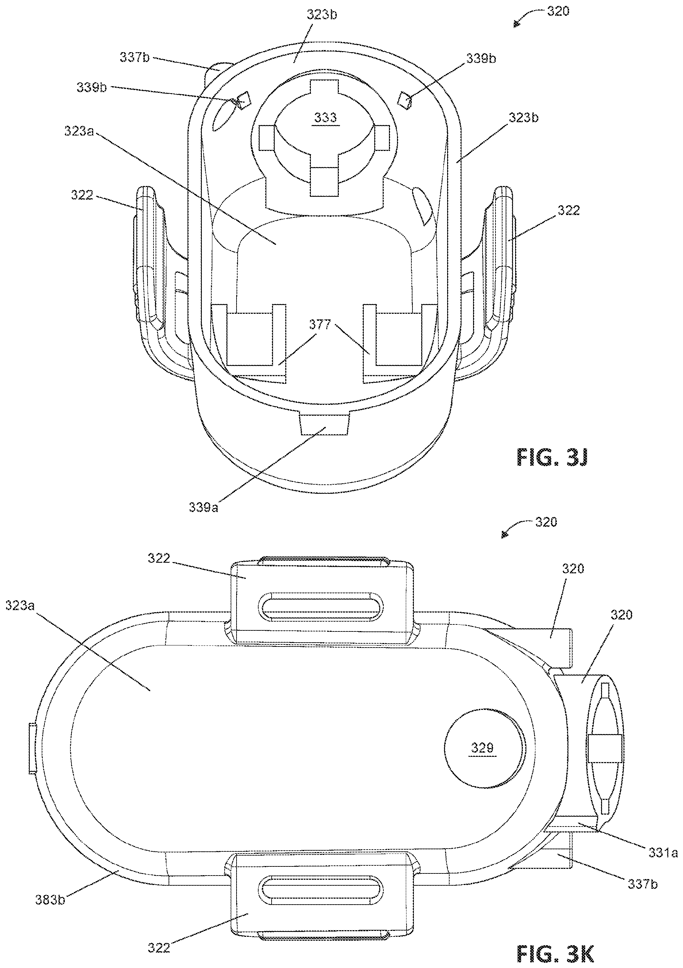

FIGS. 3H-3I illustrate top and side perspective views, FIG. 3J illustrates a bottom perspective view, and FIG. 3K illustrates a top view, of cover 320. Cover 320 can be similar or identical to cover 120 and/or 220 in some or many respects. Cover 320 can include a top portion 323a and a wall 323b that can be similar or identical to top portion 123a, 223a and wall 123b, 223b respectively. Cover 320 can include wing(s) 322 with slots 322a that can be similar or identical to wing(s) 122 with slots 122a of cover 120. Cover 320 can include protrusion 339a and protrusions 339b which can be identical to protrusion 239a, protrusions 239b (respectively) of cover 220. Cover 320 can include gas ports 337a, 337b that can be similar or identical to gas ports 237a, 237b or any of the other gas ports described herein. As shown, gas ports 337a, 337b can extend from the same end of cover 320, for example, outward from wall 323b in the same direction, and/or can be located on a same end of cover 320 that connector 331 and/or opening 333 is on. With reference to FIG. 3J, cover 320 can include one or more light source housings 377 that can be similar or identical to light source housings 177, 277 of cover 120, 220. In some implementations in which catheter housing 300 includes one or more UV light sources (for example, housed within light source housing 377), catheter housing 300 can include a battery configured to provide power to such UV light sources. In some implementations, catheter housing 300 includes a battery housing 329 connected to and/or extending outward from top portion 323a. Cover 320 can be made of any of the materials discussed above with reference to cover 120 and/or 220, among other materials. In some implementations, cover 320 is transparent or semi-transparent. In some implementations, cover 320 is made of a more rigid material than hub 360 or portions of hub 360. For example, in some implementations, cover 320 is made of a more rigid material than membrane 362.

Cover 320 includes an opening 333 (which may also be referred to herein as "cover opening") that can be similar or identical to opening 133 in some or many respects. Cover 320 includes a rim 331 (which may also be referred to herein as a "connector" or "cover connector") that can extend outward from an interior and/or exterior surface of the cover 320 (for example, of the wall 323b) around the opening 333. Rim 331 can be similar or identical to rim 131 in some or many respects. For example, rim 331 can include one or more notches 335, the number, arrangement, and characteristics of which can be similar or identical to notches 135. Rim 331 can secure to connector 344 and connector 346 as discussed further below. As shown, cover 320 can include a tab 331a extending outward from rim 331, for example, extending outward from an outer surface of rim 331. Tab 331a can be utilized when rim 331 is secured to connector 346 as discussed further below.

FIG. 3L illustrates extension set 340 (which may also be referred to herein as a "fluid tube assembly"). Extension set 340 can be similar in some or many respect to extension set 140. Extension set 340 can include a connector 343 (which can be similar or identical to connector 143 and/or 243), a tube 347 (which can be similar or identical to tube 147, 247), a connector 348 (which can be similar to identical to connector 148, 248), and sleeve 349 (which can be similar or identical to sleeve 249). Extension set 340 can include a connector 344 which can be similar or identical to connector 144. Connector 344 can engage with rim 331 in a manner that is similar or identical to that described with respect to connector 144 and rim 131 above. Rings 345a, 345b can be included in extension set 340 and secured to connector 344 as shown. With reference to FIG. 3F, connector 344 can be partially inserted through opening 333 (and rim 331) of cover 320 and secured to connector 346 in a similar manner discussed above with respect to connector 144, opening 133 (and rim 131), and connector 146. Sleeve 349 can be operably positioned by connector 346 and can engage tube 347 to inhibit relative movement of cover 320 and/or connector 346 relative to tube 347 in a similar or identical manner as that described above with respect to sleeve 249, cover 220 and connector 246. With reference to FIG. 3L, connector 346 can include a body 346a having an opening which receives tube 347 and a finger 346b extending from body 346a, and such finger 346b can contact tab 331a of cover 320 when connector 346 is threadingly secured to threads of connector 344 (which can be similar or identical to threads on connector 144 discussed above). In some cases, tab 331a is tapered such that, finger 346b slides over (for example, flexing outward from opening 333) the tapered portion of tab 331a when connector 346 is threaded onto connector 144 a given rotational amount, and, after such rotational amount is reached, finger 346b snaps inward toward opening 333. Such placement of finger 346b relative to tab 331a can prevent removal of connector 346 from connector 144 and rim 331 since tab 331a can obstruct finger. Body 346a can have a cylindrical shape, as shown, and can include threads inside an interior of body 346a that can threadingly engage threads on connector 344 (which can be similar or identical to threads 195).

FIGS. 3M-3N illustrate top perspective views, FIG. 3O illustrates a bottom perspective view, FIGS. 3P-3Q illustrate top and bottom views of hub 360, and FIG. 3R illustrates an exploded view, of hub 360. FIGS. 3S-3T illustrate cross-sectional views taken through hub 360 (see FIG. 3P). FIG. 3U illustrates an enlarged view of a portion of hub 360 shown in FIG. 3M. Hub 360 can be similar or identical to hub 260 in some or many respects. Hub 360 can include a frame 364 and membrane 362. Frame 364 can be similar or identical to frame 264 in some or many respects. For example, with reference to FIG. 3R, frame 364 can include a frame body 370 that can be similar or identical to frame body 270 in some or many respects. Frame 364 can include latch arm 373a and latch arms 373b that can be similar or identical to latch arm 273a and latch arms 273b (respectively) in some or many respects and can secure to protrusions 339a, 339b of cover 320 in a similar or identical manner as that described above with respect to latch arms 273a, 273b of frame 264 and protrusions 239a, 239b of cover 220. Frame 364 can include a flange 372 and/or openings 372a similar or identical to flange 272 and openings 272a of frame 264. Frame 364 and/or membrane 362 can comprise materials similar or identical to any of those described herein with reference to frame 164, 264 and membrane 162, 262, among others. Membrane 362 can include one or more perforations 362a that can be similar or identical to the one or more perforations 162a, 262a of membrane 162, 262. Membrane 362 can include slot or channel 369 that can be similar or identical to slot or channel 269 of membrane 262. The securement of flange 372 and slot 369 and/or manufacturing (for example, via overmolding) of frame 364 and membrane 362 can be similar or identical to that described with reference to flange 272, slot 269, frame 264, and membrane 262. Membrane 362 can include opening 363 that can be similar or identical to opening 263. Hub 360 can include an annular member 379 that can be similar or identical to annular member 279. Frame body 370 can include a groove 376 that can be similar or identical to groove 276 of frame 264, and groove 376 can receive annular member 379 in a similar or identical manner as that described with respect to annular member 279 and groove 276. In some implementations, frame 364 includes indicators 375 defined as notches along frame body 370 (see FIG. 3N). Indicators 375 can be used in a similar manner as that described above with respect to indicators 275.

Hub 360 includes structure configured to secure catheter hub 342 and/or connector 343 which acts to stabilize catheter 341 when inserted into a catheter insertion site so as to minimize movement of catheter 341. Such structure can extend across (for example, fully across opening 363) and can be connected to frame 364. With reference to FIGS. 3M-3R, hub 360 can include a mounting structure 380 and/or mounting structure 390. Mounting structure 380 can be configured to secure catheter hub 342 and mounting structure 390 can be configured to secure connector 343 (which can be a male luer connector) connectable to catheter hub 342. FIG. 3V illustrates a top perspective view of hub 360 with catheter hub 342 and connector 343 secured by mounting structures 380, 390. As shown, in some implementations, connector 343 can be retained by and between mounting structures 380, 390. In some variants, hub 360 only includes one of mounting structure 380 or 390. For example, in some variants, hub 360 includes mounting structure 380 but does not include mounting structure 390 or includes mounting structure 390 but does not include mounting structure 380. Mounting structures 380, 390 can be connected to frame 364 and can extend across opening 363. For example, mounting structures 380, 390 can connect to and extend between opposite sides of frame body 370 of frame 364. In some implementations, mounting structure 380 and/or 390 is integrally formed with frame 364. Mounting structures 380, 390 can be spaced apart from another, as shown.

With reference to FIG. 3S, mounting structure 380 can include a base 382 that extends across opening 363 between frame body 370. Base 382 can be spaced from a bottom of hub 360 (for example, a bottom surface of membrane 362) by a distance d₂. Such configuration can inhibit or prevent mounting structure 380 from contacting the subject's skin when hub 360 is in use, which in turn advantageously inhibits (for example, prevents) mounting structure 380 from causing potential irritation to the subject's skin. Mounting structure 380 can include arms 384 (which may also be referred to as "side walls") extending outward from base 382. Arms 234 can extend transverse (for example, perpendicular) to base 382, as shown. Arms 384 can be spaced from one another and such spacing can be sized to receive catheter hub 342. As shown in FIG. 3S, mounting structure 380 can include a surface 385 configured to face and/or contact catheter hub 342 when catheter hub 342 is secured by mounting structure 380. Such surface 385 can be defined between arms 384. Surface 385 can have a rounded cross-section that conforms to a shape of a cross-section of catheter hub 342. For example, surface 385 can have a circular cross-section. As shown in at least FIGS. 3S and 3U, surface 385 can be inclined, for example, inclined such that a first end of surface 385 that is closer to mounting structure 390 is higher than a second end of surface 385 that is opposite such first end. Such configuration can advantageously allow catheter hub 342 to be inclined when secured by mounting structure 380 (see FIG. 3V). In some implementations, mounting structure 380 includes protrusions 386 extending from arms 384 toward each other. Such protrusions 386 can be disposed at or near ends of arms 384 (for example, "free" or "cantilevered" ends of arms 384). Such protrusions 386 can act to inhibit catheter hub 342 from being removed from mounting structure 380. In some cases, catheter hub 342 can be removed from mounting structure 380 by flexing arms 384 away from each other, thereby moving protrusions 386 away from one another. In some cases, such flexing of arms 384 can occur if a sufficient removal force is applied to catheter hub 382 (for example, in an "upward" direction given the view shown in FIG. 3S).

With reference to FIG. 3T, mounting structure 390 can include a base 392 that extends across opening 363 between frame body 370. Base 392 can be spaced from a bottom of hub 360 (for example, a bottom surface of membrane 362) by a distance d₃. Such configuration can inhibit or prevent mounting structure 390 from contacting the subject's skin when hub 360 is in use, which in turn advantageously inhibits (for example, prevents) mounting structure 390 from causing potential irritation to the subject's skin. Mounting structure 390 can include arms 394 (which may also be referred to as "side walls") extending outward from base 392. Arms 394 can extend transverse (for example, perpendicular) to base 392, as shown. Arms 394 can be spaced from one another and such spacing can be sized to receive catheter hub 342. As shown in FIG. 3T, mounting structure 390 can include a surface 395 configured to face and/or contact connector 343 when connector 343 is secured by mounting structure 390. Surface 395 can be defined between arms 394. Surface 395 can have a rounded cross-section that conforms to a shape of a cross-section of connector 343. For example, surface 395 can have a circular cross-section. As shown in at least FIGS. 3T and 3U, surface 395 can be inclined, for example, inclined such that a first end of surface 395 that is closer to mounting structure 380 is lower than a second end of surface 395 that is opposite such first end. Such configuration can advantageously allow connector 343 to be inclined when secured by mounting structure 390 (see FIG. 3V). In some implementations, mounting structure 390 includes a back wall 397 that is adjacent such second end of surface 395 and transverse (for example, perpendicular to) to arms 394. Back wall 397 can help retain connector 343, for example, as shown in FIG. 3V and can inhibit (for example, prevent) connector 343 from being moved laterally away from catheter hub 342 (for example, an direction away from mounting structure 380). In some implementations, back wall 397 comprises a groove 397a along an end of back wall 397. Groove 397a can have a rounded shape (for example, a circular shape) that is conforms to a shape of a portion of connector 343 (for example, a portion of connector 343 that couples to an end of tube 247 as shown in FIG. 3V).

Catheter housing 300 can be assembled and secured to a subject in a similar manner as that described above with reference to catheter housing 200 and FIGS. 2BB-2EE. For example, after a caregiver inserts catheter 341 in an insertion site in accordance with conventional approaches, hub 360 can be positioned atop the catheter insertion site such that opening 363 is positioned over the portion of the catheter 341 that is outside the skin and/or over the catheter hub 342. Hub 360 can be secured to the subject's skin, for example, via adhesive securement of membrane 362 to the skin. Catheter hub 342 and/or connector 343 can then be secured by mounting structures 380, 390 for example, as illustrated in FIG. 3V. It is noted that connector 343 can be connected to catheter hub 342 before or after securement of catheter hub 342 by mounting structure 380. As discussed previously, cover 320 can be coupled to extension set 340, for example, where cover 320 is coupled to tube 347 and connector 344 extends through opening 333 and engages rim 331 of cover 320. After connector 343 and catheter hub 342 are secured to one another and secured by mounting structures 380, 390, cover 320 can be secured to hub 360, for example, via engagement of protrusions 339a, 339b on cover 320 and latch arms 373a of hub 360. Securement of cover 320 to hub 360 can therefore enclose the catheter insertion site, catheter hub 342, and/or connector 343. Connector 346 (which may be referred to as a "fluid tube lock connector") can be moved along tube 347 towards cover 320, and secured to connector 244 (for example, via engagement between threads of connector 346 and threads of connector 244). As discussed above, connector 346 can include finger 346b that can pass over and then be obstructed by tab 331a of cover 320. The connection of connector 346 to connector 344 at rim 331 can "lock" tube 347 relative to cover 320 and connector 346, for example, to inhibit movement of cover 320 and/or connector 346 relative to tube 347. Such assembly of catheter housing 300 can be a simple yet effective manner of stabilizing catheter 341 and enclosing the catheter insertion site. Catheter housing 300 can be assembled and secured to a subject in an alternative manner than that discussed above, however, and the above-described manner is not intended to be limiting.

Any of the catheter housings described herein (such as catheter housings 100, 200, 300) or portions thereof (for example, hub 160, 260, 260', 360 and/or cover 120, 120', 120", 220, 320) can include various sensors, including bio-sensors that can measure, gather, and transmit patient medical condition data. The bio-sensors can include a microprocessor. For example, the bio-sensors can include an illuminated LCD monitor for detecting, measuring, storing and/or displaying patient vital functions, including venous and arterial blood pressure, heart beats, blood oxygen levels, general and topical temperature, and local tissue humidity, and/or venous blood current speed, among others. The measurements and/or calculations performed and/or taken by these sensors can be stored on a flash storage memory mounted on the catheter housing. Alternatively, the sensor measurements can be wirelessly transmitted (or be transmitted via a wire) to a patient monitoring system for display to a care provider or user.

### Additional Considerations and Terminology

Conditional language used herein, such as, among others, "can," "could," "might," "may," "e.g.," and the like, unless specifically stated otherwise, or otherwise understood within the context as used, is generally intended to convey that certain features, elements, and/or steps are optional. Thus, such conditional language is not generally intended to imply that features, elements, and/or steps are in any way required or that one or more embodiments necessarily include logic for deciding, with or without other input or prompting, whether these features, elements, and/or steps are included or are to be always performed. The terms "comprising," "including," "having," and the like are synonymous and are used inclusively, in an open-ended fashion, and do not exclude additional elements, features, acts, operations, and so forth. Also, the term "or" is used in its inclusive sense (and not in its exclusive sense) so that when used, for example, to connect a list of elements, the term "or" means one, some, or all of the elements in the list. Further, the term "each," as used herein, in addition to having its ordinary meaning, can mean any subset of a set of elements to which the term "each" is applied.

Certain terminology can be used in the following description for the purpose of reference only, and thus are not intended to be limiting. For example, terms such as "above" and "below" refer to directions in the drawings to which reference is made. Terms such as "proximal," "distal," "front," "back," "rear," and "side" describe the orientation and/or location of portions of the components or elements within a consistent but arbitrary frame of reference which is made clear by reference to the text and the associated drawings describing the components or elements under discussion. Such terminology can include the words specifically mentioned above, derivatives thereof, and words of similar import.

Conjunctive language such as the phrase "at least one of X, Y, and Z," unless specifically stated otherwise, is otherwise understood with the context as used in general to convey that an item, term, etc. may be either X, Y, or Z. Thus, such conjunctive language is not generally intended to imply that certain embodiments require the presence of at least one of X, at least one of Y, and at least one of Z.

Language of degree used herein, such as the terms "approximately," "about," "generally," and "substantially" as used herein represent a value, amount, or characteristic close to the stated value, amount, or characteristic that still performs a desired function or achieves a desired result. For example, the terms "approximately", "about", "generally," and "substantially" may refer to an amount that is within less than 10% of, within less than 5% of, within less than 1% of, within less than 0.1% of, and within less than 0.01% of the stated amount. As another example, in certain embodiments, the terms "generally parallel" and "substantially parallel" refer to a value, amount, or characteristic that departs from exactly parallel by less than or equal to 10 degrees, 5 degrees, 3 degrees, or 1 degree. As another example, in certain embodiments, the terms "generally perpendicular" and "substantially perpendicular" refer to a value, amount, or characteristic that departs from exactly perpendicular by less than or equal to 10 degrees, 5 degrees, 3 degrees, or 1 degree.

While the above detailed description has shown, described, and pointed out novel features, it can be understood that various omissions, substitutions, and changes in the form and details of the devices or systems illustrated can be made without departing from the disclosure. As can be recognized, certain portions of the description herein can be embodied within a form that does not provide all of the features and benefits set forth herein, as some features can be used or practiced separately from others. The scope of certain embodiments disclosed herein is indicated by the appended claims rather than by the foregoing description.

## Claims

1. A catheter stabilization device (300) configured for use at a catheter insertion site on a subject, the catheter stabilization device (300) comprising:
a hub (360) configured to be secured to skin of the subject around the catheter insertion site, the hub (360) comprising:
a membrane (362) comprising an opening (363) configured to be positioned over the catheter insertion site when the hub (360) is secured to the subject's skin;
a frame (364) connected to the membrane (362) along at least a portion of a perimeter of the opening (363); and
a mounting structure (380) extending over the opening (363) of the membrane (362) and connected to the frame (364), said mounting structure (380) configured to secure to a catheter hub (342) connected to a catheter (341) that is configured to be inserted in the catheter insertion site; and
a cover (320) configured to removably secure to the hub (360) and at least partially enclose the catheter insertion site.

2. The catheter stabilization device (300) of Claim 1, wherein said mounting structure (380) is integrally formed with the frame (364).

3. The catheter stabilization device (300) of Claim 1, wherein said mounting structure is connected to and extends between opposite sides of a frame body (370) of the frame (364).

4. The catheter stabilization device (300) of Claim 1, wherein the frame (364) comprises a frame body (370), and wherein said mounting structure (380) comprises:
a base (382) extending across the opening (363) and between portions of the frame body (370); and
arms (384) spaced from one another and configured to receive the catheter hub (342).

5. The catheter stabilization device (300) of Claim 4, wherein the base (382) is spaced from a bottom of the hub (360) by a distance.

6. The catheter stabilization device (300) of Claim 4, wherein the arms (384) extend outward from and transverse to the base (382).

7. The catheter stabilization device (300) of Claim 4, wherein the mounting structure (380) comprises a surface (385) defined between the arms (384) and configured to face and/or contact the catheter hub (342) when the catheter hub (342) is secured by the mounting structure (380), said surface (385) comprising a rounded cross-section configured to conform to a shape of a cross-section of the catheter hub (342).

8. The catheter stabilization device (300) of Claim 4, wherein the arms (384) are configured to be flexed away from each other to allow the catheter hub (342) to be removed from the mounting structure (380).

9. The catheter stabilization device (300) of Claim 4, wherein the frame body (370) comprises an annular and/or oblong shape.

10. The catheter stabilization device (300) of Claim 1, wherein said frame (364) comprises a plurality of latch arms (373a, 373b) and said cover (320) comprises a plurality of latch protrusions (339a, 339b), and wherein said cover (320) and said hub (360) are configured to removably secure to one another via engagement between the plurality of latch protrusions (339a, 339b) and the plurality of latch arms (373a, 373b).

11. The catheter stabilization device (300) of Claim 1, further comprising an extension set (340), the extension set (340) comprising a male luer connector (343) configured to removably secure to the catheter hub (342), a female luer connector (348) configured to connect to a fluid source, and a fluid tube (347) connected to the male luer connector (343) and the female luer connector (348), wherein the extension set (340) is configured to be removably connected to the cover (320) and hub (360).

12. The catheter stabilization device (300) of Claim 11, wherein the cover (320) encloses the male luer connector (343) of the extension set (340) when the cover (320) is secured to the hub (360).

13. The catheter stabilization device (300) of Claim 1, wherein the frame (364) comprises a first material and the membrane (362) comprises a second material, and wherein the first material is more rigid than the second material.

14. The catheter stabilization device (300) of Claim 1, wherein the frame (364) comprises an annular shape.

15. The catheter stabilization device (300) of Claim 1, wherein, when the catheter stabilization device (300) is assembled and secured to the subject's skin, no portion of the membrane (362) is positioned between the catheter hub (342) and the subject's skin.

## Patentansprüche

1. Katheterstabilisierungsvorrichtung (300), die für eine Verwendung an einer Kathetereinführungsstelle an einem Subjekt konfiguriert ist, wobei die Katheterstabilisierungsvorrichtung (300) Folgendes umfasst:
ein Verbindungsstück (360), das dafür konfiguriert ist, an der Haut des Subjekts um die Kathetereinführungsstelle befestigt zu werden, wobei das Verbindungsstück (360) Folgendes umfasst:
eine Membran (362), die eine Öffnung (363) umfasst, die dafür konfiguriert ist, über der Kathetereinführungsstelle positioniert zu werden, wenn das Verbindungsstück (360) an der Haut des Subjekts befestigt ist,
einen Rahmen (364), der entlang mindestens eines Abschnitts eines Umfangs der Öffnung (363) mit der Membran (362) verbunden ist, und
eine Anbringungsstruktur (380), die sich über die Öffnung (363) der Membran (362) erstreckt und mit dem Rahmen (364) verbunden ist, wobei die Anbringungsstruktur (380) dafür konfiguriert ist, sich an einem Katheterverbindungsstück (342) zu befestigen, das mit einem Katheter (341) verbunden ist, der dafür konfiguriert ist, in die Kathetereinführungsstelle eingeführt zu werden, und
eine Abdeckung (320), die dafür konfiguriert ist, sich abnehmbar an dem Verbindungsstück (360) zu befestigen und die Kathetereinführungsstelle zumindest teilweise zu umschließen.

2. Katheterstabilisierungsvorrichtung (300) nach Anspruch 1, wobei die Anbringungsstruktur (380) integral mit dem Rahmen (364) geformt ist.

3. Katheterstabilisierungsvorrichtung (300) nach Anspruch 1, wobei die Anbringungsstruktur mit gegenüberliegenden Seiten eines Rahmenkörpers (370) des Rahmens (364) verbunden ist und sich zwischen denselben erstreckt.

4. Katheterstabilisierungsvorrichtung (300) nach Anspruch 1, wobei der Rahmen (364) einen Rahmenkörper (370) umfasst und wobei die Anbringungsstruktur (380) Folgendes umfasst:
eine Basis (382), die sich über die Öffnung (363) und zwischen Abschnitten des Rahmenkörpers (370) erstreckt, und
Arme (384), die voneinander beabstandet und dafür konfiguriert sind, das Katheterverbindungsstück (342) aufzunehmen.

5. Katheterstabilisierungsvorrichtung (300) nach Anspruch 4, wobei die Basis (382) von einem Unterteil des Verbindungsstücks (360) um eine Entfernung beabstandet ist.

6. Katheterstabilisierungsvorrichtung (300) nach Anspruch 4, wobei sich die Arme (384) von der Basis (382) aus nach außen und quer zu derselben erstrecken.

7. Katheterstabilisierungsvorrichtung (300) nach Anspruch 4, wobei die Anbringungsstruktur (380) eine Oberfläche (385) umfasst, die zwischen den Armen (384) definiert und dafür konfiguriert ist, dem Katheterverbindungsstück (342) gegenüberzuliegen und/oder dasselbe zu berühren, wobei das Katheterverbindungsstück (342) durch die Anbringungsstruktur (380) befestigt ist, wobei die Oberfläche (385) einen abgerundeten Querschnitt umfasst, der dafür konfiguriert ist, sich an eine Form eines Querschnitts des Katheterverbindungsstücks (342) anzupassen.

8. Katheterstabilisierungsvorrichtung (300) nach Anspruch 4, wobei die Arme (384) dafür konfiguriert sind, voneinander weg gebogen zu werden, um zu ermöglichen, dass das Katheterverbindungsstück (342) von der Anbringungsstruktur (380) entfernt wird.

9. Katheterstabilisierungsvorrichtung (300) nach Anspruch 4, wobei der Rahmenkörper (370) eine ringförmige und/oder längliche Gestalt umfasst.

10. Katheterstabilisierungsvorrichtung (300) nach Anspruch 1, wobei der Rahmen (364) eine Vielzahl von Verriegelungsarmen (373a, 373b) umfasst und die Abdeckung (320) eine Vielzahl von Verriegelungsvorsprüngen (339a, 339b) umfasst und wobei die Abdeckung (320) und das Verbindungsstück (360) dafür konfiguriert sind, sich über einen Eingriff zwischen der Vielzahl von Verriegelungsvorsprüngen (339a, 339b) und der Vielzahl von Verriegelungsarmen (373a, 373b) aneinander zu befestigen.

11. Katheterstabilisierungsvorrichtung (300) nach Anspruch 1, die ferner einen Erweiterungssatz (340) umfasst, wobei der Erweiterungssatz (340) einen Luer-Anschlussstecker (343), der dafür konfiguriert ist, sich abnehmbar an dem Katheterverbindungsstück (342) zu befestigen, eine Luer-Anschlussbuchse (348), die dafür konfiguriert ist, sich mit einer Fluidquelle zu verbinden, und eine Fluidröhre (347), die mit dem Luer-Anschlussstecker (343) und der Luer-Anschlussbuchse (348) verbunden ist, umfasst, wobei der Erweiterungssatz (340) dafür konfiguriert ist, abnehmbar mit der Abdeckung (320) und dem Verbindungsstück (360) verbunden zu werden.

12. Katheterstabilisierungsvorrichtung (300) nach Anspruch 11, wobei die Abdeckung (320) den Luer-Anschlussstecker (343) des Erweiterungssatzes (340) umschließt, wenn die Abdeckung (320) an dem Verbindungsstück (360) befestigt ist.

13. Katheterstabilisierungsvorrichtung (300) nach Anspruch 1, wobei der Rahmen (364) ein erstes Material umfasst und die Membran (362) ein zweites Material umfasst und wobei das erste Material steifer ist als das zweite Material.

14. Katheterstabilisierungsvorrichtung (300) nach Anspruch 1, wobei der Rahmen (364) eine ringförmige Gestalt umfasst.

15. Katheterstabilisierungsvorrichtung (300) nach Anspruch 1, wobei, wenn die Katheterstabilisierungsvorrichtung (300) zusammengebaut und an der Haut des Subjekts befestigt ist, kein Abschnitt der Membran (362) zwischen dem Katheterverbindungsstück (342) und der Haut des Subjekts positioniert ist.

## Revendications

1. Dispositif de stabilisation de cathéter (300) configuré pour être utilisé au niveau d'un site d'insertion de cathéter sur un sujet, le dispositif de stabilisation de cathéter (300) comprenant :
un embout (360) configuré pour être fixé à la peau du sujet autour du site d'insertion de cathéter, l'embout (360) comprenant :
une membrane (362) comprenant une ouverture (363) configurée pour être positionnée sur le site d'insertion de cathéter lorsque l'embout (360) est fixé à la peau du sujet ;
un cadre (364) relié à la membrane (362) le long d'au moins une partie d'un périmètre de l'ouverture (363) ; et
une structure de montage (380) s'étendant sur l'ouverture (363) de la membrane (362) et reliée au cadre (364), ladite structure de montage (380) étant configurée pour être fixée à un embout de cathéter (342) relié à un cathéter (341) configuré pour être inséré dans le site d'insertion de cathéter ; et
un couvercle (320) configuré pour être fixé de façon détachable à l'embout (360) et pour entourer au moins partiellement le site d'insertion de cathéter.

2. Dispositif de stabilisation de cathéter (300) selon la revendication 1, dans lequel ladite structure de montage (380) est formée intégralement avec le cadre (364).

3. Dispositif de stabilisation de cathéter (300) selon la revendication 1, dans lequel ladite structure de montage est reliée à des côtés opposés d'un corps de cadre (370) du cadre (364) et s'étend entre ceux-ci.

4. Dispositif de stabilisation de cathéter (300) selon la revendication 1, dans lequel le cadre (364) comprend un corps de cadre (370), et dans lequel ladite structure de montage (380) comprend :
une base (382) s'étendant à travers l'ouverture (363) et entre des parties du corps de cadre (370) ; et
des bras (384) espacés l'un de l'autre et configurés pour recevoir l'embout de cathéter (342).

5. Dispositif de stabilisation de cathéter (300) selon la revendication 4, dans lequel la base (382) est espacée d'une partie inférieure de l'embout (360) selon une distance.

6. Dispositif de stabilisation de cathéter (300) selon la revendication 4, dans lequel les bras (384) s'étendent vers l'extérieur et transversalement par rapport à la base (382).

7. Dispositif de stabilisation de cathéter (300) selon la revendication 4, dans lequel la structure de montage (380) comprend une surface (385) définie entre les bras (384) et configurée pour faire face à l'embout de cathéter (342) et/ou pour être en contact avec celui-ci lorsque l'embout de cathéter (342) est fixé par la structure de montage (380), ladite surface (385) comprenant une section transversale arrondie configurée pour s'adapter à une forme d'une section transversale de l'embout de cathéter (342).

8. Dispositif de stabilisation de cathéter (300) selon la revendication 4, dans lequel les bras (384) sont configurés pour être fléchis à distance l'un de l'autre pour permettre à l'embout de cathéter (342) d'être retiré de la structure de montage (380).

9. Dispositif de stabilisation de cathéter (300) selon la revendication 4, dans lequel le corps de cadre (370) comprend une forme annulaire et/ou oblongue.

10. Dispositif de stabilisation de cathéter (300) selon la revendication 1, dans lequel ledit cadre (364) comprend une pluralité de bras de verrouillage (373a, 373b) et ledit couvercle (320) comprend une pluralité de saillies de verrouillage (339a, 339b), et dans lequel ledit couvercle (320) et ledit embout (360) sont configurés pour être fixés de façon détachable l'un à l'autre par engagement entre la pluralité de saillies de verrouillage (339a, 339b) et la pluralité de bras de verrouillage (373a, 373b).

11. Dispositif de stabilisation de cathéter (300) selon la revendication 1, comprenant en outre un kit d'extension (340), le kit d'extension (340) comprenant un raccord luer mâle (343) configuré pour être fixé de façon détachable à l'embout de cathéter (342), un raccord luer femelle (348) configuré pour être raccordé à une source de fluide, et un tube de fluide (347) raccordé au raccord luer mâle (343) et au raccord luer femelle (348), dans lequel le kit d'extension (340) est configuré pour être raccordé de façon détachable au couvercle (320) et à l'embout (360).

12. Dispositif de stabilisation de cathéter (300) selon la revendication 11, dans lequel le couvercle (320) entoure le raccord luer mâle (343) du kit d'extension (340) lorsque le couvercle (320) est fixé à l'embout (360).

13. Dispositif de stabilisation de cathéter (300) selon la revendication 1, dans lequel le cadre (364) comprend un premier matériau et la membrane (362) comprend un deuxième matériau, et dans lequel le premier matériau est plus rigide que le deuxième matériau.

14. Dispositif de stabilisation de cathéter (300) selon la revendication 1, dans lequel le cadre (364) comprend une forme annulaire.

15. Dispositif de stabilisation de cathéter (300) selon la revendication 1, dans lequel, lorsque le dispositif de stabilisation de cathéter (300) est assemblé et fixé à la peau du sujet, aucune partie de la membrane (362) n'est positionnée entre l'embout de cathéter (342) et la peau du sujet.
